# EUROPEAN PATENT SPECIFICATION

(11) **EP 3 268 025 B1**
(45) Date of publication and mention of the grant of the patent: **06.05.2020**
(21) Application number: 16711082.4
(22) Date of filing: 07.03.2016
(51) Int. Cl.: A61K 38/17, C12N 15/113, A61P 35/00, A61K 45/00

(54) **TARGETING GDF6 AND BMP SIGNALING FOR ANTI-MELANOMA THERAPY**
ABZIELUNG AUF GDF6 UND BMP-SIGNALISIERUNG FÜR ANTIMELANOMTHERAPIE
CIBLAGE DE GDF6 ET DE LA SIGNALISATION MÉDIÉE PAR LES BMP POUR UNE THÉRAPIE CONTRE LE MÉLANOME

(30) Priority: 10.03.2015 US 201562130749 P; 20.05.2015 US 201562164304 P; 25.08.2015 US 201562209789 P; 23.10.2015 US 201562245614 P
(43) Date of publication of application: 17.01.2018
(73) Proprietor: University of Massachusetts, Boston, MA 02110 (US)
(72) Inventor: CEOL, Craig Joseph, Sherborn, MA 01770 (US); VENKATESAN, Arvind Murali, Virugambakkam, Chennai Tamil Nadu 600092 (IN)
(74) Representative: Zwicker, Jörk
(86) International application number: PCT/US2016/021274
(87) International publication number: WO 2016/144917

(56) References cited:
- WO-A1-95/01801
- WO-A1-2013/116781
- WO-A1-2013/132267
- Tanja Rothhammer ET AL: "Bone Morphogenic Proteins Are Overexpressed in Malignant Melanoma and Promote Cell Invasion and Migration", Cancer Research, 15 January 2005 (2005-01-15), page 448, XP055275065, United States Retrieved from the Internet: URL:http://cancerres.aacrjournals.org/cont ent/65/2/448.full.pdf
- MICHAL KUCZMA ET AL: "Modulation of bone morphogenic protein signaling in T-cells for cancer immunotherapy", JOURNAL OF IMMUNOTOXICOLOGY, vol. 11, no. 4, 27 October 2014 (2014-10-27), pages 319-327, XP55275028, ISSN: 1547-691X, DOI: 10.3109/1547691X.2013.864736
- JENNIFER M. BAILEY ET AL: "Cancer metastasis facilitated by developmental pathways: Sonic hedgehog, Notch, and bone morphogenic proteins", JOURNAL OF CELLULAR BIOCHEMISTRY, vol. 102, no. 4, 1 November 2007 (2007-11-01), pages 829-839, XP055275031, US ISSN: 0730-2312, DOI: 10.1002/jcb.21509
- WAKEFIELD LALAGE M ET AL: "Beyond TGF beta: roles of other TGF beta superfamily members in cancer", NATURE REVIEWS CANCER, vol. 13, no. 5, May 2013 (2013-05), pages 328-341, XP55274527, ISSN: 1474-175X
- WANG RN ET AL: "Bone Morphogenetic Protein (BMP) signaling in development and human diseases", GENES & DISEASES, vol. 1, 27 July 2014 (2014-07-27), pages 87-105, XP002758074, DOI: 10.1016/j.gendis.2014.07.005

## Description

### BACKGROUND

Melanoma is the most aggressive and deadliest skin cancer, killing over 9,000 Americans and at least 48,000 people worldwide each year. Gain-of-function mutations that overactivate the BRAF serine/threonine kinase are present in a majority of melanomas. However, these mutations are also found in benign nevi (moles), indicating that other genetic alterations are required for melanomagenesis. Additional pathways are also implicated in melanoma maintenance and drug resistance, as BRAF-mutant tumors either fail to response, or respond then quickly relapse, in the face of BRAF inhibitor treatment.

Two new therapies to treat melanomas have been approved recently. While these therapies represent progress in the fight against melanoma, they are not curative. For example, patients treated with the BRAFV600E inhibitor vemurafenib experience only a 3-6 month survival benefit. Advanced melanoma remains a deadly disease with a five-year survival of less than 15%.

### SUMMARY OF INVENTION

The scope of the present invention is defined in the appended claims.

In a first aspect, the invention is directed to a GDF6 inhibitor for use in treating a melanoma in a subject, comprising administering to a subject in need thereof an effective amount of an inhibitor of GDF6. The subject may be a non-human mammal or a human. The inhibiting of GDF6 activity can cause apoptosis of a melanoma cell and can reduce downstream signaling from a BMP receptor with which the GDF6 interacts when compared to a suitable control. The reducing downstream signaling from a BMP receptor can reduce SMAD 1/5/8 phosphorylation. The inhibitor can be selected from the group consisting of an antibody, an antibody fragment, an anti-sense nucleic acid, a soluble receptor polypeptide, and a small molecule. The anti-sense nucleic acid may be selected from the group consisting of a RNAi, a ribozyme, an α-anomeric nucleic acid molecule, and a peptide nucleic acid; and the RNAi can comprise a siRNA or a shRNA. The soluble receptor polypeptide can be a soluble ALK3 polypeptide, such as ALK3(24-152)-Fc fusion comprising an amino acid sequence of SEQ ID NO:9.

A second aspect discloses a screening assay method for identifying a compound that decreases GDF6 activity, comprising
(a) providing an in vitro composition that comprises at least one melanoma cell;
(b) contacting the composition with a test compound; and
(c) determining an effect of the test compound on an activity of GDF6 in the composition,
wherein a decrease in the activity of GDF6 in the presence of the compound, relative to a suitable control, identifies the compound as one that decreases GDF6 activity.

The inhibiting at least one GDF6 activity can comprise apoptosis of a melanoma cell and can comprise reducing downstream signaling from a BMP receptor with which the GDF6 interacts when compared to a suitable control. The reducing downstream signaling from a BMP receptor can comprise reducing SMAD1/5/8 phosphorylation. The test compound can be selected from the group consisting of an antibody, an antibody fragment, an anti-sense nucleic acid, a soluble receptor polypeptide, and a small molecule. The anti-sense nucleic acid may be selected from the group consisting of a RNAi, a ribozyme, an α-anomeric nucleic acid molecule, and a peptide nucleic acid; and the RNAi can comprise a siRNA or a shRNA. The soluble receptor polypeptide can be a soluble ALK3 polypeptide, such as ALK3(24-152)-Fc fusion comprising an amino acid sequence of SEQ ID NO:9.

A third aspect discloses a screening assay method for identifying a compound that decreases a GDF6 activity, comprising
(a) providing a subject that comprises at least one melanoma cell;
(b) administering to the subject a test compound; and
(c) determining an effect of the test compound on an activity of GDF6 in the subject,
wherein a decrease in the activity of GDF6 in the presence of the compound, relative to a suitable control, identifies the compound as one that decreases GDF6 activity. The inhibiting at least one GDF6 activity can comprise apoptosis of a melanoma cell and can comprise reducing downstream signaling from a BMP receptor with which the GDF6 interacts when compared to a suitable control. The reducing downstream signaling from a BMP receptor can comprise reducing SMAD1/5/8 phosphorylation. The test compound can be selected from the group consisting of an antibody, an antibody fragment, an anti-sense nucleic acid, a soluble receptor polypeptide, and a small molecule. The anti-sense nucleic acid may be selected from the group consisting of a RNAi, a ribozyme, an α-anomeric nucleic acid molecule, and a peptide nucleic acid; and the RNAi can comprise a siRNA or a shRNA. The soluble receptor polypeptide can be a soluble ALK3 polypeptide, such as ALK3(24-152)-Fc fusion comprising an amino acid sequence of SEQ ID NO:9.

In both the second and third aspects disclosed herein the use of the compound identified in the second and third aspects treat a melanoma in a subject is also disclosed. The subject may be a non-human mammal or a human.

A fourth aspect discloses a method of inhibiting melanoma formation in a subject at risk for melanoma formation, comprising administering to the subject an effective amount of an inhibitor of a GDF6 activity. The subject may be a non-human mammal or a human. The inhibiting at least one GDF6 activity can comprise apoptosis of a melanoma cell and can comprise reducing downstream signaling from a BMP receptor with which the GDF6 interacts when compared to a suitable control. The reducing downstream signaling from a BMP receptor can comprise reducing SMAD 1/5/8 phosphorylation. The test compound can be selected from the group consisting of an antibody, an antibody fragment, an anti-sense nucleic acid, a soluble receptor polypeptide, and a small molecule. The anti-sense nucleic acid may be selected from the group consisting of a RNAi, a ribozyme, an α-anomeric nucleic acid molecule, and a peptide nucleic acid; and the RNAi can comprise a siRNA or a shRNA. The soluble receptor polypeptide can be a soluble ALK3 polypeptide, such as ALK3(24-152)-Fc fusion comprising an amino acid sequence of SEQ ID NO:9.

In a fifth aspect, the invention is directed to a method of treating a melanoma in a subject, comprising
(a) obtaining a sample from a suspected area of the subject's skin;
(b) determining the presence of GDF6;
(c) obtaining a suitable control;
(d) comparing the presence of GDF6 in the sample of step (b) with the suitable control of step (c);
(e) determining if the subject displays a melanoma, wherein the presence of GDF6 in the sample but not in the suitable control indicates melanoma; and
(f) treating the subject with a GDF6 inhibitor to inhibit GDF6 if the subject has melanoma. The subject may be a non-human mammal or a human. The inhibiting of GDF6 activity can cause apoptosis of a melanoma cell and can reduce downstream signaling from a BMP receptor with which the GDF6 interacts when compared to a suitable control. The reducing downstream signaling from a BMP receptor can reduce SMAD1/5/8 phosphorylation. The test compound can be selected from the group consisting of an antibody, an antibody fragment, an anti-sense nucleic acid, a soluble receptor polypeptide, and a small molecule. The anti-sense nucleic acid may be selected from the group consisting of a RNAi, a ribozyme, an α-anomeric nucleic acid molecule, and a peptide nucleic acid; and the RNAi can comprise a siRNA or a shRNA. The soluble receptor polypeptide can be a soluble ALK3 polypeptide, such as ALK3(24-152)-Fc fusion comprising an amino acid sequence of SEQ ID NO:9.

### BRIEF DESCRIPTION OF THE DRAWINGS

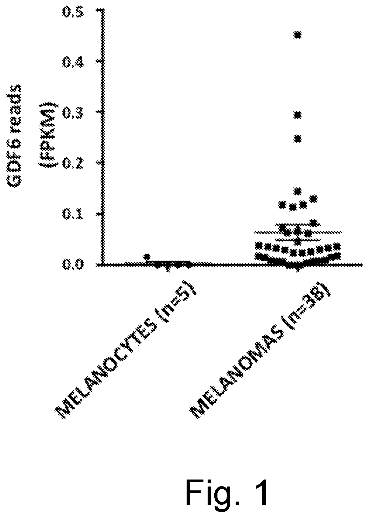

Figure 1 shows that *GDF6* mRNA is present at higher levels in human melanomas as compared to normal melanocytes. Each data point represents the normalized expression of GDF6 in an individual melanocyte sample or patient-derived melanoma. Normalized expression values were obtained through analysis of publicly-available, massively parallel RNA sequencing data.

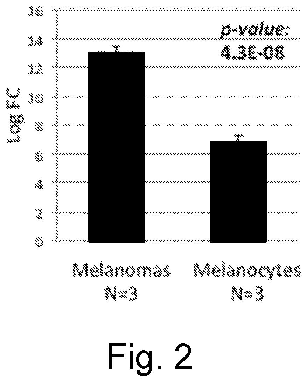

Figure 2 shows that the zebrafish *GDF6* homolog, *gdf6b,* is expressed at higher levels in melanomas as compared to normal melanocytes. Averages of normalized expression values are indicated (±SEM). Quantitative PCR was performed on three melanoma and three melanocyte samples, which were averaged, respectively.

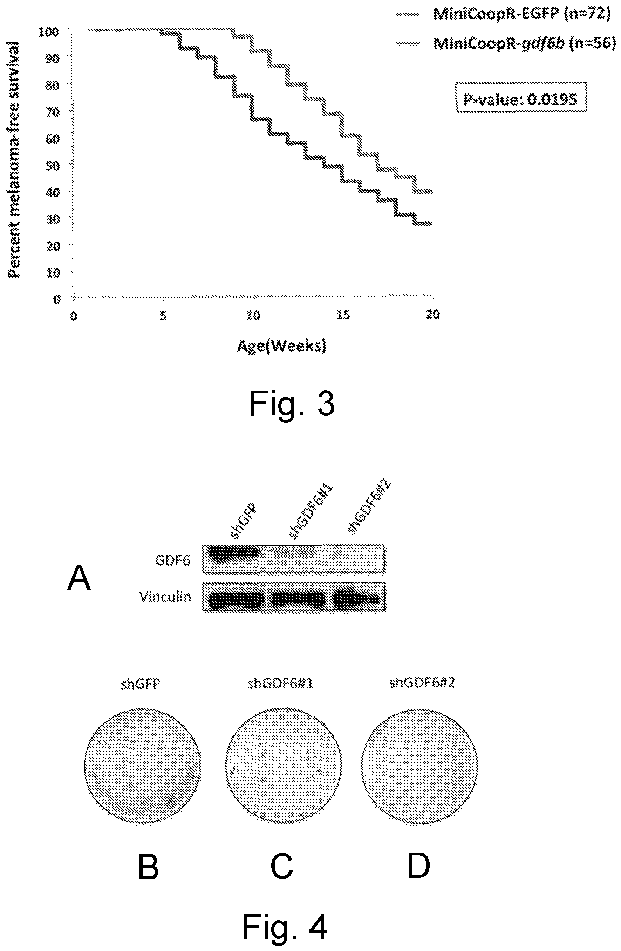

Figure 3 shows that overexpression of *gdf6b* causes acceleration of melanoma onset in zebrafish. Melanocytes expressing the *GDF6* homolog *gdf6b* or *EGFP* were reconstituted in a *BRAFV600E*-positive and *p53*-negative background. Transgenic fish were monitored weekly to determine time of melanoma onset. Tumors expressing *gdf6b* arose earlier than those expressing *EGFP.*

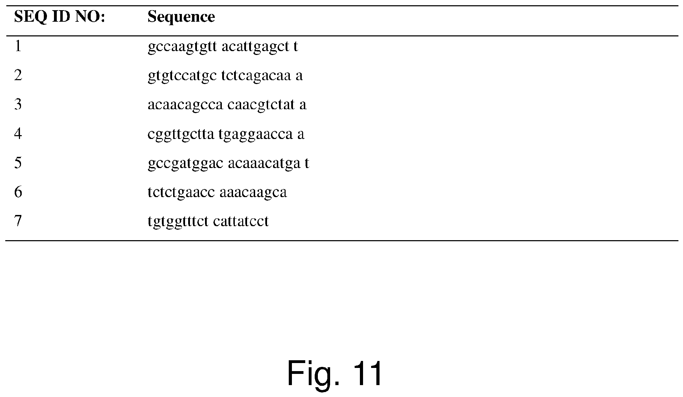

Figures 4A-4D show that knockdown of *GDF6* reduces melanoma cell proliferation in vitro. Fig. 4A shows a Western blot indicating shRNA-mediated knockdown of GDF6 protein (lanes "shGDF6#1" targeting (SEQ ID NO:1; SEQ ID NOs:1-7 sequences can be found in Fig. 11) and "shGDF6#2 (targeting SEQ ID NO:2)) as compared to a vinculin control in A375 melanoma cells, as well as compared to *EGFP* (targeting SEQ ID NO:3)-treated cells (lane "shGFP"). Figs. 4B-4D show clonogenic growth assays of control cells treated with shRNA targeting *EGFP* (Fig. 4B) and cells treated with shRNAs targeting *GDF6* (Fig. 4C, treated with shGDF6#1, and Fig. 4D, treated with shGDF6#2). Crystal violet staining indicates far fewer colonies in plates treated with shRNAs targeting GDF6 (Figs. 4C and 4D).

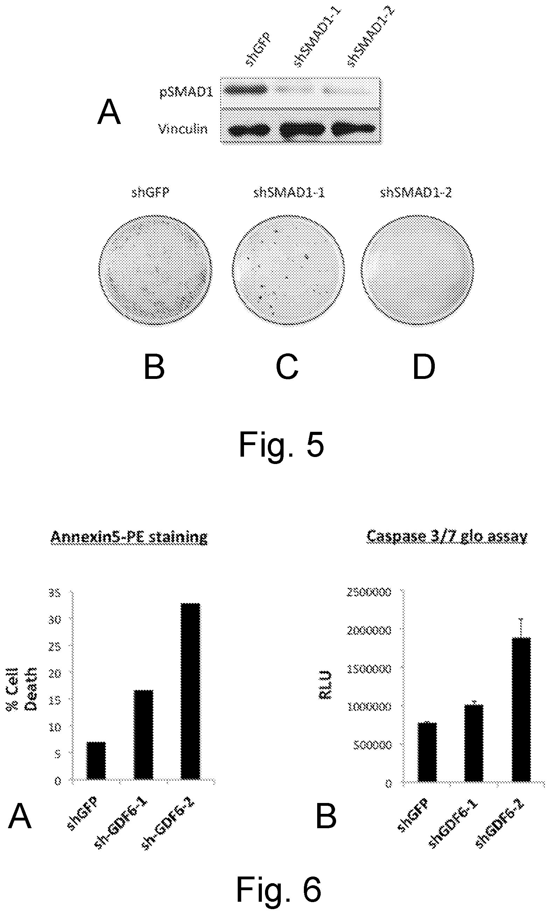

Figures 5A-5D show that knockdown of *SMAD1* reduces melanoma cell proliferation in vitro. Fig. 5A shows a Western blot indicating shRNA-mediated knockdown of SMAD1 protein (lanes "shSMAD1-1" (targeting SEQ ID NO:4) and "shSMAD1-2" (targeting SEQ ID NO:5) as compared to a vinculin control in A375 melanoma cells, as well as compared to *EGFP* (SEQ ID NO:3)-treated cells (lane "shGFP"). Figs. 5B-5D show clonogenic growth assays of control cells treated with shRNA targeting *EGFP* (Fig. 5B) and cells treated with shRNAs targeting *SMAD1* (Fig. 5C, treated with shSMAD1-1, and Fig. 5D, treated with shSMAD1-2). Crystal violet staining indicates far fewer colonies in plates treated with shRNAs targeting *SMAD1*/*5*/*8.* Knockdown of *SMAD1*/*5*/*8* shows the same phenotype as does knockdown of *GDF6,* suggesting that *GDF6* may be acting through *SMAD1*/*5*/*8* to regulate proliferation of melanoma cells.
Figures 6A-6B show that knockdown of *GDF6* causes increased melanoma cell apoptosis. A375 melanoma cells were treated with shRNAs targeting *GDF6* (treated with shGDF6-1 (targeting SEQ ID NO:1) and sh-GDF6-2 (targeting SEQ ID NO:2)) or a control shRNA targeting *EGFP* (targeting SEQ ID NO:3) (shGFP). Knockdown cells were monitored for Annexin5 staining (Fig. 6A) and Caspase 3/7 cleavage (Fig. 6B), both of which are markers for apoptosis. Knockdown of *GDF6* caused elevated apoptosis relative to control cells.

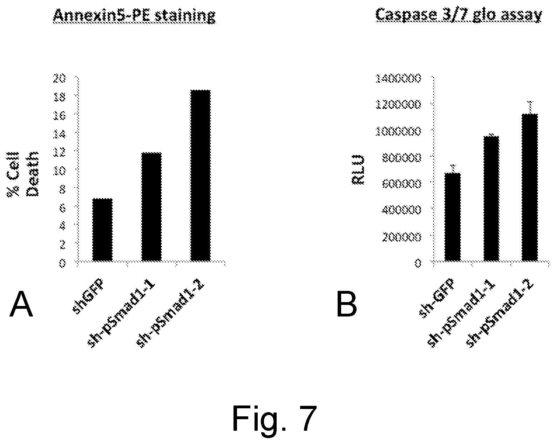

Figures 7A-7B show that knockdown of *SMAD1*/*5*/*8* causes increased melanoma cell apoptosis. A375 melanoma cells were treated with shRNAs targeting *SMAD1*/*5*/*8* (treated with shSMAD1-1 (targeting SEQ ID NO:4) and shSMAD1-2 (targeting SEQ ID NO:5) or a control shRNA targeting *EGFP* (targeting SEQ ID NO:3) (shGFP). Knockdown cells were monitored for Annexin5 staining (Fig. 7A) and Caspase 3/7 cleavage (Fig. 7B), both of which are markers for apoptosis. Knockdown of *SMAD1*/*5*/*8* caused elevated apoptosis relative to control cells.

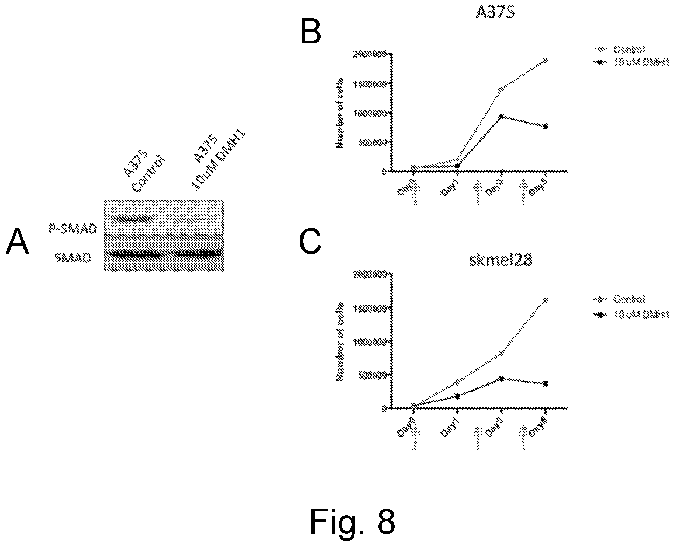

Figures 8A-8C show that inhibition of the BMP pathway signaling affects melanoma cell growth. A375 melanoma cells were treated with 10µM DMH1, a BMP pathway inhibitor, or with control media lacking this inhibitor. Fig. 8A shows a Western blot showing a reduction in BMP signaling (lane A375 10µM DMH1), as indicated by a reduced level of phospho-SMAD1 relative to total SMAD protein. Figs. 8B and 8C show growth curves of A375 and SKMEL28 melanoma cells treated with DMH1 as measured by direct cell counting. Cells treated with DMH1 grew more slowly than did control cells.

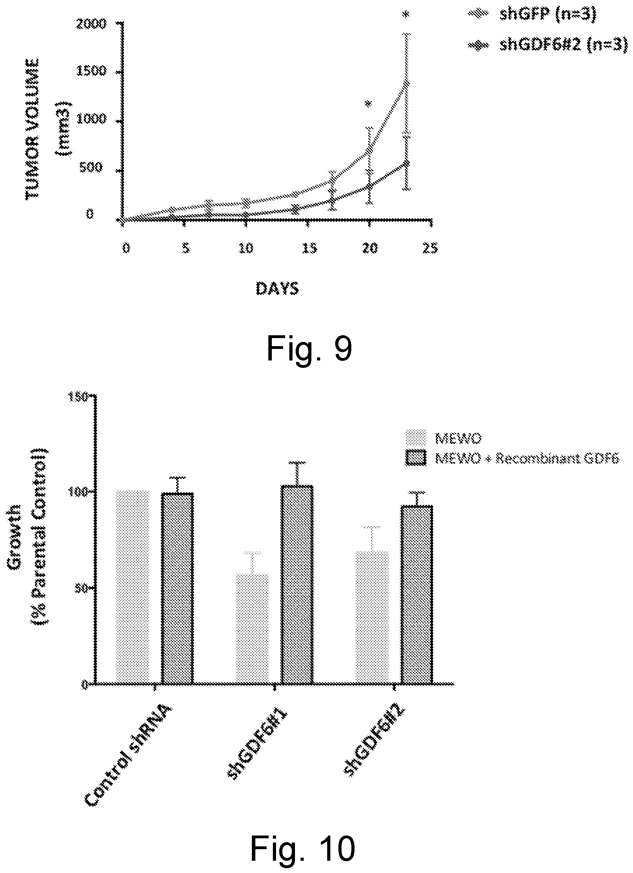

Figure 9 shows that knockdown of *GDF6* reduces melanoma growth in vivo. A375 melanoma cells were treated with a shRNA targeting *GDF6* (shGDF6#2, targeting SEQ ID NO:2) or a control shRNA targeting *EGFP* (shGFP, targeting SEQ ID NO:3). Knockdown cells were xenotransplanted into nude mice, and tumor volume was monitored. Cells subjected to *GDF6* knockdown formed tumors more slowly than control cells.
Figure 10 shows that following *GDF6* knockdown, proliferation of melanoma cells can be restored by supplementing media with recombinant GDF6 protein. MeWo melanoma cells were treated with shRNAs targeting *GDF6* (shGDF6#1, in this case targeting SEQ ID NO:6 and shGDF6#2, in this case targeting SEQ ID NO:7) or a control shRNA targeting *EGFP* (shGFP, targeting SEQ ID NO:3). When recombinant GDF6 protein was added to culture media, proliferation of *GDF6*-knockdown cells was restored. These data indicate that GDF6 can act as a soluble, extracellular protein to promote the growth of melanoma cells.
Figure 11 shows the sequences for SEQ ID NOs:1-7.

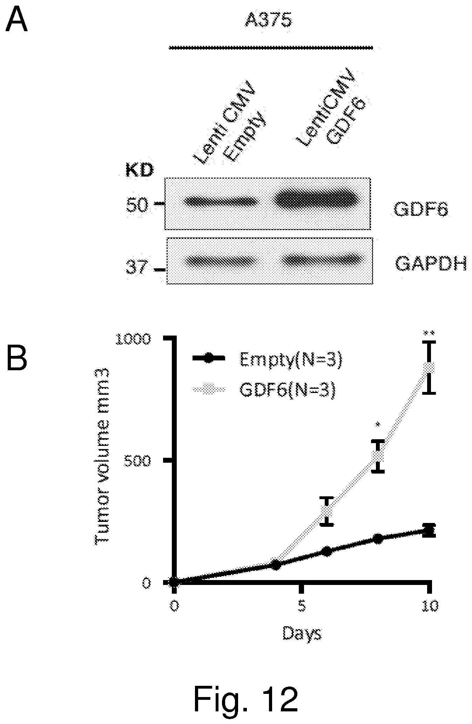

Figures 12A and 12B show that *GDF6* over expression accelerates melanoma formation in mouse xenografts. Fig. 12A shows that *GDF6* expression is upregulated (right lane, "Lenti CMV GDF6") when compared to empty vector (left lane, "Lenti CMV Empty"); also shown is glyceraldehyde 3-phosphate dehydrogenase (GAPDH) expression. Fig. 12B shows that when GDF6 is overexpressed, melanoma formation is accelerated (squares) when compared to control cells containing empty vector (circles).

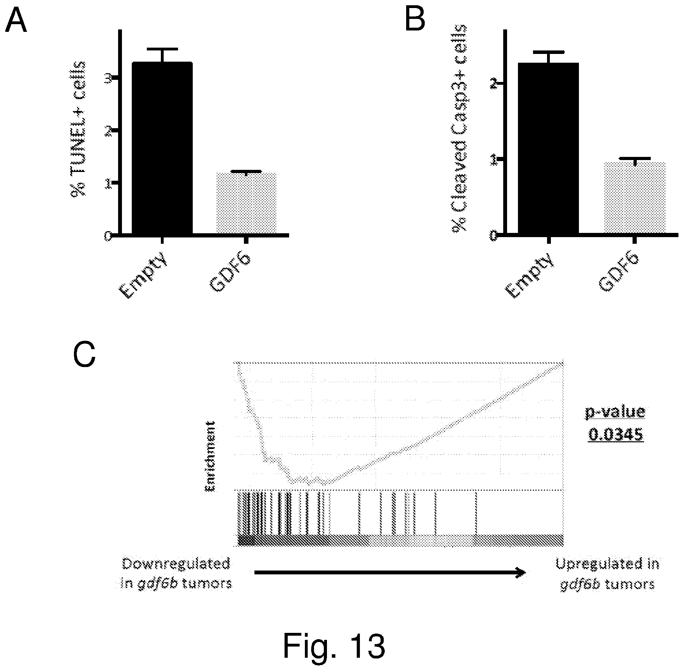

Figures 13A-13C show that over expression of *GDF6* reduces basal levels of cell death in mouse melanoma xenografts. Fig. 13A shows the quantification of tissue staining in mouse melanoma xenografts using TUNEL staining, while Fig. 13B shows the quantification of tissue staining in mouse melanoma xenografts for cleaved caspase 3 ("Empty" means cells transfected with empty vector, while "GDF6" indicates cells transfected with *GDF6* to over express *GDF6*). Fig. 13C shows that *GDF6* ortholog overexpressing-melanomas negatively correlate with a pro-apoptotic signature.

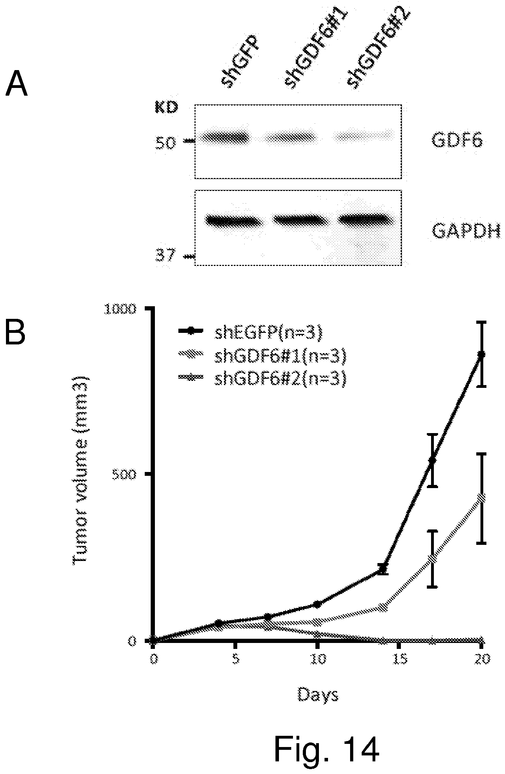

Figures 14A-14D show that knocking down either *GDF6* or *SMAD1* abrogates melanoma growth in mouse xenografts *in vivo.* Fig. 13A shows that using shRNA targeting *GDF6* (shGDF6#1 and shGDF6#2) diminishes expression of GDF6 protein when compared to control (shGFP-treated) cells, while not having an effect on expression of an internal control, GAPDH. Fig. 13B shows that tumor volumes in xenografts decrease with the degree of *GDF6* knockdown. shEGFP, circles; shGDF6#1, squares; shGDF6#2, triangles. Fig. 13C shows that using shRNA targeting *SMAD1* (shSMAD1#1 and shSMAD1#2) decreases expression of SMAD1 protein when compared to control shRNA (shGFP)-treated cells; *GDF6* shRNA treatment did not have an effect on expression of an internal control, GAPDH when compared to GAPDH expressed in control-treated cells.. Fig. 13D shows that tumor volumes in xenografts decrease when *SMAD1* expression is inhibited. shEGFP, circles; shSMAD1#1, squares.

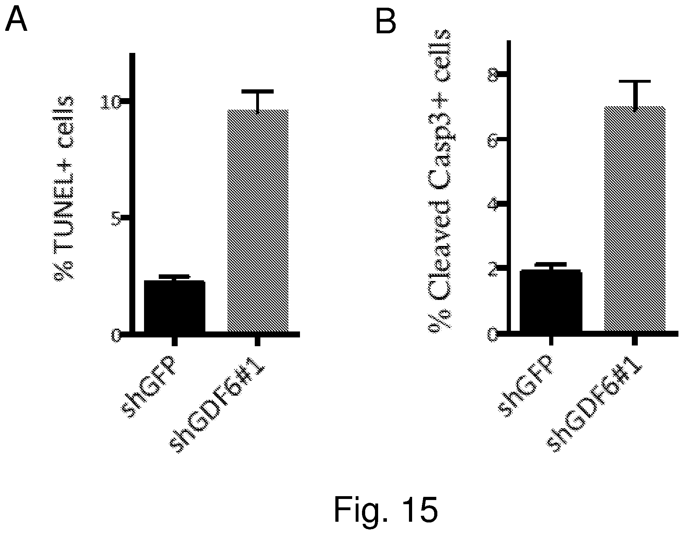

Figures 15A and 15B show that *GDF6* knockdown induces cell death in mouse melanoma xenografts *in vivo.* Fig. 15A shows the percent of cells stained by the TUNEL technique when *GDF6* expression is knocked down (shGDF6#1) compared to cells transfected with the control shRNA, shGFP. Fig. 15B shows the percent of cells stained for cleaved caspase 3 when *GDF6* expression is knocked down (shGDF6#1) compared to cells transfected with the control shRNA, shGFP.

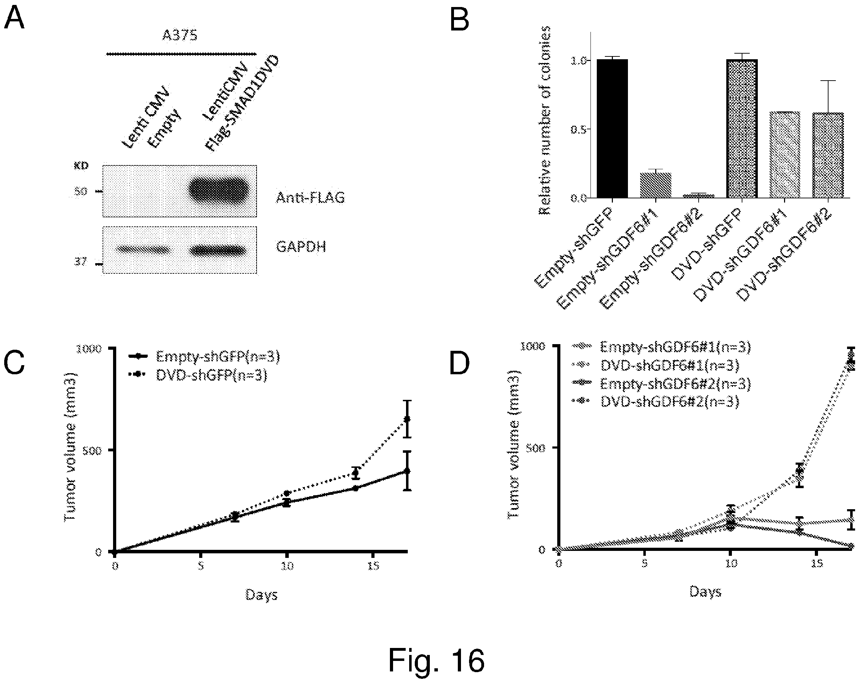

Figures 16A-16D show that expression of a *SMAD1* phosphomimic (SMAD1DVD) can rescue clonogenic potential of melanoma cells after *GDF6* knockdown *in vitro* and *in vivo.* Fig. 16A shows the expression of a FLAG-tagged SMAD1 phosphomimic (Lenti CMV Flag-SMADIDVD) compared to empty vector control cells (Lenti CMV Empty); also shown is GAPDH expression. Fig. 16B shows the relative number of colonies formed in a clonogenic assay applied to control cells and *SMAD1* phosphomimic-expressing cells treated with the indicated shRNAs. "Empty" means cells transfected with an empty vector and then treated with the shRNAs shown. "DVD" means cells transfected with the *SMAD1* phosphomimic and then treated with the shRNAs shown. Fig. 16C shows tumor volume in xenograft melanomas in mice over time, comparing "Empty-shGFP"- to "DVD-shGFP"-treated cells. Fig. 16D also shows tumor volume in xenograft melanomas in mice over time, comparing "Empty-shGDF6#1" (lighter circles, solid line), "DVD-shGDF6#1" (lighter circles, dashed line), "Empty-shGDF6#2" (darker circles, solid line), and "DVD-shGDF6#2 (darker circles, dashed line).

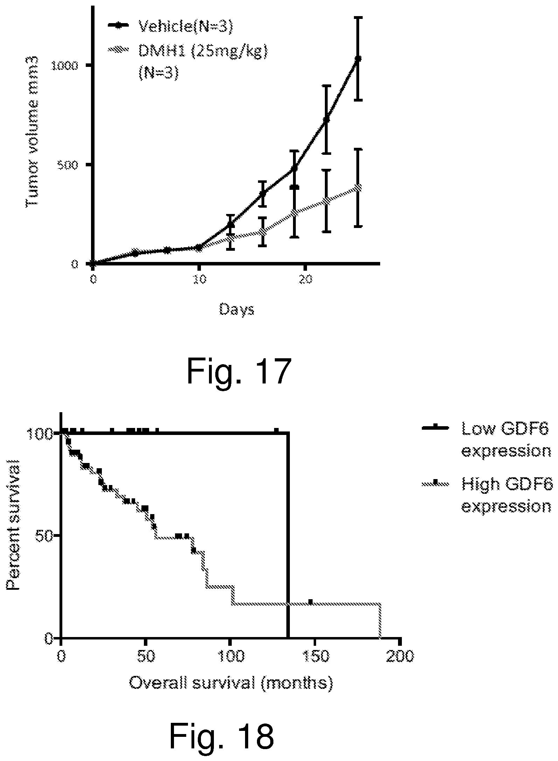

Figure 17 shows that a BMP pathway small molecule inhibitor, DMH1, abrogates melanoma growth in mouse xenografts *in vivo* as assayed by tumor volumes over time. Circles, vehicle only; squares, DMH1 inhibitor.
Figure 18 shows that *GDF6* expression correlates with poor melanoma patient survival.

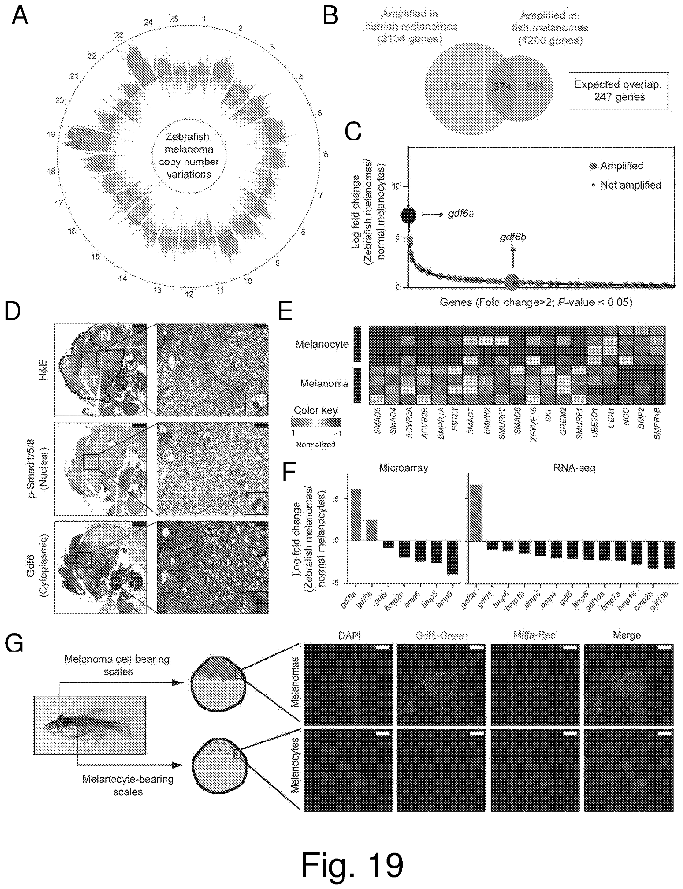

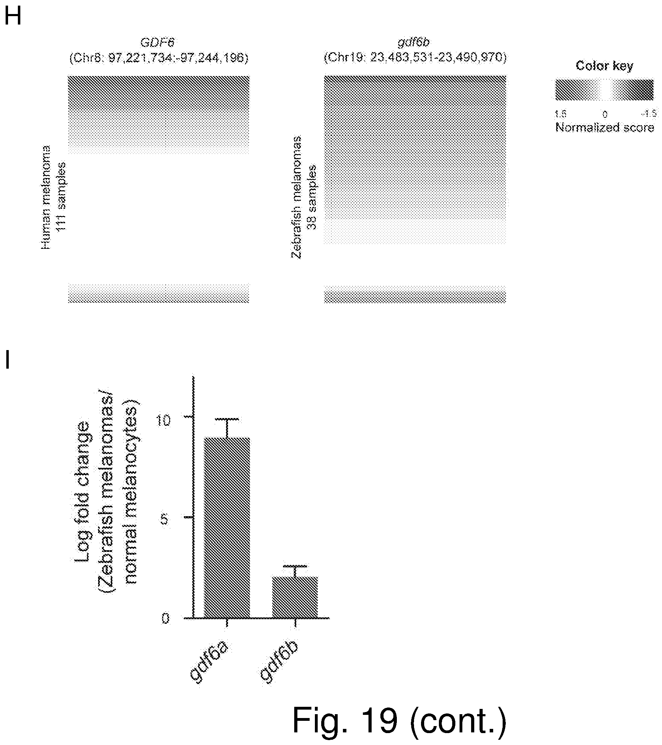

Figures 19A-19I show that *GDF6* is amplified and, along with BMP signaling, upregulated in melanomas. Fig. 19A shows CNVs of zebrafish melanomas across 25 chromosomes. JISTIC G-scores (pale red = amplifications; pale blue = deletions) and -log10-transformed Q-values (Q-value ≤ 0.25; bolded lines) are displayed. Dotted circles represent - log10-transformed Q-value of 0 (center) and 11 (outer: amplification; inner: deletion). Fig. 19B shows orthologous genes significantly amplified in human and zebrafish melanomas (from 10380 human-zebrafish gene pairs; hypergeometric test, P-value: 2.0e⁻¹⁵). Fig. 19C shows the log2-transformed fold changes of genes upregulated in zebrafish melanomas as compared to melanocytes. Orthologous genes recurrently amplified in zebrafish and human melanomas are indicated (red). *gdf6a* and *gdf6b* (big dots) are indicated. Fig. 19D shows transverse sections of a *Tg(mitfa:BRAF(V600E));p53*^{*-*/*-*} zebrafish bearing an invasive melanoma in the dorsal musculature. Top, hematoxylin and eosin staining (T, tumor; N, normal tissue). Middle, phospho-SMAD1/5/8 staining. Bottom, GDF6 staining. Left, scale bar, 500 µm. Right, scale bar, 50 µm. Fig. 19E shows a heat map of BMP pathway gene expression in zebrafish melanomas compared to melanocytes. Human orthologs of fish genes are listed. Fig. 19F shows the log2-transformed fold change of BMP ligand expression in zebrafish melanomas as compared to melanocytes (p-value<0.05). Fig. 19G shows immunostaining of *Tg(mitfa:BRAF(V600E));p53*^{*-*/*-*} zebrafish scales bearing melanoma cells (top) or normal melanocytes (bottom). DAPI (blue), Gdf6 (green), Mitfa (red), and a merged image of all channels are shown. Scale bar, 10 µm. Fig. 19H, left, shows a heat map showing copy number of the human *GDF6* locus across 111 human melanomas (y-axis). Right, heatmap showing copy number of the zebrafish *gdf6b* locus across 38 zebrafish melanomas (y-axis). Red indicates amplification, blue indicates deletion. Fig. 19I shows the log2-transformed fold change of *gdf6a* and *gdf6b* expression in zebrafish melanomas as compared to melanocytes (y-axis) as determined by qRT-PCR.

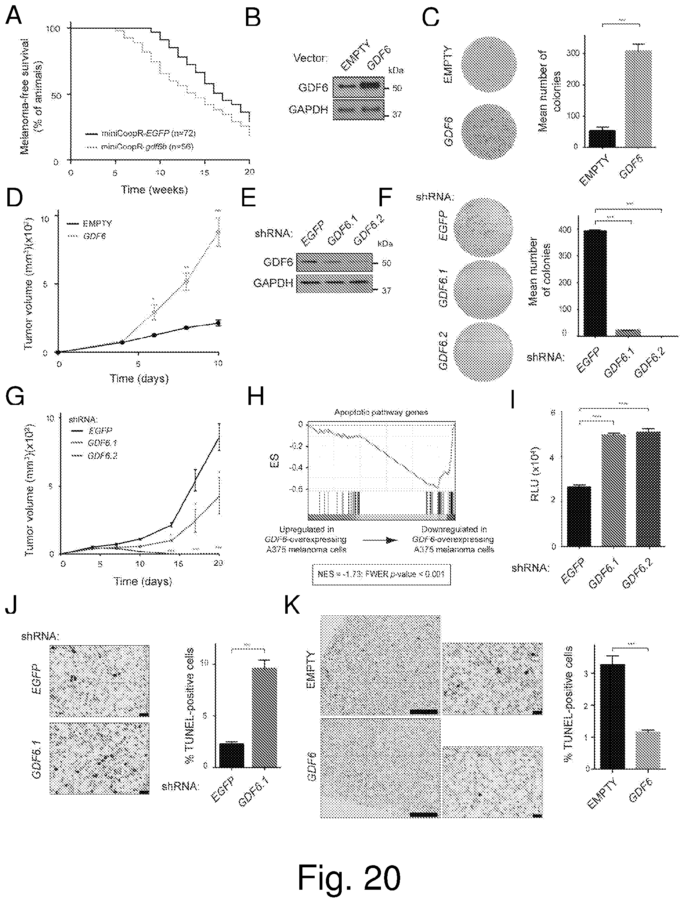

Figures 20A-20K shows *GDF6* modulation affects growth and survival of melanoma cells. Fig. 20A shows melanoma-free survival curves of *Tg(mitfa:BRAF(V600E));p53*^{*-*/}*⁻;mitfa*^{*-*/*-*} zebrafish injected with miniCoopR-*gdf6b* or miniCoopR-EGFP (*P* = 0.02, Mantel-Cox log rank test). Fig. 20B shows immunoblots of A375 melanoma cells overexpressing *GDF6.* Fig. 20C shows soft agar assay with A375 cells overexpressing *GDF6* or empty vector control. Error bars indicate s.e.m.; *n*=3. Fig. 20D shows tumor formation in mice injected with A375 cells overexpressing *GDF6* or empty vector control. Error bars indicate s.e.m.; *n*=3. Fig. 20E shows immunoblots of A375 melanoma cells expressing *shEGFP, shGDF6.1* or *shGDF6.2.* Fig. 20F shows a colony formation assay with A375 cells expressing *shEGFP, shGDF6.1* or *shGDF6.2.* Error bars indicate s.e.m.; n=3. Fig. 20G shows tumor formation in mice injected with A375 cells expressing *shEGFP, shGDF6.1* or *shGDF6.2.* Error bars indicate s.e.m.; *n*=3. Fig. 20H shows a graphical representation of the rank-ordered gene list stratified based on expression in A375 melanoma cells overexpressing *GDF6* as compared to empty vector control. By GSEA, an apoptotic gene set is negatively enriched in *GDF6*-overexpressing melanomas. ES, enrichment score, NES, normalized enrichment score. Fig. 20I shows caspase3/7 activity in A375 cells expressing *shEGFP, shGDF6.1* or *shGDF6.2.* Error bars indicate s.e.m.; *n*=3. RLU, relative luciferase units. Fig. 20J shows TUNEL-staining of mouse xenografts of A375 cells expressing *shEGFP* or *shGDF6.1.* Scale bar, 25µm. Error bars indicate s.e.m.; *n*=100 fields. Fig. 20K shows TUNEL-staining of mouse xenografts of A375 cells with empty vector (top) or *GDF6* overexpression (bottom). Scale bar, 100µm (left), 25µm (right). Error bars indicate s.e.m.; *n*=100 fields. Statistical analyses were performed with a two-tailed Student's *t*-test, **P*< 0.05, ***P*< 0.01, ****P*< 0.001.

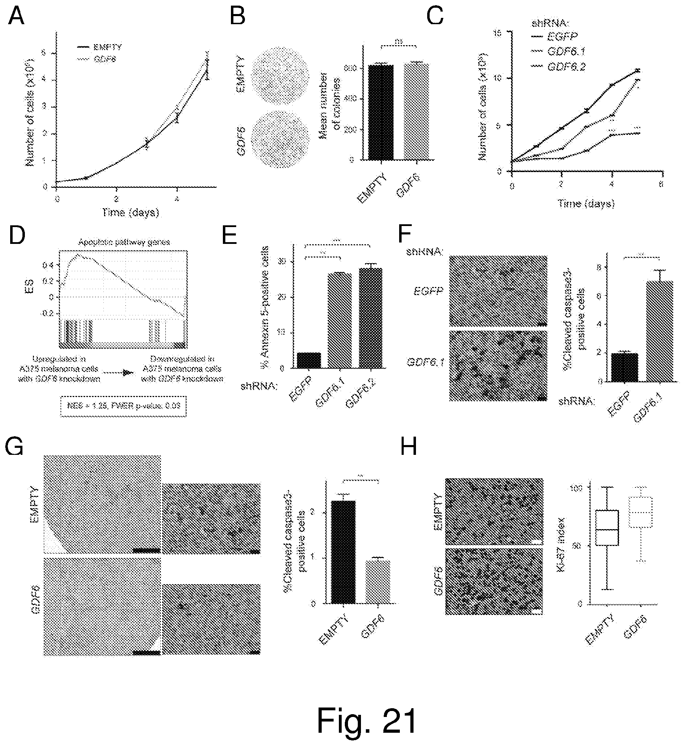

Figures 21A-21H show the effects of *GDF6* modulation on A375 melanoma cell growth and survival. Fig. 21A shows growth curves of A375 cells overexpressing *GDF6* or empty vector control. Error bars indicate s.e.m.; *n*=3. Fig. 21B, left, shows representative images of colony formation assay with A375 cells overexpressing *GDF6* or empty vector control. Fig. 21B, right, shows quantification of colony formation assay. Error bars indicate s.e.m.; *n*=3. (C) Growth curves of A375 cells expressing *shEGFP, shGDF6.1* or *shGDF6.2.* Error bars indicate s.e.m.; *n*=3. Fig. 21D shows a graphical representation of the rank-ordered gene list stratified based on expression in A375 cells expressing *shGDF6.1* as compared to control A375 cells expressing *shEGFP.* GSEA indicates expression of an apoptotic gene set is enriched upon *GDF6* knockdown. Fig. 21E shows flow cytometry analysis of annexinV-positivity of A375 cells expressing *shEGFP, shGDF6.1* or *shGDF6.2.* Error bars indicate s.e.m.; *n*=3. Fig. 21F shows cleaved caspase3-stained tumors from mouse xenografts of A375 cells expressing *shEGFP* (top) or *shGDF6.1* (bottom). Scale bar, 25µm. Error bars indicate s.e.m.; *n*=100 fields. Fig. 21G shows cleaved caspase3-stained tumors from mouse xenografts of A375 cells with empty vector (top) or *GDF6* overexpression (bottom). Scale bars, 100 µm (left), 25µm (right). Error bars indicate s.e.m.; *n*=100 fields. Fig. 21H shows Ki67-stained tumors from mouse xenografts of A375 cells overexpressing *GDF6* or empty vector control. Scale bar, 25µm. Two-tailed Student's *t*-test *P*-value< 0.001; *n*=100 fields. Statistical analyses were performed with a two-tailed Student's *t*-test, **P*< 0.05, ***P*< 0.01, ****P*< 0.001

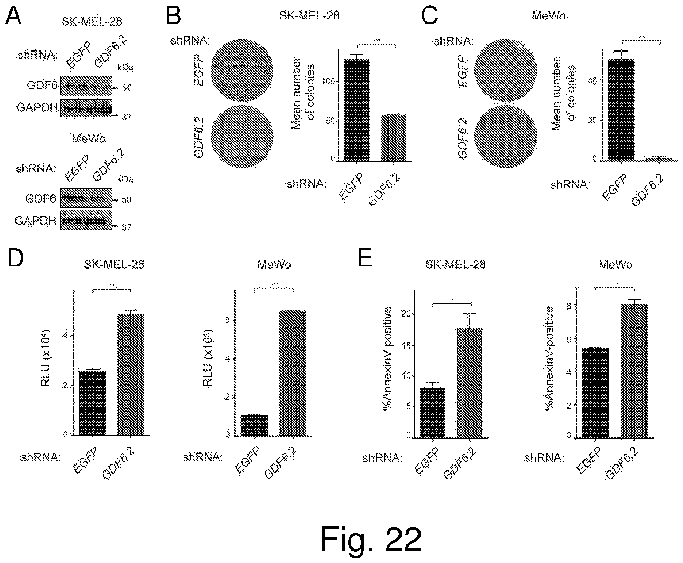

Figures 22A-22E show the effects of *GDF6* modulation on SK-MEL-28 and MeWo melanoma cell growth and survival. Fig. 22A shows immunoblots of SK-MEL-28 melanoma cells (top) and MeWo melanoma cells (bottom) expressing *shEGFP* or *shGDF6.2.* Fig. 22B shows colony formation assay with SK-MEL-28 cells expressing *shEGFP* or *shGDF6.2.* Error bars indicate s.e.m.; *n*=3. Fig. 22C shows colony formation assay with MeWo cells expressing *shEGFP* or *shGDF6.2.* Error bars indicate s.e.m.; *n*=3. Fig. 22D shows caspase3/7 activity in SK-MEL-28 cells (left) and MeWo cells (right) expressing *shEGFP* or *shGDF6.2.* Error bars indicate s.e.m.; *n*=3. RLU, relative luciferase units. Fig. 22E shows flow cytometry analysis of annexinV-positivity of SK-MEL-28 cells (left) and MeWo cells (right) expressing *shEGFP* or *shGDF6.2.* Error bars indicate s.e.m.; *n*=3. Statistical analyses were performed with a two-tailed Student's *t*-test, **P*< 0.05, ***P*< 0.01, ****P*< 0.001.

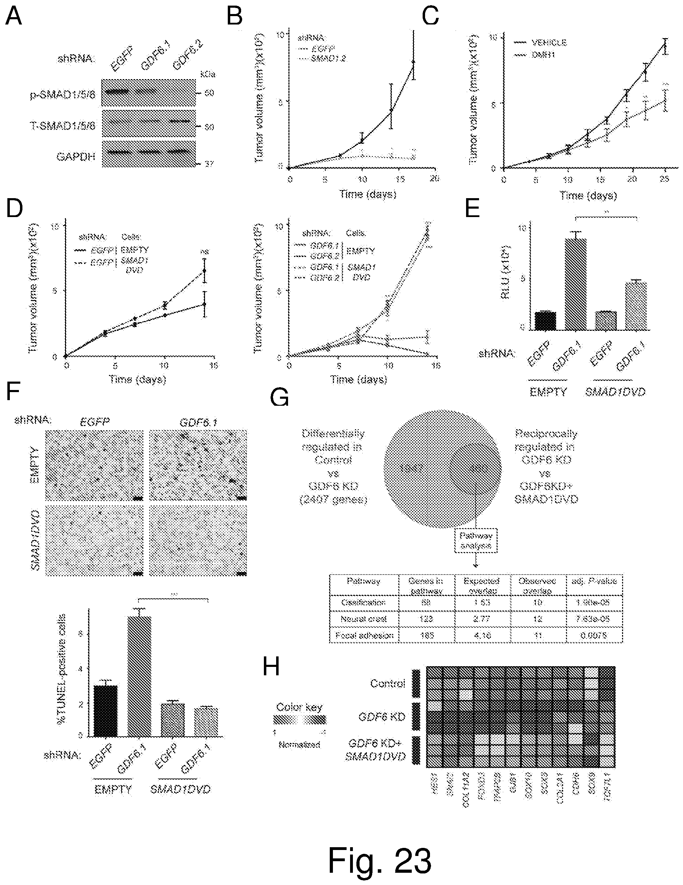

Figures 23A-23H show *GDF6* acts via *SMAD1* to promote melanoma cell survival and modulate neural crest gene expression. Fig. 23A shows immunoblots of A375 melanoma cells expressing *shEGFP, shGDF6.1* or *shGDF6.2.* Fig. 23B shows tumor formation in mice injected with A375 cells expressing *shEGFP* or *shSMAD1.2.* Error bars indicate s.e.m.; *n*=3. Fig. 23C shows tumor formation of A375 cells in mice treated with vehicle control or 25 mg/kg DMH1 every other day. Error bars indicate s.e.m.; *n*=8. Fig. 23D, left, shows tumor formation in mice injected with A375-EMPTY or A375-*SMAD1DVD* cells expressing *shEGFP.* Error bars indicate s.e.m.; *n*=3. Fig. 23D, right, shows tumor formation in mice injected with A375-EMPTY or A375-*SMAD1DVD* cells expressing *shGDF6.1* or *shGDF6.2.* Error bars indicate s.e.m.; *n*=3. Fig. 23E shows caspase3/7 activity in A375-EMPTY and A375-*SMAD1DVD* cells expressing *shEGFP* or *shGDF6.1.* Error bars indicate s.e.m.; *n*=3. RLU, relative luciferase units. Fig. 23F shows TUNEL-staining of mouse xenografts of A375-EMPTY or A375-*SMAD1DVD* cells expressing *shEGFP* or *shGDF6.1.* Scale bar, 25µm. Error bars indicate s.e.m.; *n*=100 fields. Fig. 23G, top, shows genes differentially regulated between A375-EMPTY cells expressing *shEGFP* and A375-EMPTY cells expressing *shGDF6.1* (purple circle) and genes that are reciprocally differentially regulated between A375-EMPTY cells expressing *shGDF6.1* and A375-*SMAD1DVD* cells expressing *shGDF6.1* (green circle). Fig. 23G, bottom, shows pathway analysis with reciprocally regulated genes (minimum overlap ≥ 10 genes; adjusted *P*-value < 0.01). Fig. 23H shows a heat map of neural crest gene expression identified in pathway analysis. Genes essential for survival of neural crest cells and their derivatives are in purple. Statistical analyses were performed with a two-tailed Student's *t*-test, **P*< 0.05, ***P*< 0.01, ****P*< 0.001. ns, not significant.

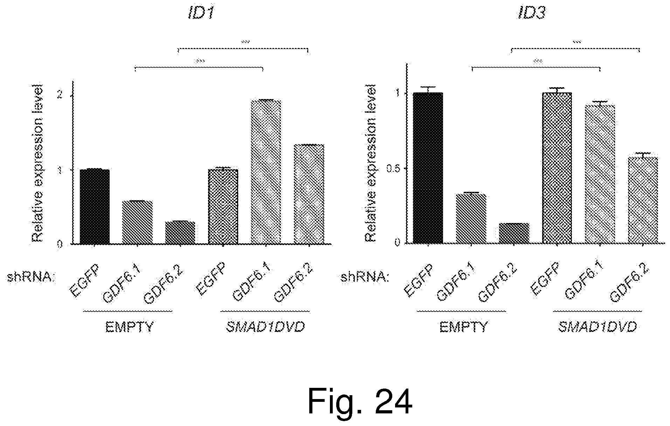

Figure 24 shows *GDF6* and *SMAD1* modulation alter expression of BMP pathway targets *ID1* and *ID3.* qRT-PCR showing relative expression of *ID1* (left) and *ID3* (right) in A375-EMPTY or A375-*SMAD1DVD* cells expressing an shRNA targeting *EGFP* or two independent *GDF6*-targeted shRNAs. Normalization to expression values from A375-EMPTY or A375-*SMAD1DVD* cells expressing an shRNA targeting *EGFP* was performed. Error bars indicate s.e.m.; *n*=3. Statistical analyses were performed with a two-tailed Student's *t*-test, **P*< 0.05, ***P*< 0.01, ****P*< 0.001.

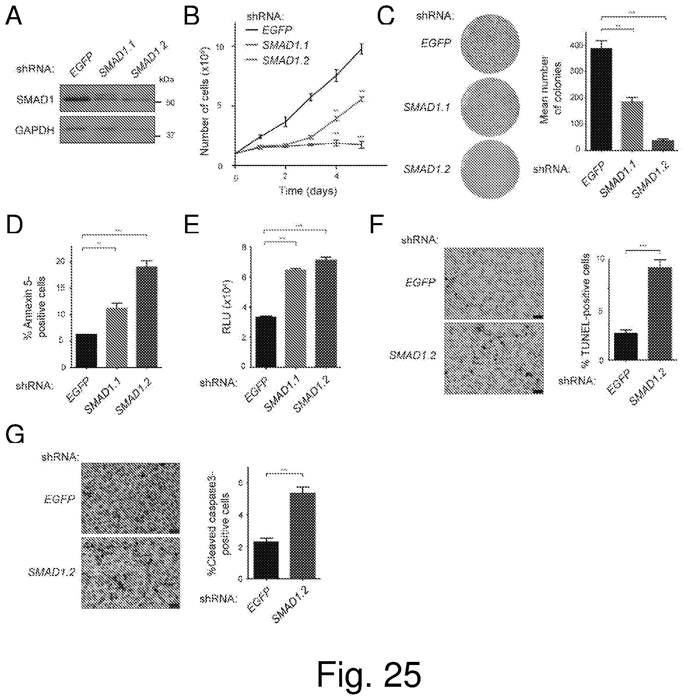

Figures 25A-25G shows that *SMAD1* modulation affects A375 melanoma cell growth and survival. Fig. 25A shows immunoblots of A375 melanoma cells expressing *shEGFP, shSMAD1.1* or *shSMAD1.2.* Fig. 25B shows growth curves of A375 cells expressing *shEGFP, shSMAD1.1* or *shSMAD1.2.* Error bars indicate s.e.m.; *n*=3. Fig. 25C shows a colony formation assay with A375 cells expressing *shEGFP, shSMAD1.1* or *shSMAD1.2.* Error bars indicate s.e.m.; *n*=3. Fig. 25D shows flow cytometry analysis of annexinV-positivity of A375 cells expressing *shEGFP, shSMAD1.1* or *shSMAD1.2.* Error bars indicate s.e.m.; *n*=3. Fig. 25E shows caspase3/7 activity in A375 cells expressing *shEGFP, shSMAD1.1* or *shSMAD1.2.* Error bars indicate s.e.m.; *n*=3. RLU, relative luciferase units. Fig. 25F shows TUNEL-staining of mouse xenografts of A375 cells expressing *shEGFP* (top) or *shSMAD1.2* (bottom). Scale bar, 25µm. Error bars indicate s.e.m.; *n*=100 fields. Fig. 25G shows cleaved caspase3-staining of mouse xenografts of A375 cells expressing *shEGFP* (top) or *shSMAD1.2* (bottom). Scale bar, 25µm. Error bars indicate s.e.m.; *n*=100 fields. Statistical analyses were performed with a two-tailed Student's *t*-test, **P*< 0.05, ***P*< 0.01, ****P*< 0.001.

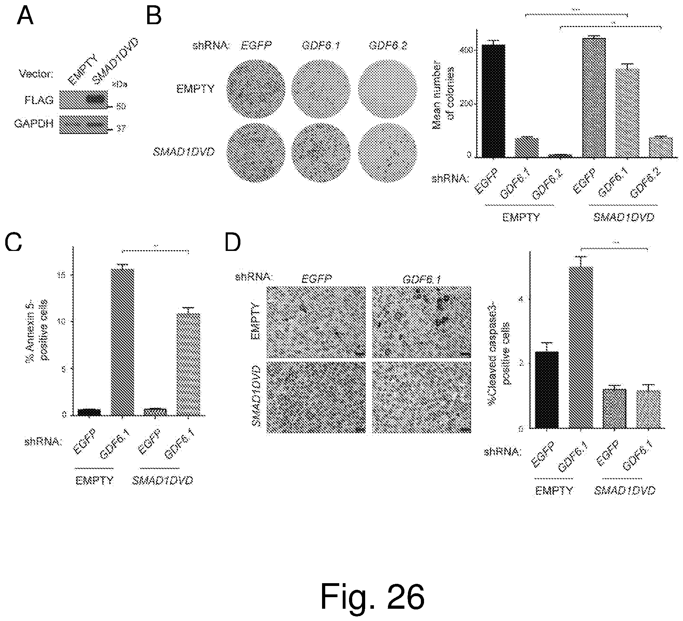

Figures 26A-26D show the activated *SMAD1DVD* variant rescues effects of *GDF6* loss in A375 melanoma cells. Fig. 26A shows immunoblots of A375 cells overexpressing Flag-tagged *SMAD1DVD.* Fig. 26B shows colony formation assay of A375-EMPTY (top) or A375-*SMAD1DVD* (bottom) cells expressing *shEGFP, shGDF6.1* or *shGDF6.2.* Error bars indicate s.e.m.; *n*=3. Fig. 26C shows flow cytometry analysis of annexinV-positivity of A375-EMPTY or A375-*SMAD1DVD* cells expressing *shEGFP* or *shGDF6.1.* Error bars indicate s.e.m.; *n*=3. Fig. 26D shows cleaved caspase3-staining of mouse xenografts of A375-EMPTY or A375-*SMAD1DVD* cells expressing *shEGFP* or *shGDF6.1.* Scale bar, 25µm. Error bars indicate s.e.m.; *n*=100 fields. Statistical analyses were performed with a two-tailed Student's *t*-test, **P*< 0.05, ***P*< 0.01, ****P*< 0.001.

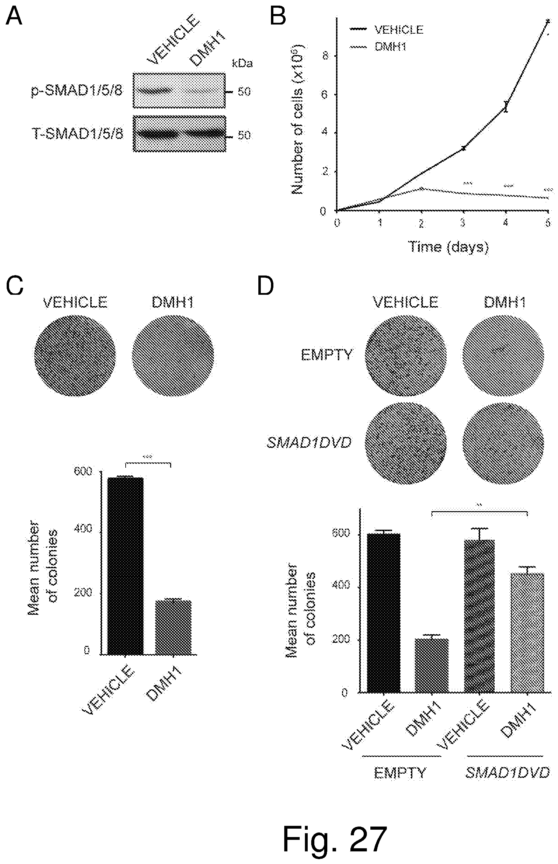

Figures 27A-27D show that the BMP pathway inhibitor DMH1 inhibits A375 melanoma cell growth. Fig. 27A shows immunoblots of A375 melanoma cells treated with 0.1% DMSO (vehicle) or 10µM DMH1 in 0.1% DMSO. Fig. 27B shows growth curves of A375 cells treated with 0.1% DMSO (vehicle) or 10µM DMH1 in 0.1% DMSO. Error bars indicate s.e.m.; *n*=3. Fig. 27C shows a colony formation assay of A375 cells treated with 0.1% DMSO (vehicle) or 10µM DMH1 in 0.1% DMSO. Error bars indicate s.e.m.; *n*=3. Fig. 27D shows a colony formation assay with A375-EMPTY and A375-*SMAD1DVD* cells treated with 0.1% DMSO (vehicle) or 10µM DMH1 in 0.1% DMSO. Error bars indicate s.e.m.; *n*=3. Statistical analyses were performed with a two-tailed Student's *t*-test, **P*< 0.05, ***P*< 0.01, ****P*< 0.001.

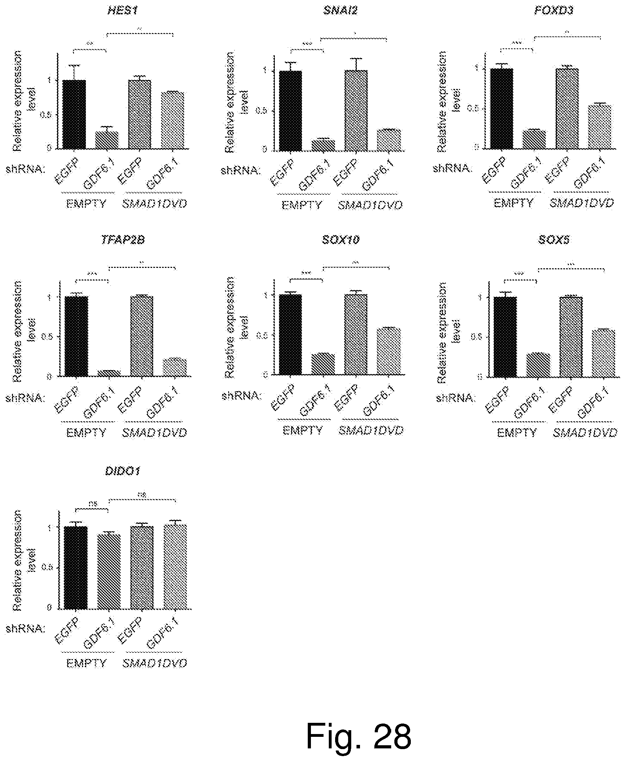

Figure 28 shows *GDF6* knockdown and *SMAD1DVD* alter expression of genes important for survival of neural crest cells and their derivatives. Relative FPKM values from RNA-seq showing expression of genes involved in survival of neural crest cells and their derivatives in A375-EMPTY or A375-*SMAD1DVD* cells expressing an shRNA targeting *EGFP* or the *GDF6*-targeted shRNA *GDF6.1.* Normalization to FPKM values from A375-EMPTY or A375-*SMAD1DVD* cells expressing an shRNA targeting *EGFP* was performed. Of note, *DIDO1,* a BMP target that can promote melanoma cell survival, was not differentially regulated by *GDF6* or *SMAD1* modulation. Error bars indicate s.e.m.; *n*=3. Statistical analyses were performed with a two-tailed Student's *t*-test, **P*< 0.05, ***P*< 0.01, ****P*< 0.001.

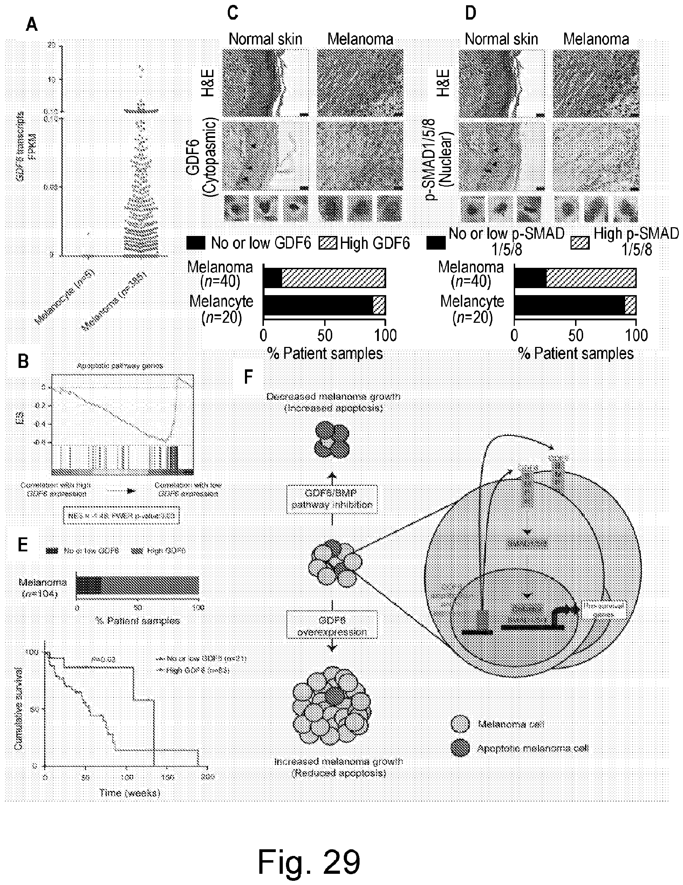

Figures 29A-29F show GDF6 expression and prognostic significance in patient-derived melanomas. Fig. 29A shows *GDF6* transcript FPKM values of normal human melanocytes and melanomas. Fig. 29B shows Graphical representation of the rank-ordered gene list stratified based on *GDF6* expression levels in patient-derived melanomas (TCGA). By GSEA, an apoptotic gene set is negatively correlated with *GDF6* expression in patient-derived melanomas. Fig. 29C shows staining of adjacent normal skin and melanoma sections. Top, hematoxylin and eosin. Middle, GDF6 immunostaining. Melanocytes in normal skin sections are indicated (arrowheads). Scale bar, 25µm. Images of individual cells are shown below. Bottom, percentages of patient samples with no or low, and high GDF6 expression in normal melanocytes and melanomas. Fig. 29D shows staining of adjacent normal skin and melanoma sections. Top, hematoxylin and eosin. Middle, phospho-SMAD 1/5/8 immunostaining. Melanocytes in normal skin sections are indicated (arrowheads). Scale bar, 25µm. Images of individual cells are shown below. Bottom, percentages of patient samples with no or low, and high phospho-SMAD 1/5/8 expression in normal melanocytes and melanomas. Fig. 29E, top, shows percentages of patient samples with no or low, and high GDF6 expression in the melanoma tissue microarray. Fig. 29E, bottom, shows Kaplan-Meier curves showing overall survival of patients with no or low GDF6 expression (blue line) versus high GDF6 expression (red line). Statistical analysis was performed with a Mantel-Cox log rank test. Fig. 29F shows schematic showing locus amplification and expression of GDF6. GDF6 acts via BMP-SMAD1 signaling to promote melanoma cell survival. Levels of GDF6 and BMP signaling modulate tumor growth

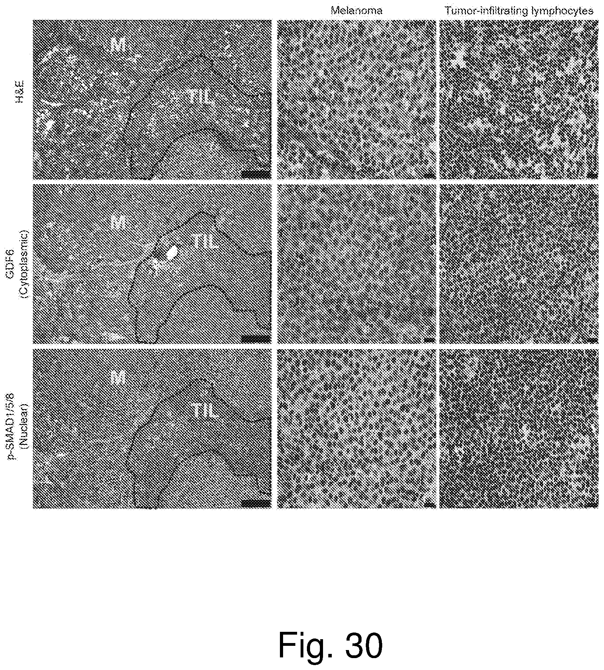

Figure 30 shows GDF6 and p-SMAD1/5/8 expression in a patient melanoma section. Sections of a metastatic human melanoma with tumor-infiltrating lymphocytes (TIL). Top, hematoxylin and eosin staining. Middle, GDF6 staining. Bottom, phospho-SMAD 1/5/8 staining. Left, low magnification. Scale bar, 50 µm. Center, melanoma region, and, right, TIL region. Scale bar, 25 µm.

### DETAILED DESCRIPTION

In searching for melanoma oncogenes, the inventors discovered that the bone morphogenetic protein (BMP) GDF6 plays important roles in melanoma initiation and maintenance. GDF6 (also known as BMP13), a member of the TGF-beta superfamily of proteins, is a secretory factor known to be involved in skeletal development and repair. Loss-of-function mutation in GDF6, and its paralogs GDF5 and GDF7, are associated with rare skeletal diseases, including Klippel-Feil syndrome (Storm et al. (1994) Nature 369:639-643; Wolfman et al. (1997) J Clin Invest 100:321-330; Aspenbertg and Forslund (1999) Acta Orthop Scand 70:51-54; Tassabehji et al. (2008) Hum Mutat 29:1017-1027). In animal studies, GDF6 has been shown to be involved in retinal and skeletal development (Sidi et al., (2003) Proc. Natl. Acad. Sci. U.S.A. 18:3315-20; Settle (2003) Dev Biol 254:116-1130; Hanel and Hensey (2006) BMC Devl Biol 6:43)).

The *GDF6* gene is recurrently amplified in human melanomas, and the GDF6 protein is highly expressed in nearly all melanomas. Knockdown of GDF6 leads to apoptosis of melanoma cells in culture, and xenotransplanted melanomas with GDF6 knocked down grow poorly as compared to control xenotransplants. GDF6 is a secreted protein, and exogenously provided GDF6 enables cells with GDF6 knockdown to survive. Lastly, non-transformed, normal cells with GDF6 knockdown survive showing no adverse effects. Taken together, the inventors' data indicate that melanoma cells produce GDF6 to enable their own survival. This pro-survival signaling is tumor-specific as normal melanocytes do not express GDF6, and normal cells that do express GDF6 are insensitive to GDF6 knockdown. These data identify GDF6 as an attractive drug target, as therapies targeting GDF6 would be predicted to selectively kill melanoma cells.

*GDF6* has hallmarks of being an important melanoma gene. It is copy number amplified and highly expressed in tumors but not normal tissue. It is sufficient to accelerate melanoma onset, and its knockdown stops proliferation of melanoma cells. All melanoma cell lines and a majority of tumor samples analyzed to date show GDF6 expression, so a large fraction of melanomas may be targetable by anti- GDF6 therapy. Furthermore, the *GDF6* locus is recurrently amplified in breast, ovarian and neural cancers, and GDF6 may prove to be a viable target in these tumor types as well. The mature form of GDF6 is a soluble, extracellular ligand, so therapies such as the proposed antibody-based approach would not need to enter cells in order to target it.

So that the invention may be more readily understood, certain terms are first defined.

### Definitions:

A "melanoma-related molecule," as used herein, means a GDF6 molecule and a BMP signaling pathway molecule, such as SMAD1/5/8 or activin receptor-like kinase-3 (ALK3).

Various methodologies of the instant invention include steps that involve comparing a value, level, feature, characteristic, property, etc. to a "suitable control", referred to interchangeably herein as an "appropriate control". A "suitable control" or "appropriate control" is any control or standard familiar to one of ordinary skill in the art useful for comparison purposes. In one embodiment, a "suitable control" or "appropriate control" is a value, level, feature, characteristic, property, etc. determined prior to performing a methodology, as described herein. For example, a transcription rate, mRNA level, translation rate, protein level, biological activity, cellular characteristic or property, genotype, phenotype, etc. can be determined prior to performing a methodology. In another embodiment, a "suitable control" or "appropriate control" is a value, level, feature, characteristic, property, etc. determined in a cell or organism, e.g., a control or normal cell or organism, exhibiting, for example, normal traits. In yet another embodiment, a "suitable control" or "appropriate control" is a predefined value, level, feature, characteristic, property, etc.

The term "contacting" (i.e., contacting a cell e.g. a cell, with a compound) includes incubating the compound and the cell together in vitro (e.g., adding the compound to cells in culture) as well as administering the compound to a subject such that the compound and cells of the subject are contacted in vivo. The term "contacting" does not include exposure of cells to a melanoma-related molecule modulator that may occur naturally in a subject (i.e., exposure that may occur as a result of a natural physiological process).

As used herein, the term "test compound" refers to a compound that has not previously been identified as, or recognized to be, a modulator of the activity being tested. The term "library of test compounds" refers to a panel comprising a multiplicity of test compounds.

It is disclosed that small molecules can be used as test compounds. The term "small molecule" is a term of the art and includes molecules that are less than about 7500, less than about 5000, less than about 1000 molecular weight or less than about 500 molecular weight. In one embodiment, small molecules do not exclusively comprise peptide bonds. It is further disclosed that small molecules are not oligomeric. Exemplary small molecule compounds which can be screened for activity include, but are not limited to, peptides, peptidomimetics, nucleic acids, carbohydrates, small organic molecules (e.g., Cane et al. 1998. Science 282: 63), and natural product extract libraries. It is also disclosed that the compounds are small, organic non-peptidic compounds. It is further disclosed that a small molecule is not biosynthetic. For example, a small molecule is preferably not itself the product of transcription or translation.

### Screening assays

The methods disclosed herein are suitable for use in methods to identify and/or characterize potential pharmacological agents, e.g. identifying new pharmacological agents from a collection of test substances and/or characterizing mechanisms of action and/or side effects of known pharmacological agents. Cell-free as well as cell-based assays are contemplated. In the cell-based assays disclosed herein, at least one component important or crucial to the function of the melanoma cell (e.g., GDF6, BMP signaling (including SMAD 1/5/8)) is assayed in vitro in the presence of a test compound and the ability of the test compound to bind to or modulate the activity of the component is determined. An effect on the component is an indicator of the pharmacological potential of the compound. Expression of BMP pathway molecules can be assayed. BMP pathway molecules can be assayed for expression and/or any biochemical activity characteristic of said component. Transcription factor expression and phosphorylation state can be assayed, such as SMAD 1/5/8. Transcription factor activity can be assayed. Transcription-based assays (e.g., featuring a reporter gene readout) are exemplary. Cell-based assays preferably feature cells expressing GDF6, cells expressing at least one BMP signaling pathway protein and at least a transcription factor which regulates transcription of said protein, or a complement of said transcription factors and proteins.

The various forms of the term "modulate" include stimulation (e.g., increasing or upregulating a particular response or activity) and inhibition (e.g., decreasing or downregulating a particular response or activity).

A "modulator of melanoma-related molecules" includes a modulator of these molecules' expression, processing, post-translational modification, and/or activity. The term includes agents, for example a compound or compounds which modulate transcription of GDF6 molecule, a SMAD 1/5/8 molecule, or a BMP signaling pathway molecule gene, processing of melanoma-related molecules mRNA (e.g., splicing), translation of an GDF6 molecule, a SMAD 1/5/8 molecule, or a BMP signaling pathway molecule mRNA, post-translational modification GDF6 molecule, a SMAD 1/5/8 molecule, or a BMP signaling pathway molecule protein (e.g., glycosylation, ubiquitination) or activity of GDF6 molecule, a SMAD 1/5/8 molecule, or a BMP signaling pathway molecule protein, such as ALK3. A "modulator of melanoma-related molecules" includes compounds that directly or indirectly modulate melanoma-related molecules activity. For example, an indirect modulator of melanoma-related molecules activity can modulate a non-GDF6 molecule, a non-SMAD 1/5/8 molecule, or a non-BMP signaling pathway molecule which is in a signal transduction pathway that includes one of these molecules. Examples of modulators that directly modulate melanoma-related molecules expression, processing, post-translational modification, and/or activity include antisense or siRNA nucleic acid molecules that bind to melanoma-related molecules mRNA or genomic DNA, antibodies that bind to melanoma-related molecules and modulate (i.e., inhibit) melanoma-related molecules, activating antibodies that bind to melanoma-related molecules and modulate (i.e., enhance) melanoma-related molecules activity, melanoma-related molecules peptides that modulate (inhibit or enhance) the interaction of melanoma-related molecules with a target molecule, and expression vectors encoding melanoma-related molecules that allow for increased expression melanoma-related molecules activity in a cell, dominant negative forms of melanoma-related molecules, as well as chemical compounds that act to specifically modulate the activity of melanoma-related molecules.

As used interchangeably herein, the terms "melanoma-related molecule activity," "biological activity of melanoma-related molecule" or "functional activity of melanoma-related molecule," include activities exerted by a melanoma-related molecule protein on a melanoma-related molecule responsive cell or tissue, e.g., a melanoma cell, or on a melanoma-related molecule nucleic acid molecule or protein target molecule, as determined in vivo, or in vitro, according to standard techniques. Melanoma-related molecule activity can be a direct activity, such as an association with a melanoma-related molecule-target molecule. Alternatively, a melanoma-related molecule activity is an indirect activity, such as a downstream biological event mediated by interaction of the melanoma-related molecule protein with a melanoma-related molecule target molecule. The biological activities of melanoma-related molecules are described herein and include: e.g., enabling melanoma cell survival (GDF6), and BMP pathway signaling (SMD1/3/8). These findings provide for the use of melanoma-related molecules for as drug targets and as targets for modulation of these biological activities in cells and for therapeutic intervention in diseases such as melanoma.

### Cell-free assays

An assay is disclosed herein that is a cell-free assay in which a melanoma-related molecule (or biologically active portion thereof) is contacted with a test compound and the ability of the test compound to bind to the melanoma-related molecule polypeptide (or bioactive fragment thereof) is determined. Binding of the test compound to the melanoma-related molecule polypeptide (or bioactive fragment thereof) can be accomplished, for example, by coupling the test compound or the melanoma-related molecule polypeptide (or bioactive fragment thereof) with a radioisotope or enzymatic label such that binding of the test compound to the melanoma-related molecule polypeptide (or bioactive fragment thereof) can be determined by detecting the labeled compound or polypeptide in a complex. For example, test compounds or polypeptides can be labeled with ¹²⁵I, ³⁵S, ¹⁴C, or ³H, either directly or indirectly, and the radioisotope detected by direct counting of radioemmission or by scintillation counting. Alternatively, test compounds or polypeptides can be enzymatically labeled with, for example, horseradish peroxidase, alkaline phosphatase, or luciferase, and the enzymatic label detected by determination of conversion of an appropriate substrate to product.

Binding of the test compound to the melanoma-related molecule polypeptide (or bioactive fragment thereof) can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (e.g., BIAcore™). Changes in the optical phenomenon of surface plasmon resonance (SPR) can be used as an indication of real-time reactions between biological molecules.

It is disclosed that the assay includes contacting the melanoma-related molecule polypeptide (or biologically active portion thereof) with a melanoma-related molecule target molecule (or a bioactive fragment thereof) to form an assay mixture, contacting the assay mixture with a test compound, and determining the ability of the test compound to interact with the melanoma-related molecule polypeptide (or bioactive fragment thereof), wherein determining the ability of the test compound to interact with the melanoma-related molecule polypeptide (or bioactive fragment thereof) comprises determining the ability of the test compound to preferentially bind to the melanoma-related molecule polypeptide (or the bioactive portion thereof) as compared to the melanoma-related molecule target molecule. It is further disclosed that the assay includes contacting the melanoma-related molecule polypeptide (or biologically active portion thereof) with a melanoma-related molecule target molecule (or a bioactive fragment thereof) to form an assay mixture, contacting the assay mixture with a test compound, and determining the ability of the test compound to modulate (e.g., stimulate or inhibit) binding between the melanoma-related molecule polypeptide and the melanoma-related molecule target molecule (or a bioactive fragment thereof).

It is also disclosed that the assay is a cell-free assay in which a melanoma-related molecule polypeptide (or bioactive portion thereof) is contacted with a test compound and the ability of the test compound to modulate (e.g., stimulate or inhibit) the activity of the melanoma-related molecule polypeptide (or biologically active portion thereof) is determined.

Further disclosed is that the cell-free assay involves contacting a melanoma-related molecule polypeptide (or biologically active portion thereof) with a melanoma-related molecule target molecule which binds the melanoma-related molecule polypeptide to form an assay mixture, contacting the assay mixture with a test compound, and determining the ability of the test compound to preferentially modulate the activity of a melanoma-related molecule binding partner or target molecule, as compared to the melanoma-related molecule polypeptide (or biologically active portion thereof).

It is further disclosed that in the assay methods disclosed herein, it may be desirable to immobilize either a melanoma-related molecule or its binding partner/target molecule to facilitate separation of complexed from uncomplexed forms of one or both of the proteins, as well as to accommodate automation of the assay. Binding of a test compound to a melanoma-related molecule polypeptide, or interaction of a melanoma-related molecule polypeptide with a target molecule in the presence and absence of a candidate compound, can be accomplished in any vessel suitable for containing the reactants. Examples of such vessels include microtiter plates, test tubes, and micro-centrifuge tubes. It is disclosed that a fusion protein can be provided which adds a domain that allows one or both of the proteins to be bound to a matrix. For example, glutathione-S-transferase/melanoma-related molecule fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and the non-adsorbed target protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of melanoma-related molecule binding or activity determined using standard techniques.

Additional exemplary melanoma-related molecule fusion proteins include, but are not limited to, chitin binding domain (CBD) fusion proteins, hemagglutinin epitope tagged (HA)-fusion proteins, His fusion proteins (e.g., His₆ tagged proteins), FLAG tagged fusion proteins, AU1 tagged proteins, and the like.

Other techniques for immobilizing proteins on matrices can also be used in the screening assays of the invention. For example, either a melanoma-related molecule polypeptide or a melanoma-related molecule target molecule can be immobilized utilizing conjugation of biotin and streptavidin. Biotinylated melanoma-related molecule polypeptide or target molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with melanoma-related molecule polypeptide or target molecules but which do not interfere with binding of the melanoma-related molecule polypeptide to its target molecule can be derivatized to the wells of the plate, and unbound target or melanoma-related molecule polypeptide trapped in the wells by antibody conjugation. Methods for detecting such complexes, in addition to those described above for the glutathione S-transferase- (GST-) immobilized complexes, include immunodetection of complexes using antibodies reactive with the melanoma-related molecule polypeptide or target molecule as well as enzyme-linked assays which rely on detecting an enzymatic activity associated with the melanoma-related molecule polypeptide or target molecule.

In yet another aspect of the disclosure, it is disclosed that, the melanoma-related molecule polypeptides can be used as "bait proteins" in a two-hybrid assay or three-hybrid assay (see, e.g., U.S. Pat. No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with melanoma-related molecules ("melanoma-related molecule-binding proteins" or "melanoma-related molecule-target molecules") and are involved in melanoma-related molecule activity. Such melanoma-related molecule-target molecules are also likely to be involved in the regulation of cellular activities modulated by the melanoma-related molecule polypeptides.

At least one exemplary two-hybrid system is based on the modular nature of most transcription factors, which consist of separable DNA-binding and activation domains. Briefly, the assay utilizes two different DNA constructs. In one construct, the gene that codes for a melanoma-related molecule polypeptide is fused to a gene encoding the DNA binding domain of a known transcription factor (e.g., GAL-4). In the other construct, a DNA sequence, from a library of DNA sequences that encodes an unidentified protein "prey" or "sample" is fused to a gene that codes for the activation domain of the known transcription factor. If the "bait" and the "prey" proteins are able to interact, in vivo, forming a melanoma-related molecule-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the melanoma-related molecule polypeptide.

Another exemplary two-hybrid system, referred to in the art as the CytoTrap™ system, is based in the modular nature of molecules of the Ras signal transduction cascade. Briefly, the assay features a fusion protein comprising the "bait" protein and Son-of-Sevenless (SOS) and the cDNAs for unidentified proteins (the "prey") in a vector that encodes myristylated target proteins. Expression of an appropriate bait-prey combination results in translocation of SOS to the cell membrane where it activates Ras. Cytoplasmic reconstitution of the Ras signaling pathway allows identification of proteins that interact with the bait protein of interest, for example, melanoma-related molecule protein. Additional mammalian two hybrid systems are also known in the art and can be utilized to identify melanoma-related molecule interacting proteins.

### Cell-based assays

Further dislosed is an assay that is a cell-based assay in which a cell which expresses a melanoma-related molecule polypeptide, or biologically active portion thereof, is contacted with a test compound and the ability of the test compound to modulate the activity of the melanoma-related molecule polypeptide, or biologically active portion thereof, determined. The cell, for example, can be of mammalian origin. The melanoma-related molecule polypeptide, for example, can be expressed heterologously or native to the cell. Determining the ability of the test compound to modulate the activity of a melanoma-related molecule polypeptide, or biologically active portion thereof, can be accomplished by assaying for any of the activities of a melanoma-related molecule polypeptide described herein.

Determining the ability of a test compound to modulate the activity of a melanoma-related molecule polypeptide, or biologically active portion thereof, can also be accomplished by assaying for the activity of a melanoma-related molecule target molecule. Disclosed is that the cell which expresses the melanoma-related molecule polypeptide, or biologically active portion thereof, further expresses a melanoma-related molecule target molecule, or biologically active portion thereof.

Further disclosed is that an assay is a cell-based assay in which a cell which expresses a melanoma-related molecule polypeptide, or biologically active portion thereof, is contacted with a bioactive peptide derived from a melanoma-related molecule target molecule and a test compound and the ability of the test compound to modulate the activity of the melanoma-related molecule polypeptide, or biologically active portion thereof, determined.

According to the cell-based assays disclosed herein, determining the ability of the test compound to modulate the activity of the melanoma-related molecule polypeptide or biologically active portion thereof, can be determined by assaying for any of the native activities of a melanoma-related molecule polypeptide described herein, for example, assaying for the phosphorylation state of SMAD 1/5/8, or for apoptosis. Further disclosed is that an assay is a cell-based assay comprising contacting a cell expressing a melanoma-related molecule and the expression of the melanoma-related molecule is monitored. Moreover, the activity of the melanoma-related molecule polypeptide or biologically active portion thereof, can be determined by assaying for an indirect activity which is coincident the activity of a melanoma-related molecule polypeptide. Furthermore, determining the ability of the test compound to modulate the activity of the melanoma-related molecule polypeptide or biologically active portion thereof, can be determined by assaying for an activity which is not native to the melanoma-related molecule polypeptide, but for which the cell has been recombinantly engineered. For example, the cell can be engineered to express a melanoma-related molecule target molecule which is a recombinant protein comprising a bioactive portion of a melanoma-related molecule target molecule operatively linked to a non-melanoma-related molecule target molecule polypeptide. It is also disclosed that the cell-based assays disclosed herein comprise a final step of identifying the compound as a modulator of melanoma-related molecule activity.

### Test Compounds

The test compounds of the present disclosure can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, K. S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner U.S. Pat. No. 5,223,409), spores (Ladner U.S. Pat. No. '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner supra.).

It is disclosed that the library may be a natural product library, e.g., a library produced by a bacterial, fungal, or yeast culture. In another embodiment, the library is a synthetic compound library.

### Pharmaceutical compositions

The invention pertains to uses of the above-described agents for therapeutic treatments as described infra. Accordingly, it is disclosed that the modulators of the present invention can be incorporated into pharmaceutical compositions suitable for administration. Such compositions typically comprise the nucleic acid molecule, protein, antibody, or modulatory compound and a pharmaceutically acceptable carrier. A "pharmaceutically acceptable carrier" includes all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition disclosed herein is formulated to be compatible with its intended route of administration. Examples of routes of administration include parenteral, e.g., intravenous, intradermal, subcutaneous, intraperitoneal, intramuscular, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifingal agents, for example, parabens, chlorobutanol, phenol, ascorbic acid, thimerosal, and the like. In many cases, it will be preferable to include isotonic agents, for example, sugars, polyalcohols such as manitol and sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

It is disclosed that the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. Although compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the EC50 (i.e., the concentration of the test compound which achieves a half-maximal response) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions for administration.

### Using GDF6 or BMP signaling as diagnostic markers of melanoma

Since the inventors have discovered that GDF6 and BMP signaling correlate with melanoma initiation and maintenance, it is disclosed that these molecules can be used as diagnostic markers of melanoma. In brief, samples from a subject are collected and analyzed for the expression of GDF6 or BMP signaling molecules (such as phosphorylated SMAD 1/5/8). Detection of these molecules in a tissue when compared to a suitable control (such as cognate healthy tissue) indicates the presence or commencement of a melanoma or cancer.

### Sample preparation

Cells or tissue samples are collected from a subject. The subject is a vertebrate, more preferably a mammal, such as a monkey, dog, cat, rabbit, cow, pig, goat, sheep, horse, rat, mouse, guinea pig, etc.; and most preferably a human. Any technique to collect the desired sample may be employed, including biopsy or surgery.

### Detecting GDF6 and BMP-signaling molecules: Antibody-based methods

Immunochemical methods to detect protein expression, such as GDF6 or BMP-signaling proteins, are well known and include Western blotting, immunoaffinity purification, immunoprecipitation, enzyme-linked immunosorbent assay (ELISA), dot or slot blotting, radioimmunoassay (RIA), fluorescent immunoassay, chemiluminescent immunoassay (CMIA), immunohistochemical detection, immunocytochemical staining, and flow cytometry

Selected antibodies that are useful for detecting GDF6 are available from Thermo Fisher Scientific, Inc. (Waltham, MA; product nos. PA5-14394, PA5-20568), Biorbyt (San Francisco, CA; product nos. orb29645, orb75023, orb38022, orb157096), antibodies-online.com (Atlanta, GA; product nos. ABIN391699, ABIN655618, ABIN223491, ABIN342682, ABIN499881, ABIN950666, ABIN1411548, ABIN1757462, ABIN1858990, ABIN1805120, ABIN1411547, ABIN961880, ABIN1841949, ABIN1393653, ABIN1411550, ABIN1393657, ABIN1387414, ABIN1757463, ABIN1393654, ABIN1393655, ABIN1858989, ABIN1411545, ABIN1393656, ABIN1393658, ABIN1492198, ABIN1411546, ABIN1001913, ABIN1587025, ABIN541367), Lifespan Biosciences (Seattle, WA; product nos. LS-C83887, LS-C100696, LS-C146966 ,LS-C302848, LS-C166429, LS-C300549, LS-C294355, LS-C294354), Aviva Systems Biology (San Diego, CA; product nos. OAPB00713, OAAB07917, OAAB04538), R&D Systems (Minneapolis, MN; product no. AF855), Santa Cruz Biotechnology (Dallas, TX; product no. sc-6902), Atlas Antibodies (Stockholm, Sweden; product no. HPA045206), St. John's Laboratory (Atlanta, GA; product nos. STJ50764, STJ41772), Cloud-Clone Corp. (Houston, TX; product no. PAC111Mu01), Acris Antibodies (Atlanta, GA; product nos. AP51812PUN, AP30360PU-N), Abgent (Atlanta, GA; product nos. AP11672a, AP7483b), Origene (Rockville, MD; product no. TA306608), Abbiotec (Atlanta, GA; product no. 253907), United States Biological (Salem, MA; product nos. G8989-46, 35985, 140562), Abbexa (Atlanta, GA; product nos. abx33429, abx26376), Abcam (Cambridge, MA; product nos. ab135661, ab73288, ab170532), ProSci (Loveland, CO; product no. 4691), Abnova (Atlanta, GA; product no. PAB13050), GeneTex (Irvine, CA; product no. GTX81418), and Novus Biologicals (Littleton, CO; product nos. NBP1-91934, NBP1-76364, NBP1-45406).

Over 500 antibodies for detecting SMAD1/5/8 are commercially available; some can distinguish between phosphorylated and unphosphorylated SMAD1/5/8. Some of these are available from Rockland (Limerick, PA; product nos. 600-401-B23S, 600-401-B29S, 600-401-B23), Lifespan Biosciences (Seattle, WA; product nos. LS-B9239, LS-B10841, LS-C117925, LS-C80224, LS-C39125, LS-C138352, LS-C89093, LS-C132135, LS-C132134, LS-C89094, LS-C132133, LS-C31108, LS-C47059, LS-C39124, LS-C47102, LS-C79984, LS-C49618, LS-C89095, LS-C17859, LS-C48441, LS-C117539, LS-C137103, LS-C200706, LS-C177750, LS-C297547, LS-C240995, LS-C295417, LS-C295415, LS-C313543, LS-C240994, LS-C182556, LS-C240993, LS-C150071, LS-C191736, LS-C205361, LS-C191292, LS-C205161, LS-C133301, LS-C313542, LS-C241001, LS-C199577, LS-C150314, LS-C240996, LS-C176672, LS-C184123, LS-C172147, LS-C133300, LS-C248202, LS-C133299, LS-C305603, LS-C205282, LS-C184471, LS-C199578, LS-C133298, LS-C75853, LS-C190250, LS-C172149, LS-C172148, LS-C154246, LS-C156106, LS-C192196, LS-C152338, LS-C154249, LS-C295416, LS-C335924, LS-C331155, LS-C203001, LS-C335947, LS-C331261, LS-C325187, LS-C313635, PA1-41141, PA1-41238, PA1-41079, PA5-17122), Life Technologies (Grand Island, NY; product nos. 385400, 700047, 701168, 710199), Biorbyt (product nos. orb137917, orb126689, orb14693, orb128756, orb127512, orb106504, orb128757, orb14985, orb106255, orb137645, orb27546, orb159690, orb166630, orb129744, orb76556, orb106505, orb106256, orb76562, orb77385, orb11383, orb16322, orb158427, orb193076, orb154203, orb162780, orb162781, orb158426, orb192073, orb193031, orb191858, orb48619, orb193811), antibodies-online.com (product nos. ABIN183694, ABIN482828, ABIN393545, ABIN393492ABIN309906, ABIN393407, ABIN482239, ABIN481808, ABIN1532647, ABIN258473, ABIN1531627, ABIN1531198, ABIN1532214, ABIN483263, ABIN964908, ABIN223178, ABIN127139, ABIN964911, ABIN309632, ABIN319351, ABIN324489, ABIN202590, ABIN401471, ABIN272123, ABIN401472, ABIN401465, ABIN319257, ABIN401466, ABIN189594, ABIN933125, ABIN927059, ABIN349628, ABIN349630, ABIN274227, ABIN273707, ABIN273706, ABIN274226, ABIN1881815, ABIN598792, ABIN732164, ABIN517613, ABIN517612, ABIN207527, ABIN517616, ABIN325894, ABIN732162, ABIN605427, ABIN598791, ABIN732165, ABIN732161, ABIN561713, ABIN517610, ABIN561711, ABIN732167, ABIN791052, ABIN650448, ABIN517609, ABIN598790, ABIN732158, ABIN517615, ABIN598793, ABIN517614, ABIN781341, ABIN732163, ABIN363042, ABIN517611, ABIN732160, ABIN215474, ABIN352292, ABIN207569, ABIN241890, ABIN874784, ABIN915706, ABIN746022, ABIN915709, ABIN874781, ABIN498431, ABIN746020, ABIN746018, ABIN915710, ABIN204726, ABIN746027, ABIN746025, ABIN362414, ABIN874783, ABIN746021, ABIN746023, ABIN874782, ABIN746024, ABIN915707, ABIN1836313, ABIN1874849, ABIN1870609, ABIN958266, ABIN1874850, ABIN1835197, ABIN1821900, ABIN950121, ABIN1513536, ABIN1816440, ABIN1838551, ABIN204727, ABIN1860587, ABIN1831955, ABIN352291, ABIN1823822, ABIN1104364, ABIN1823368, ABIN1831609, ABIN1804509, ABIN1874848, ABIN967044, ABIN1104365, ABIN1815373, ABIN959573, ABIN1860586, ABIN534398, ABIN437988, ABIN643268, ABIN802764, ABIN1765877, ABIN1848036, ABIN446925, ABIN1579581, ABIN1682045, ABIN408128, ABIN1765875, ABIN1845442, ABIN1765874, ABIN1579582, ABIN1847220, ABIN1579580, ABIN1847368, ABIN641219, ABIN1856906, ABIN556465, ABIN1682803, ABIN1092467, ABIN184544, ABIN1579579, ABIN546471, ABIN548306, ABIN448968, ABIN967042, ABIN967043), Boster Immunoleader (Atlanta, GA; product nos. PA2114, PA1331), Aviva Systems Biology (San Diego, CA; product nos. OAAF00650, OAAF01080, OAAF00206, OAAF00021, OAEC02036, OAEC02038, OAEC02039, OAEC01471, OAEC01470, OAEC00286, OAEC00646, OAAB15530, OAAF02467, OARA03110, OARA03111, OARA03112, OARA03109, ARP32003_P050, OABB00290, ARP38692_T100, ARP38693_T10), NovaTeinBio (Woburn, MA; product no. 67445), and Biosensis (Temecula, CA; product no. R-1368-100).

If additional antibodies are desired, they can be produced using well-known methods, some of which are described below.

An approach using antibodies to detect the presence of an antigen usually includes one or more of the following steps:
(1) attaching the sample being tested for an antigen, such GDF6 or SMAD1/5/8, to an appropriate substrate;
(2) washing the sample with buffer or water;
(3) blocking non-specific antibody binding sites;
(4) applying the antibody; and
(5) detecting bound antibody, either *via* a detectable labeled-secondary antibody that recognizes the primary antibody or a detectable label that has been directly attached to, or associated with, the bound (anti-GDF6 or anti-SMAD1/5/8) antibody.

Substrates may be washed with any solution that does not interfere with epitope structure. Common buffers include saline and biological buffers, such as bicine, tricine, and Tris.

Non-specific binding sites are blocked by applying a protein solution, such as bovine serum albumin (BSA; denatured or native), milk proteins, or in the cases wherein the detecting reagent is a secondary antibody, normal serum or immunoglobulins from a non-immunized host animal whose species is the same origin as the detecting antibody. For example, a procedure using a secondary antibody made in goats would employ normal goat serum (NGS).

The substrate is then reacted with the antibody of interest. The antibody may be applied in any form, such as F_{ab} fragments and derivatives thereof, purified antibody (by affinity, precipitation, *etc*.), supernatant from hybridoma cultures, ascites, serum or recombinant antibodies expressed by recombinant cells. The antibody may be diluted in buffer or media, often with a protein carrier such as the solution used to block non-specific binding sites; the useful antibody concentration is usually determined empirically. In general, polyclonal sera, purified antibodies and ascites may be diluted 1:50 to 1:200,000, more often, 1:200 to 1:500. Hybridoma supernatants may be diluted 1:0 to 1:10, or may be concentrated by dialysis or ammonium sulfate precipitation (or any other method that retains the antibodies of interest but at least partially removes the liquid component and preferably other small molecules, such as salts) and diluted if necessary. Incubation with antibodies may be carried out for as little as 20 minutes at 37° C, 1 to 6 hours at room temperature (approximately 22° C), or 8 hours or more at 4° C.

To detect an antibody-antigen complex, a label may be used. The label may be coupled to the binding antibody or to a second antibody that recognizes the first antibody and is incubated with the sample after the primary antibody incubation and thorough washing. Suitable labels include fluorescent moieties, such as fluorescein isothiocyanate; fluorescein dichlorotriazine and fluorinated analogs of fluorescein; naphthofluorescein carboxylic acid and its succinimidyl ester; carboxyrhodamine 6G; pyridyloxazole derivatives; Cy2, 3 and 5; phycoerythrin; fluorescent species of succinimidyl esters, carboxylic acids, isothiocyanates, sulfonyl chlorides, and dansyl chlorides, including propionic acid succinimidyl esters, and pentanoic acid succinimidyl esters; succinimidyl esters of carboxytetramethylrhodamine; rhodamine Red-X succinimidyl ester; Texas Red sulfonyl chloride; Texas Red-X succinimidyl ester; Texas Red-X sodium tetrafluorophenol ester; Red-X; Texas Red dyes; tetramethylrhodamine; lissamine rhodamine B; tetramethylrhodamine; tetramethylrhodamine isothiocyanate; naphthofluoresceins; coumarin derivatives; pyrenes; pyridyloxazole derivatives; dapoxyl dyes; Cascade Blue and Yellow dyes; benzofuran isothiocyanates; sodium tetrafluorophenols; 4,4-difluoro-4-bora-3a,4a-diaza-*s*-indacene. Suitable labels further include enzymatic moieties, such as alkaline phosphatase or horseradish peroxidase; radioactive moieties, including ³⁵S and ¹³⁵I-labels; avidin (or streptavidin)-biotin-based detection systems (often coupled with enzymatic or gold signal systems); and gold particles. In the case of enzymatic-based detection systems, the enzyme is reacted with an appropriate substrate, such as 3, 3'-diaminobenzidine (DAB) for horseradish peroxidase; preferably, the reaction products are insoluble. Gold-labeled samples, if not prepared for ultrastructural analyses, may be chemically reacted to enhance the gold signal; this approach is especially desirable for light microscopy. The choice of the label depends on the application, the desired resolution and the desired observation methods. For fluorescent labels, the fluorophore is excited with the appropriate wavelength and the sample observed using a microscope, confocal microscope, or FACS machine. In the case of radioactive labeling, the samples are contacted with autoradiography film, and the film developed; alternatively, autoradiography may also be accomplished using ultrastructural approaches. Alternatively, radioactivity may be quantified using a scintillation counter.

### Morphological-coupled approaches:

The presence of GDF6 or SMAD 1/5/8 can be ascertained by immunolocalization. Generally, cells or tissue are preserved by fixation, exposed to an antibody that recognizes the epitope of interest, such as GDF6 or SMAD 1/5/8, and the bound antibody detected.

Any tissue or even an entire organism is appropriate for fixation. Tissue may be from any organ (such as skin), and may be harvested after or prior to fixation.

Fixation, if desired, may be by any known means; the requirements are that the protein to be detected is not rendered unrecognizable by the binding agent, most often an antibody. Appropriate fixatives include paraformaldehyde-lysine-periodate, formalin, paraformaldehyde, methanol, acetic acid-methanol, glutaraldehyde, acetone, Karnovsky's fixative, *etc.* The choice of fixative depends on variables such as the protein of interest, the properties of a particular detecting reagent (such as an antibody), the method of detection (fluorescence, enzymatic) and the method of observation (epifluorescence microscopy, confocal microscopy, light microscopy, electron microscopy, *etc.*). The sample is usually first washed, most often with a biological buffer, prior to fixation. Fixatives are prepared in solution or in biological buffers; many fixatives are prepared immediately prior to applying to the sample. Suitable biological buffers include saline (*e.g*., phosphate buffered saline), N-(carbamoylmethyl)-2-aminoethanesulfonic acid (ACES), N-2-acetamido-2-iminodiacetic acid (ADA), bicine, bis-tris, 3-cyclohexylamino-2-hydroxy-1-propanesulfonic acid (CAPSO), ethanolamines, glycine, N-2-hydroxyethylpiperazine-N'-2-ethanesulfonic acid (HEPES), 2-N-morpholinoethanesulfonic acid (MES), 3-N-morpholinopropanesulfonic acid (MOPS), 3-N-morpholino-2-hyrdoxy-propanesulfonic acid (MOPSO), piperazine-N,N'-bis(2-ethanesulfonic acid) (PIPES), tricine, triethanolamine, *etc.* An appropriate buffer is selected according to the sample being analyzed, appropriate pH, and the requirements of the detection method. A useful buffer is phosphate buffered saline (PBS). After fixation, the sample may be stored in fixative, preferably fresh, or temporarily or indefinitely, at a temperature between about 4° C to about 22° C. In some cases, depending on the characteristics of the sample, the sample may be attached to a substrate, such as to a glass coverslip, microscope slide or plastic. Such substrates may be treated to enhance attachment; such treatments included charging the substrate, coating the substrate with an adhesive material, such as poly-(L or D or combination)-lysine, extracellular matrix molecules or compositions, *etc.*

After fixation from 5 minutes to 1 week, depending on the sample size, sample thickness, and viscosity of the fixative, the sample is washed in buffer. If the sample is thick or sections are desired, the sample may be embedded in a suitable matrix. For cryosectioning, sucrose is infused, and embedded in a matrix, such as OCT Tissue Tek (Andwin Scientific; Canoga Park, CA) or gelatin. Samples may also be embedded in paraffin wax, or resins suitable for electron microscopy, such as epoxy-based (Araldite, Polybed 812, Durcupan ACM, Quetol, Spurr's, or mixtures thereof; Polysciences, Warrington, PA), acrylates (London Resins (LR White, LR gold), Lowicryls, Unicryl; Polysciences), methylacrylates (JB-4, OsteoBed; Polysciences), melamine (Nanoplast; Polysciences) and other media, such as DGD, Immuno-Bed (Polysciences) and then polymerized. Resins that are especially appropriate include hydrophilic resins (such as Lowicryls, London Resins, water-soluble Durcupan, *etc*.) since these are less likely to denature the protein of interest during polymerization and will not repel antibody solutions. When embedded in wax or resin, samples are dehydrated by passing them through a concentration series of ethanol or methanol; in some cases, other solvents may be used, such as polypropylene oxide. Embedding may occur after the sample has been reacted with the detecting agents, or samples may be first embedded, sectioned (via microtome, cyrotome, or ultramicrotome), and then the sections reacted with the detecting reagents. In some cases, the embedding material may be partially or completely removed before detection to facilitate antigen access.

In some instances, the GDF6 or SMAD 1/5/8 epitope(s) to which the antibody binds may be rendered unavailable because of fixation. Antigen retrieval methods can be used to make the antigen available for antibody binding. Many recourses are available. Common methods include using heat supplied from autoclaves, microwaves, hot water or buffers, pressure cookers, or other sources of heat. Often the sources of heat are used in sequence; the samples must often be in solution (*e.g*., microwave treatments). Detergent treatment may also unmask antigens, such as sodium dodecyl sulfate (SDS, 0.25% to 1%) or other denaturing detergents. Chemical methods include strong alkalis (such as NaOH), prolonged immersion in water, urea, formic acid and refixation in zinc sulfate-formalin. In other instances, proteolytic enzyme treatment will modify the antigen such that it is available to the antibody. Any number of proteases may be used, such as trypsin. These methods may be combined to achieve optimal results. The choice of the antigen retrieval method will depend on the sample, its embedment (if any), and the anti-GDF6 or anti-SMAD 1/5/8 antibody.

Especially in the cases of immunofluorescent or enzymatic product-based detection, background signal due to residual fixative, protein cross-linking, protein precipitation or endogenous enzymes may be quenched, using, *e.g*., ammonium chloride or sodium borohydride or a substance to deactivate or deplete confounding endogenous enzymes, such as hydrogen peroxide which acts on peroxidases. To detect intracellular proteins in samples that are not to be sectioned, samples may be permeabilized. Permeabilizing agents include detergents, such as t-octylphenoxypolyethoxyethanols, polyoxyethylenesorbitans, and other agents, such as lysins, proteases, *etc.*

Non-specific binding sites are blocked by applying a protein solution, such as bovine serum albumin (BSA; denatured or native), milk proteins, or preferably in the cases wherein the detecting reagent is an antibody, normal serum or IgG from a non-immunized host animal whose species is the same is the same origin of the detecting antibody.

### Biochemical assay-based approaches:

In some aspects, extracting GDF6 or SMAD 1/5/8 before detection may be desirable. This may be achieved in any number of ways, such as simple cell extraction, differential extraction or mechanical disruption. Extracting reagents are well known. For example, solvents such as methanol may be occasionally useful. More likely, detergents, such as t-octylphenoxypolyethoxyethanol (also known as polyethylene glycol tert-octylphenyl ether) are particularly useful for simple extractions. Also useful are glucopyranosides, maltopyranosides, maltosides, polyoxyethylene esters, other polyoxyethylene ethers, salts of alginic, caprylic, cholic 1-decanesulfonic, deoxycholic, dioctyl sulfosuccinate, 1-dodecanesulfonic, glyocholic, glycodeoxycholic, 1-heptanesulfonic, 1-hexanesulfonic, N-lauroylsacrosine, lauryl sulfate (*e.g*., SDS), 1-nonanesulfonic, 1-octanesulfonic, 1-pentanesulfonic, taurocholic and tauodexycholic acids; sodium 7-ethyl-2-methyl-4-undecyl sulfate, and sodium 2-ethylhexyl sulfate. Other useful detergents include(3-[(3-cholamidopropyl)dimethylammonio] -1 -propane-sulfonate, (3-[(3-cholamidopropyl)dimethylammonio]-2-hydroxy-1-propane-sulfonate, N-decyl-, N-dodecyl-, N-hexadecyl-, N-octadecyl-, N-tetradecyl-N,N-dimethyl-3-ammonio-1-propanesulfonates and phosphatidylcholine. Less useful, but may be helpful in some cases, are alkyltrimethylammonium bromides, benzalkonium chloride, benzethonium chloride, benzyldimethyldodecylammonium bromide, benzyldimethylhexadecylammonium chloride, cetyldimethylethylammonium bromide, cetylpyridinium, decamethonium bormide, dimethyldioctadecylammonium bromide, methylbenzethonium chloride, methyltiroctylammonium chloride, and N,N',N'-polyoxyehtlylene(10)-N-tallow-1,3-diaminopropane. The different extracting reagents may be used singly or in combination; they may be prepared in simple aqueous solutions or suitable buffers.

Polyethylene glycol ter-octylphenyl ether is particularly useful for differential extraction by taking advantage of the low cloud point to separate membrane proteins from soluble proteins into two different phases. Extraction buffers may contain protease inhibitors, such as aprotinin, benzamidine, antipain, pepstatin and iodoacetamide.

Extracts are then assayed for GDF6 or SMAD1/5/8. Preferably, GDF6 or SMAD1/5/8 is detected using an immunochemical assay technique. Various types of enzyme linked immunosorbent assays (ELISAs) to detect proteins are known, and these are applicable to GDF6 and SMAD1/5/8 detection. However, ELISA-like assays employing alternative labeling techniques may also be used. These include Radio Immunoassay (RIA), Fluorescent Immunoassay (FIA), Chemiluminescent Immunoassay (CMIA) and other non-enzyme linked antibody binding assays and procedures. Various assay formats including competitive (reagent limited) and immunometric assays may be used. In addition, heterogeneous assays and homogenous assays including agglutination assay, nephelometry and turbidimetry, enzyme-multiplied immunoassay technique (EMIT®), and fluorescence polarization may be used.

The double antibody-sandwich ELISA technique is especially useful. The basic protocol for a double antibody-sandwich ELISA is as follows: A plate is coated with anti-GDF6 or anti- SMAD1/5/8 antibodies (capture antibodies). The plate is then washed with a blocking agent, such as BSA, to block non-specific binding of proteins (antibodies or antigens) to the test plate. The test sample is then incubated on the plate coated with the capture antibodies. The plate is then washed, incubated with anti-GDF6 or anti- SMAD1/5/8 antibodies, washed again, and incubated with a specific antibody-labeled conjugates and the signal appropriately detected.

In other ELISAs, proteins or peptides are immobilized onto a selected surface, the surface can have, or treated to have, an affinity for polypeptide attachment, such as the wells of a specially-treated polystyrene microtiter plates. After washing to remove incompletely adsorbed material, one would then generally desire to bind or coat with a nonspecific protein that is known to be antigenically neutral with anti-GDF6 or anti-SMAD 1/5/8 antibodies, such as BSA or casein, onto the well bottom. This step allows for blocking of nonspecific adsorption sites on the immobilizing surface and thus reduces the background caused by nonspecific binding of antibodies onto the surface. When the antibodies were created in an animal by conjugating a polypeptide to a protein (*e.g*., BSA), a different protein is usually used as a blocking agent, because of the possibility of the presence of antibodies to the blocking protein the antibody composition.

After binding of GDF6 or SMAD 1/5/8 to the well, coating with a non-reactive material to reduce background, and washing to remove unbound material, the immobilizing surface is contacted with an anti-GDF6 or anti-SMAD1/5/8 antibody composition in a manner conducive to immune complex (antigen/antibody) formation. Such conditions include diluting the antibody composition with diluents such as BSA, bovine γ globulin (BGG) and PBS/Polyoxyethylenesorbitan monolaurate. These added agents also assist in the reduction of nonspecific background signal. The layered antibody composition is then allowed to incubate for, *e.g.,* from about two to four hours at 25° C to 37° C. Following incubation, the antibody composition-contacted surface is washed so as to remove non-immunocomplexed material. One washing procedure includes washing with a PBS/polyoxyethylenesorbitan monolaurate or borate buffer solution.

Following formation of specific immunocomplexes between the test sample and the antibody and subsequent washing, immunocomplex formation is detected using a second antibody having specificity for the anti-GDF6 or anti- SMAD 1/5/8 antibody. For detection, the secondary antibody is associated with detectable label, such as an enzyme or a fluorescent molecule.

### Western blotting

Western blotting methods are well known. Generally, a protein sample is subjected to sodium dodecyl sulfate-polyacrylamide gel electrophoresis (SDS-PAGE) at such conditions as to yield an appropriate separation of proteins within the sample. The proteins are then transferred to a membrane (*e.g.,* nitrocellulose, nylon, *etc*.) in such a way as to maintain the relative positions of the proteins to each other.

Visibly labeled proteins of known molecular weight may be included within a lane of the gel. These proteins serve as a control to insure adequate transfer of the proteins to the membrane, as well as molecular weight markers for determining the relative molecular weight of other proteins on the blot. Alternatively, unlabeled marker proteins are detected after transfer with Brilliant Blue (G or R; Sigma; St. Louis, MO) other protein dyes. After protein transfer, the membrane is submersed in a blocking solution to prevent nonspecific binding of the primary antibody.

The primary antibody, e.g., anti-GDF6 or anti- SMAD1/5/8, may be labeled and the presence and molecular weight of the antigen may be determined by detecting the label at a specific location on the membrane. However, the primary antibody may not be labeled, and the blot is further reacted with a labeled second antibody. This secondary antibody is immunoreactive with the primary antibody; for example, the secondary antibody may be one to rabbit immunoglobulins and labeled with alkaline phosphatase.

### Prognostic assays

It is also disclosed that diagnostic methods can furthermore be used to identify subjects having, or at risk of developing, a neoplasia at an early stage of disease development. Prognostic assays can be used to identify a subject having or at risk for developing a neoplasia, such as a subject who has a family history of harmful neoplasias, especially cancers, such as melanoma. A method for identifying such an individual would include a test sample obtained from a subject.

### Kits

It is further disclosed that kits, containers, packs, or dispensers containing GDF6 and/or SMAD 1/5/8 probes and detection reagents, together with instructions for administration, may be assembled. When supplied as a kit, the different components may be packaged in separate containers and admixed immediately before use. Such packaging of the components separately may permit long-term storage without losing the active components' functions.

Kits may also include reagents in separate containers that facilitate the execution of a specific test, such as diagnostic tests. The components of a kit are an anti-GDF6 agent or anti-SMAD 1/5/8 agent used to probe for GDF6 or SMAD 1/5/8, a control sample, and optionally a composition to detect GDF6 or SMAD1/5/8. Examples of anti-GDF6 and anti-SMAD1/5/8 agents include an anti-GDF6 or anti- SMAD1/5/8 antibody or fragment thereof; if labeled, then a GDF6- or SMAD1/5/8-binding detection reagent is superfluous. Examples of detection reagents include: labeled secondary antibodies, for example, an anti-mouse polyclonal antibody made in donkey and then tagged with a fluorophore such as rhodamine. Control components may include: normal serum from the animal in which a secondary antibody was made; a solution containing GDF6 and/or SMAD1/5/8 polypeptide; a dot blot of GDF6 or SMAD 1/5/8 protein to assay GDF6- or SMAD1/5/8-binding reagent reactivity. Other components may include buffers, fixatives, blocking solutions, microscope slides and/or cover slips or other suitable substrates for analysis, such as microtiter plates; detergent or detergent solutions or other permeabilizing reagents; miscellaneous reagents, protease inhibitors, various containers and miscellaneous tools and equipment to facilitate the assays.

In many cases, especially convenient kits may be assembled not only with the components listed above, but also with means for collecting a sample.

### (a) Containers or vessels

Reagents included in kits can be supplied in containers of any sort such that the life of the different components are preserved and are not adsorbed or altered by the materials of the container. For example, sealed glass ampules may contain lyophilized GDF6 or SMAD1/5/8 binding reagents (such as anti-GDF6 or anti- SMAD1/5/8) or buffers that have been packaged under a neutral, non-reacting gas, such as nitrogen. Ampules may consist of any suitable material, such as glass, organic polymers (i.e., polycarbonate, polystyrene, etc.), ceramic, metal or any other material typically employed to hold reagents. Other examples of suitable containers include simple bottles that may be fabricated from similar substances as ampules, and envelopes that may have foil-lined interiors, such as aluminum or alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, *etc.*

### (b) Instructional materials

Kits may also be supplied with instructional materials. Instructions may be printed on paper or other substrate and/or may be supplied as an electronic-readable medium, such as a floppy disc, CD-ROM, DVD-ROM, DVD, videotape, audio tape, *etc.* Detailed instructions may not be physically associated with the kit; instead, a user may be directed to an internet web site specified by the manufacturer or distributor of the kit, or supplied as electronic mail.

### Methods of the Invention

The present invention provides for GDF6 inhibitors for use intreating a subject at risk of (or susceptible to) a disorder or having a disorder that would benefit from modulation of a melanoma-related molecule. The GDF6 inhibitors are particularly useful for treating subjects displaying melanoma.

Desired therapeutic effects include a modulation of any melanoma-related molecule-associated activity, as described herein. A preferred therapeutic effect is modulation of the expression or activity of the melanoma-related molecule. Desired therapeutic effects also include a decrease in melanoma-related molecule mRNA expression, melanoma-related molecule protein levels, or melanoma-related molecule activity, as described herein. Desired therapeutic effects also include, but are not limited to curing or healing the subject, alleviating, relieving, altering or ameliorating a disease or disorder in the subject or at least one symptom of said disease or disorder in the subject, or otherwise improving or affecting the health of the subject.

Ameliorating at least one symptom of the disease or disorder being treated in satisfactory although amelioration of several, if not all, symptoms of the disease or disorder is preferred.

A featured aspect of the invention pertains to methods of modulating the melanoma-related molecules for therapeutic purposes.

Thus, the present invention features methods of treatment or therapeutic methods. Disclosed is a method of treating a subject (e.g., a human subject in need thereof) with a modulatory compound identified according to the present invention (e.g., a melanoma-related molecule modulator), such that a desired therapeutic effect is achieved.

Further disclosed is a method for preventing in a subject, melanoma, by administering to the subject an agent which modulates the expression or activity of the melanoma-related molecule. Subjects at risk for such disorders can be identified, for example, using methods disclosed herein or any one or a combination of diagnostic or prognostic assays known in the art. Administration of a prophylactic agent can occur prior to the manifestation of symptoms characteristic of melanoma, such that a disease is prevented or, alternatively, delayed in its progression.

A compound that diminishes the expression and/or activity of a melanoma-related molecule or the expression and/or activity a melanoma-related-binding molecule, may be used for treating a subject. Agents for use can be known (e.g., sense or antisense nucleic acid molecules encoding a melanoma-related molecule or interacting molecules or the polypeptides they encode) or can be identified, e.g., using the screening assays disclosed herein (e.g., a melanoma-related molecule antagonist, a peptidomimetic of a melanoma-related molecule antagonist, a melanoma-related molecule peptidomimetic, or other small molecule).

Modulatory methods of the invention involve contacting a cell with an agent that modulates the activity and/or expression of a melanoma-related molecule and/or a melanoma-related molecule interacting molecule.

These modulatory methods can be performed in vitro (e.g., by contacting the cell with the agent) or, alternatively, in vivo (e.g., by administering the agent to a subject). As such, the present invention provides methods of treating an individual afflicted with a condition or disorder that would benefit from down-modulation of at least one melanoma-related molecule. In one embodiment, the method involves administering an agent (e.g., an agent identified by a screening assay described herein), or combination of agents downregulates the expression and/or activity of a melanoma-related molecule.

### Subjects and patients

In a preferred aspect, the invention features a method of treating a subject displaying melanoma.

Identification or selection of a subject in need thereof can be accomplished by any skilled medical practitioner or researcher using art-recognized diagnostic skills or techniques

The effectiveness of treatment of a subject with a modulatory compound of the invention can be monitored by (i) detecting the level of melanoma-related molecules; (ii) detecting the presence of a melanoma-related molecule; (iii) comparing the levels of melanoma-related molecules pre-administration and post administration; and (iv) altering the administration of the modulator to the subject accordingly.

### Therapeutic Methods

### Inhibiting antibodies

The present invention also includes a melanoma-related molecule inhibiting antibody or biologically active portion thereof for use in therapeutic methods comprising administering to a subject a therapeutically effective dose, such that melanoma-related molecule biological activity in said subject is decreased to levels detectable in normal or control individuals. Preferred antibodies include monoclonal antibodies, including humanized, chimeric and human monoclonals or fragments thereof. To generate such antibodies, a proteolytic or synthetic melanoma-related molecule fragment (alone or linked to a suitable carrier or hapten) can be used to immunize a subject (e.g., a mammal including, but not limited to a rabbit, goat, mouse or other mammal). For example, the methods described in U.S. Pat. Nos. 5,422,110; 5,837,268; 5,708,155; 5,723,129; and 5,849,531, can be used. The immunogenic preparation can further include an adjuvant, such as Freund's complete or incomplete adjuvant, or similar immunostimulatory agent. Immunization of a suitable subject with an immunogenic proteolytic or synthetic melanoma-related molecule fragment preparation induces a polyclonal anti-melanoma-related molecule antibody response. The anti-melanoma-related molecule antibody titer in the immunized subject can be monitored over time by standard techniques, such as with an enzyme linked immunosorbent assay (ELISA) using immobilized melanoma-related molecules. Subsequently, the sera from the immunized subjects can be tested for their inhibitory activity using any of the bioassays described herein.

Alternatively, it is also possible to immunize subjects with plasmids expressing a melanoma-related molecule using DNA immunization technology, such as that disclosed in U.S. Pat. No. 5,795,872, Ricigliano et al., "DNA construct for immunization" (1998), and in U.S. Pat. No. 5,643,578, Robinson et al., "Immunization by inoculation of DNA transcription unit" (1997).

The antibody molecules directed against a melanoma-related molecule can be isolated from the mammal (e.g., from the blood) and further purified by well-known techniques, such as protein A chromatography to obtain the IgG fraction. At an appropriate time after immunization, e.g., when the antibody titers are highest, antibody-producing cells can be obtained from the subject and used to prepare e.g., monoclonal antibodies by standard techniques, such as the hybridoma technique originally described by Kohler and Milstein (1975) Nature 256:495-497) (see also, Brown et al. (1981) J. Immunol. 127:539-46; Brown et al. (1980) J. Biol. Chem. 255:4980-83; Yeh et al. (1976) Proc. Natl. Acad. Sci. USA 76:2927-31; and Yeh et al. (1982) Int. J. Cancer 29:269-75), the more recent human B cell hybridoma technique (Kozbor et al. (1983) Immunol Today 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, N.Y. (1980); E. A. Lemer (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a melanoma-related molecule immunogen as described above, and the culture supernatants of the resulting hybridoma cells are screened to identify a hybridoma producing a monoclonal antibody that binds a melanoma-related molecule.

Any of the many well-known protocols used for fusing lymphocytes and immortalized cell lines can be applied for the purpose of generating an anti-melanoma-related molecule monoclonal antibody (see, e.g., G. Galfre et al. (1977) Nature 266:55052; Gefter et al. Somatic Cell Genet., cited supra; Lemer, Yale J. Biol. Med., cited supra; Kenneth, Monoclonal Antibodies, cited supra). Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin, and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from American Type Culture Collection (ATCC; Rockville, MD). Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind melanoma-related molecule, e.g., using a standard ELISA assay. The antibodies can then be tested for melanoma-related molecule inhibitory activity using, for example, the assays described herein.

A method is disclosed that involves administering to an isolated tissue or cell line from the subject a modulatory compound identified according to the methodology described herein, such that a desired effect is achieved. Further disclosed is a method that involves genetically-engineering a tissue or cell line, e.g., a tissue or cell line from a subject or patient, such that melanoma-related molecule expression or activity is decreased.

### Antisense nucleic acid molecules

The present invention also includes a melanoma-related molecule antisense nucleic acid molecule that is complementary to a gene encoding a melanoma-related molecule or to a portion of said gene, or a recombinant expression vector encoding said antisense nucleic acid molecule for use in therapeutic methods comprising administering to a subject a therapeutically effective dose. The use of antisense nucleic acids to down-regulate the expression of a particular protein in a cell is well known in the art (see e.g., Weintraub, H. et al., Antisense RNA as a molecular tool for genetic analysis, Reviews--Trends in Genetics, Vol. 1(1) 1986; Askari, F. K. and McDonnell, W. M. (1996) N. Eng. J. Med. 334:316-318; Bennett, M. R. and Schwartz, S. M. (1995) Circulation 92:1981-1993; Mercola, D. and Cohen, J. S. (1995) Cancer Gene Ther. 2:47-59; Rossi, J. J. (1995) Br. Med. Bull. 51:217-225; Wagner, R. W. (1994) Nature 372:333-335). An antisense nucleic acid molecule comprises a nucleotide sequence that is complementary to the coding strand of another nucleic acid molecule (e.g., an mRNA sequence) and accordingly is capable of hydrogen bonding to the coding strand of the other nucleic acid molecule. Antisense sequences complementary to a sequence of an mRNA can be complementary to a sequence found in the coding region of the mRNA, the 5' or 3' untranslated region of the mRNA or a region bridging the coding region and an untranslated region (e.g., at the junction of the 5' untranslated region and the coding region). Furthermore, an antisense nucleic acid can be complementary in sequence to a regulatory region of the gene encoding the mRNA, for instance a transcription initiation sequence or regulatory element. Preferably, an antisense nucleic acid is designed so as to be complementary to a region preceding or spanning the initiation codon on the coding strand or in the 3' untranslated region of an mRNA.

Given the coding strand sequences encoding melanoma-related molecules that are known in the art, antisense nucleic acids of the invention can be designed according to the rules of Watson and Crick base pairing. The antisense nucleic acid molecule can be complementary to the entire coding region of a melanoma-related molecule mRNA, but more preferably is an oligonucleotide which is antisense to only a portion of the coding or noncoding region of a melanoma-related molecule mRNA. For example, the antisense oligonucleotide can be complementary to the region surrounding the translation start site of a melanoma-related molecule mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides may be used. Examples of modified nucleotides which may be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection.

The antisense nucleic acid molecules of the invention are typically administered to a subject or generated in situ such that they hybridize with or bind to cellular mRNA and/or genomic DNA encoding a melanoma-related molecule or a melanoma-related molecule-interacting molecule protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

In another embodiment, an antisense nucleic acid of the invention is a compound that mediates RNAi. RNA interfering agents include, but are not limited to, nucleic acid molecules including RNA molecules which are homologous to the target gene or genomic sequence, e.g., a melanoma-related molecule, or a fragment thereof, "short interfering RNA" (siRNA), "short hairpin" or "small hairpin RNA" (shRNA), and small molecules which interfere with or inhibit expression of a target gene by RNA interference (RNAi). RNA interference is a post-transcriptional, targeted gene-silencing technique that uses double-stranded RNA (dsRNA) to degrade messenger RNA (mRNA) containing the same sequence as the dsRNA (Sharp, P.A. and Zamore, P.D. 287, 2431-2432 (2000); Zamore, P.D., et al. Cell 101, 25-33 (2000). Tuschl, T. et al. Genes Dev. 13, 3191-3197 (1999)). The process occurs when an endogenous ribonuclease cleaves the longer dsRNA into shorter, 21- or 22-nucleotide-long RNAs, termed small interfering RNAs or siRNAs. The smaller RNA segments then mediate the degradation of the target mRNA. Kits for synthesis of RNAi are commercially available from, e.g. New England Biolabs (Ipswich, MA) and Ambion/Life Technologies (Grand Island, NY). In one embodiment one or more of the chemistries described above for use in antisense RNA can be employed.

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach, 1988, Nature 334:585-591) may be used to catalytically cleave a melanoma-related molecule mRNA transcripts to thereby inhibit translation of a melanoma-related molecule mRNA. A ribozyme having specificity for a melanoma-related molecule-encoding nucleic acid can be designed based upon the nucleotide sequence or another nucleic acid molecule encoding another a melanoma-related molecule polypeptide by one skilled in the art. For example, a derivative of a Tetrahymena L-19 IVS RNA can be constructed in which the nucleotide sequence of the active site is complementary to the nucleotide sequence to be cleaved in a melanoma-related molecule-encoding mRNA. See, e.g., Cech et al. U.S. Pat. No. 4,987,071; and Cech et al. U.S. Pat. No. 5,116,742. Alternatively, a melanoma-related molecule mRNA may be used to select a catalytic RNA having a specific ribonuclease activity from a pool of RNA molecules. See, e.g., Bartel, D. and Szostak, J. W., 1993, Science 261:1411-1418.

Alternatively, gene expression can be inhibited by targeting nucleotide sequences complementary to the regulatory region of a melanoma-related molecule (e.g., the melanoma-related molecule promoter and/or enhancers) to form triple helical structures that prevent transcription of the a melanoma-related molecule gene in target cells. See generally, Helene, C., 1991, Anticancer Drug Des. 6(6):569-84; Helene, C. et al., 1992, Ann. N.Y. Acad. Sci. 660:27-36; and Maher, L. J., 1992, Bioassays 14(12):807-15.

In yet another embodiment, the melanoma-related molecule nucleic acid molecules of the present invention can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al., 1996, Bioorganic & Medicinal Chemistry 4 (1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. et al., 1996, supra; Perry-O'Keefe et al., 1996, Proc. Natl. Acad. Sci. USA 93: 14670-675.

PNAs of a melanoma-related molecule nucleic acid molecules may be used in therapeutic and diagnostic applications. For example, PNAs may be used as antisense or antigene agents for sequence-specific modulation of gene expression by, for example, inducing transcription or translation arrest or inhibiting replication. PNAs of a melanoma-related molecule nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B., 1996, supra)); or as probes or primers for DNA sequencing or hybridization (Hyrup B. et al., 1996, supra; Perry-O'Keefe supra).

In another embodiment, PNAs of a melanoma-related molecule can be modified, (e.g., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of a melanoma-related molecule nucleic acid molecules can be generated which may combine the advantageous properties of PNA and DNA. Such chimeras allow DNA recognition enzymes, (e.g., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B., 1996, supra). The synthesis of PNA-DNA chimeras can be performed as described in Hyrup B., 1996, supra and Finn P. J. et al., 1996, Nucleic Acids Res. 24 (17): 3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, may be used as a between the PNA and the 5' end of DNA (Mag, M. et al., 1989, Nucleic Acid Res. 17: 5973-88). PNA monomers are then coupled in a stepwise manner to produce a chimeric molecule with a 5' PNA segment and a 3' DNA segment (Finn P. J. et al., 1996, supra). Alternatively, chimeric molecules can be synthesized with a 5' DNA segment and a 3' PNA segment (Peterser, K. H. et al., 1975, Bioorganic Med. Chem. Lett. 5: 1119-11124).

In other embodiments, the oligonucleotide may include other appended groups such as peptides (e.g., for targeting host cell receptors in vivo), or agents facilitating transport across the cell membrane (see, e.g., Letsinger et al., 1989, Proc. Natl. Acad. Sci. US. 86:6553-6556; Lemaitre et al., 1987, Proc. Natl. Acad. Sci. USA 84:648-652; PCT Publication No. WO88/09810) or the blood-brain barrier (see, e.g., PCT Publication No. WO89/10134). In addition, oligonucleotides can be modified with hybridization-triggered cleavage agents (See, e.g., Krol et al., 1988, Bio-Techniques 6: 958-976) or intercalating agents. (See, e.g., Zon, 1988, Pharm. Res. 5:539-549). To this end, the oligonucleotide may be conjugated to another molecule, (e.g., a peptide, hybridization triggered cross-linking agent, transport agent, or hybridization-triggered cleavage agent).

Antisense polynucleotides may be produced from a heterologous expression cassette in a transfectant cell or transgenic cell. Alternatively, the antisense polynucleotides may comprise soluble oligonucleotides that are administered to the external milieu, either in the culture medium in vitro or in the circulatory system or in interstitial fluid in vivo. Soluble antisense polynucleotides present in the external milieu have been shown to gain access to the cytoplasm and inhibit translation of specific mRNA species.

The invention features expression vectors for in vivo or in vitro transfection and expression of a melanoma-related molecule (or a biologically active portion thereof) in particular cell types so as to decrease the activity of at least one melanoma-related molecule in said cell. Expression constructs encoding an anti-sense melanoma-related molecule may be administered in any biologically effective carrier, e.g. any formulation or composition capable of effectively delivering the recombinant gene to cells in vivo. Approaches include insertion of the DNA encoding an anti-sense of melanoma-related molecule in viral vectors including recombinant retroviruses, adenovirus, adeno-associated virus, and herpes simplex virus-1, or recombinant bacterial or eukaryotic plasmids. Viral vectors infect cells directly; plasmid DNA can be delivered with the help of, for example, cationic liposomes (lipofectin) or derivatized (e.g. antibody conjugated), polylysine conjugates, gramacidin S, artificial viral envelopes or other such intracellular carriers, as well as direct injection of the gene construct or Ca₂PO₄ precipitation carried out in vivo. It will be appreciated that because transduction of appropriate target cells represents the critical first step in gene therapy, choice of the particular gene delivery system will depend on such factors as the phenotype of the intended target and the route of administration, e.g. locally or systemically. Furthermore, it will be recognized that particular gene constructs provided for in vivo transduction of melanoma-related molecule expression are also useful for in vitro transduction of cells, such as for use in the ex vivo tissue culture systems described below.

A preferred approach for in vivo introduction of nucleic acid into a cell is by use of a viral vector containing the desired nucleic acid. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells which have taken up viral vector nucleic acid.

Retrovirus vectors and adeno-associated virus vectors are generally understood to be the recombinant gene delivery system of choice for the transfer of exogenous genes in vivo, particularly into humans. These vectors provide efficient delivery of genes into cells, and the transferred nucleic acids are stably integrated into the chromosomal DNA of the host. A major prerequisite for the use of retroviruses is to ensure the safety of their use, particularly with regard to the possibility of the spread of wild-type virus in the cell population. The development of specialized cell lines (termed "packaging cells") which produce only replication-defective retroviruses has increased the utility of retroviruses for gene therapy, and defective retroviruses are well characterized for use in gene transfer for gene therapy purposes (for a review see Miller (1990) Blood 76:271). Thus, recombinant retrovirus can be constructed in which part of the retroviral coding sequence (gag, pol, env) has been replaced by nucleic acid encoding one of the subject proteins rendering the retrovirus replication defective. The replication defective retrovirus is then packaged into virions which can be used to infect a target cell through the use of a helper virus by standard techniques. Protocols for producing recombinant retroviruses and for infecting cells in vitro or in vivo with such viruses can be found in Current Protocols in Molecular Biology, Ausubel, F. M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines for preparing both ecotropic and amphotropic retroviral systems include .psi.Crip, .psi.Cre, .psi.2 and .psi.Am. Retroviruses have been used to introduce a variety of genes into many different cell types, including neuronal cells, in vitro and/or in vivo (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad. Sci. USA 88:8377-8381; Chowdhury et al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Pat. No. 4,868,116; U.S. Pat. No. 4,980,286; PCT Application WO 89/07136; PCT Application WO 89/02468; PCT Application WO 89/05345; and PCT Application WO 92/07573). Furthermore, it has been shown that it is possible to limit the infection spectrum of retroviruses and consequently of retroviral-based vectors, by modifying the viral packaging proteins on the surface of the viral particle (see, for example PCT publications WO93/25234 and WO94/06920). For instance, strategies for the modification of the infection spectrum of retroviral vectors include: coupling antibodies specific for cell surface antigens to the viral env protein (Roux et al. (1989) PNAS 86:9079-9083; Julan et al. (1992) J Gen Virol 73:3251-3255; and Goud et al. (1983) Virology 163:251-254); or coupling cell surface receptor ligands to the viral env proteins (Neda et al. (1991) J Biol Chem 266:14143-14146). Coupling can be in the form of the chemical cross-linking with a protein or other variety (e.g. lactose to convert the env protein to an asialoglycoprotein), as well as by generating fusion proteins (e.g. single-chain antibody/env fusion proteins). This technique, while useful to limit or otherwise direct the infection to certain tissue types, can also be used to convert an ecotropic vector into an amphotropic vector.

Moreover, use of retroviral gene delivery can be further enhanced by the use of tissue- or cell-specific transcriptional regulatory sequences that control expression of a melanoma-related molecule gene of the retroviral vector.

Another viral gene delivery system useful in the present invention utilizes adenovirus-derived vectors. The genome of an adenovirus can be manipulated such that it encodes and expresses a gene product of interest but is inactivated in terms of its ability to replicate in a normal lytic viral life cycle. See for example Berkner et al. (1988) Biotechniques 6:616; Rosenfeld et al. (1991) Science 252:431-434; and Rosenfeld et al. (1992) Cell 68:143-155. Suitable adenoviral vectors derived from the adenovirus strain Ad type 5 dl324 or other strains of adenovirus (e.g., Ad2, Ad3, Ad7 etc.) are well known to those skilled in the art. Recombinant adenoviruses can be advantageous in certain circumstances in that they can be used to infect a wide variety of cell types, including airway epithelium (Rosenfeld et al. (1992) cited supra), endothelial cells (Lemarchand et al. (1992) Proc. Natl. Acad. Sci. USA 89:6482-6486), adipocytes (Hertzel et al. (2000) J Lipid Res. 41:1082-1086), hepatocytes (Herz and Gerard (1993) Proc. Natl. Acad. Sci. USA 90:2812-2816) and muscle cells (Quantin et al. (1992) Proc. Natl. Acad. Sci. USA 89:2581-2584). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al. cited supra; Haj-Ahmand and Graham (1986) J. Virol. 57:267). Most replication-defective adenoviral vectors currently in use and therefore favored by the present invention are deleted for all or parts of the viral E1 and E3 genes but retain as much as 80% of the adenoviral genetic material (see, e.g., Jones et al. (1979) Cell 16:683; Berkner et al., supra; and Graham et al. in Methods in Molecular Biology, E. J. Murray, Ed. (Humana, Clifton, N.J., 1991) vol. 7. pp. 109-127). Expression of the inserted melanoma-related molecule gene can be under control of, for example, the E1A promoter, the major late promoter (MLP) and associated leader sequences, the E3 promoter, or exogenously added promoter sequences.

Yet another viral vector system useful for delivery of the DNA encoding an anti-sense melanoma-related molecule is the adeno-associated virus (AAV). Adeno-associated virus is a naturally occurring defective virus that requires another virus, such as an adenovirus or a herpes virus, as a helper virus for efficient replication and a productive life cycle. (For a review see Muzyczka et al. (1992) Curr. Topics in Micro. and Immunol. 158:97-129). It is also one of the few viruses that may integrate its DNA into non-dividing cells, and exhibits a high frequency of stable integration (see for example Flotte et al. (1992) Am. J. Respir. Cell. Mol. Biol. 7:349-356; Samulski et al. (1989) J. Virol. 63:3822-3828; and McLaughlin et al. (1989) J. Virol. 62:1963-1973). Vectors containing as little as 300 base pairs of AAV can be packaged and can integrate. Space for exogenous DNA is limited to about 4.5 kb. An AAV vector such as that described in Tratschin et al. (1985) Mol. Cell. Biol. 5:3251-3260 can be used to introduce DNA into cells. A variety of nucleic acids have been introduced into different cell types using AAV vectors (see for example Hermonat et al. (1984) Proc. Natl. Acad. Sci. USA 81:6466-6470; Tratschin et al. (1985) Mol. Cell. Biol. 4:2072-2081; Wondisford et al. (1988) Mol. Endocrinol. 2:32-39; Tratschin et al. (1984) J. Virol. 51:611-619; and Flotte et al. (1993) J. Biol. Chem. 268:3781-3790).

In addition to viral transfer methods, such as those illustrated above, non-viral methods can also be employed to cause expression of an anti-sense melanoma-related molecule (or a biologically active portion thereof) in the tissue of an animal. Most nonviral methods of gene transfer rely on normal mechanisms used by mammalian cells for the uptake and intracellular transport of macromolecules. In preferred embodiments, non-viral gene delivery systems of the present invention rely on endocytic pathways for the uptake of the subject melanoma-related molecule-encoding DNA by the targeted cell. Exemplary gene delivery systems of this type include liposomal derived systems, poly-lysine conjugates, and artificial viral envelopes.

In clinical settings, the gene delivery systems for therapeutic anti-sense melanoma-related molecule administration can be introduced into a patient by any of a number of methods, each of which is familiar in the art. For instance, a pharmaceutical preparation of the gene delivery system can be introduced systemically, e.g. by intravenous injection. In this case, specific transduction of the protein in the target cells arises from specificity of transfection provided by the gene delivery vehicle, (i.e., cell-type or tissue-type specificity due to transcriptional regulatory sequences controlling recombinant gene expression). In other embodiments, initial delivery of the recombinant gene is more limited with introduction into the animal being quite localized. For example, the gene delivery vehicle can be introduced by catheter (see U.S. Pat. No. 5,328,470) or by stereotactic injection (e.g. Chen et al. (1994) PNAS 91: 3054-3057). The DNA encoding a melanoma-related molecule (or a biologically active portion thereof), can be delivered in a gene therapy construct by electroporation using techniques described, for example, by Dev et al. ((1994) Cancer Treat Rev 20:105-115).

The present invention also includes a naked plasmid DNA compound (such as is taught, for example in Wolff et al., (1990) Science 247:1465-1468) for use in therapy comprising injecting an area of a subject's body with an effective amount. A naked plasmid DNA compound comprises a nucleic acid molecule encoding the desired molecule or biologically active portion thereof, operatively linked to a naked plasmid DNA vector capable of being taken up by and expressed in a recipient cell located in the body area. Preferred naked plasmid DNA vectors of the present invention include those known in the art. When administered to a subject, a naked plasmid DNA compound of the present invention transforms cells within the subject and directs the production of the antisense melanoma related molecule, or biologically active portion thereof, in the cell.

As used herein, an effective amount of a naked plasmid DNA to administer to a subject comprises an amount needed to alleviate at least one symptom of the disease or disorder being treated and, preferably, is an amount sufficient to prevent or cure the disease or disorder. The mode of administration, number of doses and frequency of dose capable of being decided upon, in any given situation, by one of skill in the art without resorting to undue experimentation.

In one embodiment, a recombinant expression vector is introduced into the cell, the vector containing a nucleic acid molecule which encodes an antisense melanoma-related molecule protein. The term "vector" refers to a nucleic acid molecule capable of transporting another nucleic acid to which it has been linked. A preferred vector is an "expression vector" which is capable of directing the expression of gene contained therein. In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions. In a preferred embodiment, genetic engineering is of a subject or patient's own cells which are isolated from the subject or patient's body, transfected or infected according to the techniques described in detail herein, and reintroduced or returned to the body of the subject or patient.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; Gene Expression Technology: Methods in Enzymology 185, Academic Press, San Diego, Calif. (1990). Regulatory sequences include those which direct constitutive expression of a nucleotide sequence in many types of host cells and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, and the like.

When using in mammalian cells, e.g., human cells, the expression vector's control functions are often provided by viral regulatory elements. For example, commonly used promoters are derived from polyoma, Adenovirus 2, cytomegalovirus and Simian Virus 40. Alternatively, tissue-specific regulatory elements are used to control expression of the desired nucleic acid. Tissue-specific regulatory elements are known in the art. Preferred tissue-specific promoters include skin-specific promoters.

Vector DNA can be introduced via conventional transformation or transfection techniques. The terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd, ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding the melanoma-related molecule protein or can be introduced on a separate vector. Cells stably transfected with the introduced nucleic acid can be identified by drug selection (e.g., cells that have incorporated the selectable marker gene will survive, while the other cells die).

The invention also provides for reduction of the expression and/or activity of a melanoma-related molecule in a cell utilizing an intracellular antibody specific for a melanoma-related molecule discussed herein. The use of intracellular antibodies to inhibit protein function in a cell is known in the art (see e.g., Carlson, J. R. (1988) Mol. Cell. Biol. 8:2638-2646; Biocca, S. et al. (1990) EMBO J. 9:101-108; Werge, T.M. et al. (1990) FEBS Letters 274:193-198; Carlson, J.R. (1993) Proc. Natl. Acad Sci. USA 90:7427-7428; Marasco, W.A. et al. (1993) Proc. Natl. Acad. Sci. USA 90:7889-7893; Biocca, S. et al. (1994) Bio/Technology 12:396-399; Chen, S-Y. et al. (1994) Human Gene Therapy 5:595-601; Duan, L et al. (1994) Proc. Natl. Acad. Sci. USA 91:5075-5079; Chen, S-Y. et al. (1994) Proc. Natl. Acad. Sci. USA 91:5932-5936; Beerli, R.R. et al. (1994) J. Biol. Chem. 269:23931-23936; Beerli, R.R. et al. (1994) Biochem. Biophys. Res. Commun. 204:666-672; Mhashilkar, A.M. et al. (1995) EMBO J. 14:1542-1551; Richardson, J.H. et al. (1995) Proc. Natl. Acad. Sci. USA 92:3137-3141; PCT Publication No. WO 94/02610 by Marasco et al.; and PCT Publication No. WO 95/03832 by Duan et al.).

To inhibit protein activity using an intracellular antibody, a recombinant expression vector is prepared which encodes the antibody chains in a form such that, upon introduction of the vector into a cell, the antibody chains are expressed as a functional antibody in an intracellular compartment of the cell. For inhibition of transcription factor activity according to the inhibitory methods of the invention, preferably an intracellular antibody that specifically binds the transcription factor is expressed within the nucleus of the cell. Nuclear expression of an intracellular antibody can be accomplished by removing from the antibody light and heavy chain genes those nucleotide sequences that encode the N-terminal hydrophobic leader sequences and adding nucleotide sequences encoding a nuclear localization signal at either the N- or C-terminus of the light and heavy chain genes (see e.g., Biocca, S. et al. (1990) EMBO J. 9:101-108; Mhashilkar, A. M. et al. (1995) EMBO J. 14:1542-1551). A preferred nuclear localization signal to be used for nuclear targeting of the intracellular antibody chains is the nuclear localization signal of SV40 Large T antigen (see Biocca, S. et al. (1990) EMBO J. 9:101-108; Mhashilkar, A. M. et al. (1995) EMBO J. 14:1542-1551).

To prepare an intracellular antibody expression vector, antibody light and heavy chain cDNAs encoding antibody chains specific for the target protein of interest, e.g., a melanoma-related molecule protein, is isolated, typically from a hybridoma that secretes a monoclonal antibody specific for a melanoma-related molecule protein. For example, antibodies can be prepared by immunizing a suitable subject, (e.g., rabbit, goat, mouse or other mammal) with a melanoma-related molecule protein immunogen. An appropriate immunogenic preparation can contain, for example, recombinantly expressed a melanoma-related molecule protein or a chemically synthesized melanoma-related molecule peptide. The preparation can further include an adjuvant, such as Freund's complete or incomplete adjuvant, or similar immunostimulatory compound. Antibody-producing cells can be obtained from the subject and used to prepare monoclonal antibodies by standard techniques, such as the hybridoma technique as previously described herein.

Alternative to preparing monoclonal antibody-secreting hybridomas, a monoclonal antibody that binds to a melanoma-related molecule can be identified and isolated by screening a recombinant combinatorial immunoglobulin library (e.g., an antibody phage display library) with the protein, or a peptide thereof, to thereby isolate immunoglobulin library members that bind specifically to the protein. Kits for generating and screening phage display libraries are commercially available (e.g., the Pharmacia Recombinant Phage Antibody System, Catalog No. 27-9400-01; and the Stratagene SurfZAP.TM. Phage Display Kit, Catalog No. 240612). Additionally, examples of methods and compounds particularly amenable for use in generating and screening antibody display library can be found in, for example, Ladner et al. U.S. Pat. No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Fuchs et al. (1991) Bio/Technology 9:1370-1372; Hay et al. (1992) Hum Antibod Hybridomas 3:81-85; Huse et al. (1989) Science 246: 1275-1281; Griffiths et al. (1993) EMBO J. 12: 725-734; Hawkins et al. (1992) J Mol Biol 226:889-896; Clarkson et al. (1991) Nature 352: 624-628; Gram et al. (1992) PNAS 89:3576-3580; Garrad et al. (1991) Bio/Technology 9: 1373-1377; Hoogenboom et al. (1991) Nuc Acid Res 19:4133-4137; Barbas et al. (1991) PNAS 88:7978-7982; and McCafferty et al. Nature (1990) 348:552-554.

Once a monoclonal antibody of interest specific for a melanoma-related molecule has been identified (e.g., either a hybridoma-derived monoclonal antibody or a recombinant antibody from a combinatorial library, including monoclonal antibodies to a melanoma-related molecule that are already known in the art), DNAs encoding the light and heavy chains of the monoclonal antibody are isolated by standard molecular biology techniques. For hybridoma-derived antibodies, light and heavy chain cDNAs can be obtained, for example, by PCR amplification or cDNA library screening. For recombinant antibodies, such as from a phage display library, cDNA encoding the light and heavy chains can be recovered from the display package (e.g., phage) isolated during the library screening process. Nucleotide sequences of antibody light and heavy chain genes from which PCR primers or cDNA library probes can be prepared are known in the art. For example, many such sequences are disclosed in Kabat, E. A., et al. (1991) Sequences of Proteins of Immunological Interest, Fifth Edition, U.S. Department of Health and Human Services, NIH Publication No. 91-3242 and in the "Vbase" human germline sequence database.

Once obtained, the antibody light and heavy chain sequences are cloned into a recombinant expression vector using standard methods. As discussed above, the sequences encoding the hydrophobic leaders of the light and heavy chains are removed and sequences encoding a nuclear localization signal (e.g., from SV40 Large T antigen) are linked in-frame to sequences encoding either the amino- or carboxy terminus of both the light and heavy chains. The expression vector can encode an intracellular antibody in one of several different forms. For example, in one embodiment, the vector encodes full-length antibody light and heavy chains such that a full-length antibody is expressed intracellularly. In another embodiment, the vector encodes a full-length light chain but only the VH/CH1 region of the heavy chain such that a Fab fragment is expressed intracellularly. In the most preferred embodiment, the vector encodes a single chain antibody (scFv) wherein the variable regions of the light and heavy chains are linked by a flexible peptide linker (e.g., (Gly₄Ser₃ and expressed as a single chain molecule. To inhibit transcription factor activity in a cell, the expression vector encoding the NIP45-specific intracellular antibody is introduced into the cell by standard transfection methods as described hereinbefore.

### Peptidic compounds

In another embodiment, an inhibitory compound of the invention is a peptidic compound derived from a melanoma-related molecule amino acid sequence. In particular, the inhibitory compound comprises a portion of a melanoma-related molecule (or a mimetic thereof) that mediates interaction of a melanoma-related molecule with a target molecule such that contact of the melanoma-related molecule with this peptidic compound competitively inhibits the interaction of the melanoma-related molecule with the target molecule.

The peptidic compounds of the invention can be made intracellularly in immune cells by introducing into the immune cells an expression vector encoding the peptide. Such expression vectors can be made by standard techniques. The peptide can be expressed in intracellularly as a fusion with another protein or peptide (e.g., a GST fusion). Alternative to recombinant synthesis of the peptides in the cells, the peptides can be made by chemical synthesis using standard peptide synthesis techniques. Synthesized peptides can then be introduced into cells by a variety of means known in the art for introducing peptides into cells (e.g., liposome and the like) or administered to a subject using any suitable route of administration.

### Soluble receptor polypeptides and Fc fusions

Soluble receptor polypeptides can be effective GDF6 antagonists. For example, a soluble portion of a polypeptide binds GDF6, thus inhibiting or reducing an activity of GDF6. Such molecules can be provided as a fusion peptide along with a second peptide which promotes solubility. To illustrate, a soluble peptide can be provided as part of a fusion polypeptide with all or a fragment of the hinge or Fc portion of the immunoglobulin, which can promote solubility and/or serum stability. In certain embodiments, this can include fusion proteins thereof, e.g., soluble GDF6 receptor Fc-fusion polypeptide, also known as "GDF6-TRAP" (International Patent Application publication WO2010114860).

In some embodiments, soluble ALK3 polypeptides can be used to inhibit an activity of melanoma-related molecule, such as GDF6. "ALK3" refers to a family of activin receptor-like kinase-3 (ALK3) (also referred to as bone morphogenetic protein receptor, type IA (BMPR1A), or activin A receptor, type II-like kinase (ACVRLK)) proteins from any species and variants derived from such ALK3 proteins by mutagenesis or other modification. Members of the ALK3 family are generally transmembrane proteins, composed of a ligand-binding extracellular domain with a cysteine-rich region, a transmembrane domain, and a cytoplasmic domain with predicted serine/threonine kinase activity. "Soluble ALK3 polypeptide" generally refers to polypeptides comprising an extracellular domain of an ALK3 protein. The term "soluble ALK3 polypeptide" includes any naturally occurring extracellular domain of an ALK3 protein as well as any variants thereof (including mutants, fragments and peptidomimetic forms). Reference to ALK3 refers to any one of the currently identified forms.

The term "ALK3 polypeptide" includes polypeptides comprising any naturally occurring polypeptide of an ALK3 family member as well as any variants thereof (including mutants, fragments, fusions, and peptidomimetic forms) that retain a useful activity. For example, ALK3 polypeptides include polypeptides derived from the sequence of any known ALK3 having a sequence at least about 80% identical to the sequence of an ALK3 polypeptide, and preferably at least 85%, 90%, 95%, 97%, 99% or greater identity. For example, an ALK3 polypeptide may bind to and inhibit the function of BMPs. Preferably, an ALK3 polypeptide inhibits an activity of GDF6.

A BMP-binding ALK3 polypeptide is one that retains the ability to bind to BMPs, such as to GDF6 (BMP13). Preferably, a BMP-binding ALK3 polypeptide will bind to a BMP with a dissociation constant of 1 nM or less. The amino acid sequence of human ALK3 precursor protein is provided SEQ ID NO:8 (extracellular domain is aa 24-152). The extracellular domain of an ALK3 protein binds to BMP and is generally soluble, and thus can be termed a soluble, BMP-binding ALK3 polypeptide. Additional examples of soluble, BMP-binding ALK3 polypeptides are disclosed in International Patent Application publication WO2010114860. The ALK3(24-152)-Fc fusion polypeptide sequence is shown in SEQ ID NO:9; SEQ ID NOs:8 and 9 are shown in Table 1. ALK3(24-152)-Fc fusion consists of a fusion of the ALK3 extracellular domain of human ALK3 (native residues 24-152) fused C-terminally with a human Fc domain via a linker of sequence TGGG. Other examples of soluble, BMP-binding ALK3 polypeptides comprise a signal sequence in addition to the extracellular domain of an ALK3 protein, for example, the native ALK3 leader sequence, the tissue plasminogen activator (TPA) leader, or the honey bee melittin leader. Functionally active fragments of ALK3 polypeptides can be obtained by screening polypeptides recombinantly produced from the corresponding fragment of the nucleic acid encoding an ALK3 polypeptide. In addition, fragments can be chemically synthesized using techniques such as conventional Merrifield solid phase f-Moc or t-Boc chemistry. The fragments can be produced (recombinantly or by chemical synthesis) and tested to identify those peptidyl fragments that can function as antagonists (inhibitors) of ALK3 protein or signaling mediated by BMPs, such as GDF6.

Different elements of the fusion proteins may be arranged in any manner that is consistent with the desired functionality. For example, an ALK3 polypeptide may be placed C-terminal to a heterologous domain, or, alternatively, a heterologous domain may be placed C-terminal to an ALK3 polypeptide. The ALK3 polypeptide domain and the heterologous domain need not be adjacent in a fusion protein, and additional domains or amino acid sequences may be included C- or N-terminal to either domain or between the domains.

It is noted that recombinant ALK3(24-152)-Fc fusion when produced using CHO cells may be heterogeneous in that some amino acids encoded by the leader sequence remain at the N-terminus (e.g., Ala and Gly) (see International Patent Application publication WO2010114860 for further details).

| Table 1 | |
|---|---|
| ALK3(24-152)-Fc fusion sequences | |
| SEQ ID NO:8 | |
| | |
| | |
| | |
| SEQ ID NO:9 | |
| | |
| | |
| | |

Other inhibitory agents that may be used to specifically inhibit the activity of a melanoma-related molecule protein are chemical compounds that directly inhibit melanoma-related molecule activity or inhibit the interaction between melanoma-related molecules and target molecules. Such compounds can be identified using screening assays that select for such compounds.

### EXAMPLES

### Example 1: GDF6 is recurrently amplified in human and zebrafish melanomas

To study melanoma we have used the zebrafish, *Danio rerio,* because genetic studies can be conducted rapidly and a melanoma model we developed in zebrafish closely mimics tumors that are observed in humans. Human melanomas have complex genomes, and it is difficult to determine which of the genetic changes present in a tumor contributed to the onset of the tumor and which genetic changes occurred incidentally and had no impact on tumor progression. With the goal of identifying novel melanoma genes, we compared the genomes of zebrafish melanomas with those of human melanomas. Changes shared by tumors in both species are likely to be important, and we used this approach to derive a short list of candidate oncogenes. GDF6 was on this short list because it is recurrently amplified in melanomas of both species - most zebrafish melanomas have excess copies of the region of chromosome 19 that contains the *gdf6b* gene (the zebrafish *GDF6* ortholog); in human melanomas, the interval containing *GDF6* is frequently amplified. Specifically, extra copies of *GDF6* are present in human melanomas, and extra copies of a zebrafish *gdf6b,* are present in melanomas from zebrafish. See Examples 17 and 18 for further details.

### Example 2: GDF6 and gdf6b mRNA is greater in tumors than normal cells and tissue

Recurrently amplified intervals are enriched for oncogenes, and these oncogenes typically act through overexpression of their mRNA and protein products. We analyzed *GDF6* mRNA expression in both human and zebrafish melanomas and found that its expression in tumors is greater than in normal melanocytes (Figs. 1, 2). Furthermore, we stained human tissues with antibodies that recognize GDF6 protein and found that the GDF6 protein is present in melanomas but absent from normal melanocytes. Similar findings were observed in zebrafish using antibodies that specifically recognize the zebrafish gdf6b protein. These findings are consistent with the possibility that *GDF6* could act as an oncogene through overexpression of its normal gene product during the process of tumor initiation and progression. See Examples 18 and 19 for further details.

### Example 3: Phosphorylated SMAD1/5/8 is present in melanomas but not normal melanocytes

GDF6 is a bone morphogenetic protein (BMP) family member. BMP factors are secreted proteins that typically act by binding to BMP receptors and signaling through SMAD1/5/8 transcription factors. To determine whether melanomas had active signaling through this pathway, we stained human tissues using an antibody that is specific for the activated, phosphorylated variant of SMAD 1/5/8. As with GDF6 protein itself, phosphorylated SMAD 1/5/8 was present in melanomas but not normal melanocytes. See Example 19 for further details.

### Example 4: Melanomas arise quickly in a zebrafish model when gdf6b is expressed in melanocytes

We used studies in zebrafish and human tissue culture as well as xenotransplantation to determine that GDF6 affects melanoma progression. To test the effect of a gene on melanoma progression in zebrafish, we use a system in which we reconstitute melanocytes in a melanoma-prone strain and ask whether a gene expressed in these reconstituted melanocytes affects melanoma onset. When *gdf6b* was expressed in this system, melanomas arose more quickly (Fig. 3), indicating it is an oncogene that accelerates melanoma progression. See Example 20 for further details.

### Example 5: GDF6 appears to act through SMAD1/5/8 and other components of the BMP signaling pathway to enable melanoma cell survival

Cultured human melanoma cells were used to study the effects of *GDF6* knockdown. Using multiple, independent shRNAs to achieve knockdown, we found that reduction of *GDF6* in melanoma cell lines caused a reduction in proliferation (Figs. 4A-D) that was brought about by induction of apoptosis, or programmed cell death (Fig. 6A-B). Knockdown of SMAD1/5/8 caused similar defects (Figs. 5A-D; Fig. 7), and in an orthogonal approach, a small molecule inhibitor was used to abrogate BMP signaling activity, and growth defects were also observed (Fig. 8A-C). Taken together, these data suggest that GDF6 acts through SMAD1/5/8 and other components of the BMP signaling pathway to enable melanoma cell survival. See Example 22 for further details.

### Example 6: GDF6 knockdown reduces melanoma growth in vivo

An art-accepted model to test for an agent's effects on melanoma is that of using immunocompromised ("nude") mice which are then injected subcutaneously with melanoma cells, and then concomitant with injection of the cells, sometime after injection, and/or after tumor establishment, the test agent is appropriately administered (e.g., intravenously, peritoneally, orally, etc.). Control and treated animals are monitored in part for tumor growth, tumor stabilization, and tumor regression (see for example, Yang et al. 2010. Cancer Res., 70:5518-5527 and Bollag et al. 2010. Nature. 467:596-599).

To determine if GDF6 knockdown affects the tumor initiating capability of cultured melanoma cells, we knocked GDF6 down in A375 melanoma cells and xenotransplanted these cells into nude mice (Fig. 9). As expected, knockdown of GDF6 caused impaired tumor initiation as compared to knockdown of a control gene. GDF6 encodes a pro-protein that is cleaved to generate mature GDF6, which is secreted from the cell. See Example 20 for further details.

### Example 7: Following GDF6 knockdown, proliferation of melanoma cells can be restored by supplementing with recombinant GDF6 protein

To determine if secreted GDF6 can facilitate the growth of melanoma cells, we knocked down GDF6 using shRNAs and then treated these knockdown cells with recombinant GDF6 protein (Fig. 10). Knockdown cells treated with recombinant GDF6 were able to sustain their proliferation, suggesting that the secreted, extracellular form of GDF6 is an important, pro-survival ligand for melanoma cells.

### Example 8: Summary of examples and GDF6 knockdown in cells expressing a constitutively active SMAD1 variant

Regions of recurrent gene copy number variation (CNV) contain genes that affect tumor progression. To identify these genes we defined regions of recurrent CNV in zebrafish melanomas and compared these regions to ones recurrently altered in human melanomas. In the set of genes that were recurrently amplified in both species we found the BMP factor GDF6 (Example 1). Because we found BMP signaling to be upregulated in melanomas, we investigated the role of GDF6 in melanoma progression. In analyses of both zebrafish and humans, GDF6 mRNA and protein were upregulated in melanomas as compared to normal melanocytes (Example 2). In functional assessments, we found that overexpression of GDF6 accelerated melanoma onset in zebrafish (Example 4). Furthermore, knockdown of GDF6 in melanoma cell lines led to apoptotic cell death in culture in vitro and in mice xenotransplants in vivo (Examples 5, 6). Addition of recombinant GDF6 protein to the media rescued the melanoma cells from undergoing GDF6 shRNA-induced apoptosis, suggesting that GDF6 acts as a secretory factor in aiding melanoma cell survival (Example 4). GDF6, like other BMP factors, is predicted to signal through SMAD1/5/8 transcription factors, and similar growth defects were observed when SMAD1 was knocked down in melanoma cells (Example 5).

To further define the relationship between GDF6 and SMAD1 in melanoma, GDF6 knockdown was performed in cells expressing a constitutively active SMAD1 variant. This variant rescued the death caused by GDF6 knockdown, suggesting that, at least in part, GDF6 acts through SMAD1 to promote melanoma cell survival. These data establish a role for BMP signaling in melanoma and identify a novel secretory factor, GDF6, which mediates this role. GDF6 is an excellent therapeutic target due to its secretory nature and its expression in a majority of human melanomas.

### Example 9: Overexpression of GDF6 accelerates melanoma formation in mouse xenografts

Melanoma cells were transfected with a Lenti CMV vector or a Lenti CMV vector containing the *GDF6* construct. Expression of *GDF6* protein was upregulated in those cells receiving the *GDF6* construct, but not in those cells receiving the empty vector (Fig. 12A). When the cells were transplanted into mice, tumor volumes rapidly increased over time in *GDF6-*overexpressing cells, whereas tumor volumes for cells transfected with empty vector increased much more slowly (Fig. 12B). These results suggest that *GDF6*-overexpression accelerates melanoma formation in mouse xenografts. See Example 20 for further details.

### Example 10: GDF6 overexpression reduces basal levels of cell dell death in mouse xenografts

The transplanted cells from Example 9 were examined for cell death markers. When tissue from xenografts containing cells overexpressing *GDF6* are subjected to TUNEL staining, about only 1% of cells were stained; in contrast, when control cells (empty vector transfected cells) were similarly stained, a little over 3% of the cells showed TUNEL staining (Fig. 13A). Similar results were observed when the cells were stained with another cell death marker, cleaved caspase 3, wherein cell death is reduced by half in *GDF6*-overexpressing cells when compared to controls (1% *GDF6* vs. about 2% control; Fig. 13B). GDF6 ortholog overexpressing melanomas were observed to negatively correlate with a pro-apoptotic signature (Fig. 13C). These results suggest that *GDF6* overexpression reduces basal levels of cell death in mouse xenografts. See Example 21 for further details.

### Example 11: GDF6 or SMAD1 knockdown abrogates melanoma growth in mouse xenografts in vivo

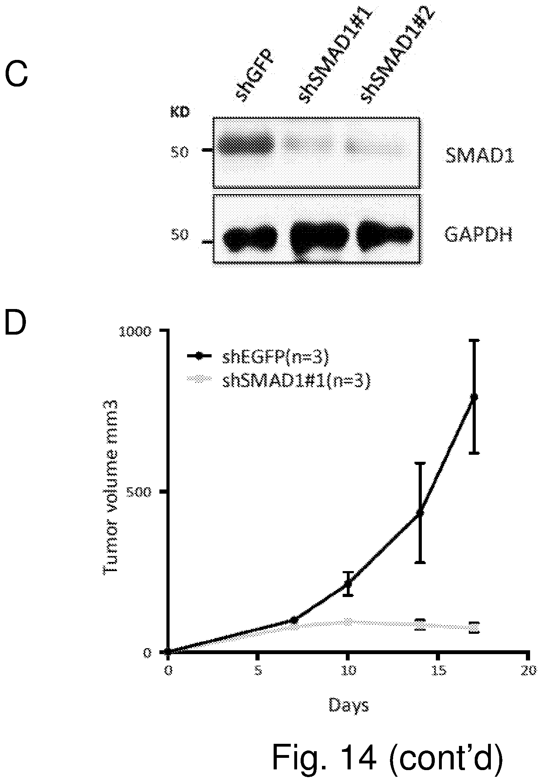

Melanoma cells where treated with shRNAs, including two targeted to *GDF6,* shGDF6#1 and shGDF6#2, and a control shRNA, shGFP. When *GDF6* protein expression was examined in shRNA-treated cells, it was observed that shGDF6#1 partially inhibited *GDF6* protein expression while shGDF6#2 was more effective than shGDF6#1 when compared to shGFP-treated controls (Fig. 14A). When these cells are transplanted to mice, shGDF6#2-treated cells showed very little tumor formation as measured by tumor volume, while shGDF6#1-treated cells showed tumors intermediate between the control-treated (shEGFP) cells and the shGDF6#2-treated cells (Fig. 14B). Similar results were observed for cells treated with shRNA targeting *SMAD1. SMAD1* protein expression was significantly decreased for cells treated with shSMAD1#1 and shSMAD1#2 when compared to control-treated cells (treated with shGFP; Fig. 14C). When transplanted in mice, control cells formed tumors over time as measured by tumor volume, while shSMAD1#1-treated cells formed only very small tumors over the same time period (Fig. 14D). See Examples 20 and 22 for further details.

### Example 12: GDF6 knockdown induces cell death in mouse xenografts in vivo

When transplanted cell xenograft tissue from Example 11 was observed for TUNEL staining, only about 2.5% of control (shGFP-treated) cells showed staining, while about 10% of GDF6-knocked down cells stained (Fig. 15A). Similar results were obtained staining for another cell death marker, cleaved caspase 3. Staining of control cells (shGFP) was observed at about 2%, while about 7% of *GDF6*-knocked down cells were stained. These results suggest that *GDF6*-knockdown induces cell death in mouse xenografts *in vivo.* See Example 21 for further details.

### Example 13: SMAD1 phosphomimic can rescue the clonogenic potential of melanoma cells after GDF6 knockdown in vitro and in vivo

Melanoma cells were transfected with a vector comprising a FLAG-tagged SMAD1DVD, encoding a SMAD1 variant that is a phosphomimic of SMAD1 or the same vector without the SMAD1DVD construct. Protein expression analysis showed good expression of SMAD1DVD protein (as detected by the FLAG epitope), while empty vector-transfected control cells showed no staining (Fig. 16A). These cells were then treated with an array of shRNAs, including control (shGFP) shRNA, and shRNAs targeting *GDF6.* The shRNA-treated cells were then subjected to a clonogenic growth assay. When *GDF6* was knocked down in cells transfected with the empty vector, few colonies formed when compared to the control (empty vector-transfected cells treated with shGFP; Fig. 16B). When cells expressing the SMAD1 phosphomimic were treated with shRNAs targeting *GDF6* (shGDF6#1 and shGDF6#2) and subjected to the same clonogenic growth assay, the ability to form colonies was partially restored (Fig. 16B). When these cells were transplanted to mice to make xenografts, empty vector control cells and *SMAD1* phosphomimic expressing cells, both treated with the control shRNA, shGFP, formed tumors over time (Fig. 16C). However, when the *SMAD1* phosphomimic cells have *GDF6* knocked down with either shGDF6#1 or shGDF6#2, tumor formation accelerated after day 15 (Fig. 16D). However, in cells not expressing the phosphomimic (empty vector-transfected cells) and *GDF6* expression knocked down with either shGDF6#1 or shGDF6#2, only small tumors formed (Fig. 16D). These results suggest that *SMAD1* phosphomimic can rescue the clonogenic potential of melanoma cells after *GDF6* knockdown *in vitro.* See Example 22 for further details.

### Example 14: A BMP pathway small molecule inhibitor, DMH1, abrogates melanoma growth in mouse xenografts in vivo

Mouse melanoma xenografts were treated with a BMP pathway small molecule inhibitor (DMH1) or vehicle only. In DMH1-treated cells, smaller tumors arose and grew more slowly as measured by tumor volume then those treated with vehicle alone (Fig. 17). See Example 22 for further details.

### Example 15: GDF6 expression correlates with poor melanoma patient survival

Survival of subjects suffering from low *GDF6* expressing-tumors and high *GDF6* expressing-tumors was plotted over time; Fig. 18 shows the results. Over time, fewer of those suffering from high *GDF6* expressing-tumors survived when compared to those suffering from low *GDF6* expressing-tumors. These observations suggest that GDF6 expression correlates with poor melanoma patient survival. See Example 24 for further details.

### Example 16: Effect of activin receptor-like kinase 3 (ALK3)-Fc fusion on melanoma cells

ALK3 binds GDF6 (Mazerbourg et al. 2005. J Biol Chem 280:32122-0320 and can potentially trap GDF6 ligand in vivo. For these experiments, an ALK3-Fc fusion will be used; this fusion is between a human immunoglobulin Fc domain and the extracellular domain of human ALK3 (see International Patent Application publication WO2010114860 for further details).

In a first experiment, ALK3-Fc will be added to cultured melanoma cells and measures of cell viability will be monitored to understand if ALK3-Fc can recapitulate the effect of GDF6 knockdown.

Melanoma cells will be maintained in culture using standard methods in either DMEM+FBS or minimal medium. ALK3-Fc will be added to culture media (at 0.5-1µg/mL media) and measures of cell growth and viability will be determined. These measures will include cell counting, cell viability assessed using MTT (3-(4,5-dimethylthiazol-2-yl)-2,5-diphenyltetrazolium bromide), and assessing colony formation. Initially two lines of A375 melanoma cells will be treated. One line is stably transfected with a vector construct; it is anticipated that this cell line will be sensitive to ALK3-Fc-mediated inhibition of GDF6. Another line is stably transfected with a SMAD1-DVD construct; it is anticipated that this cell line will be resistant to ALK3-Fc-mediated inhibition because SMAD1-DVD over-activates BMP signaling downstream of the ALK3-Fc block. In previous experiments, A375+vector cells were sensitive to GDF6 shRNA inhibition whereas A375+SMAD1-DVD cells were resistant. The use of both cell lines is important to establish specificity of ALK3-Fc treatment; that is, it is important to establish that ALK3-Fc is not generally cytotoxic.

In a second experiment, melanomas will be established in immunocompromised mice. Mice with tumors (see Example 6 for description of mouse model) will be injected with ALK3-Fc and tumor regression measured. Initial experiments will treat mice with tumors seeded by established melanoma cell lines so that drug effect and dosage can be determined. Secondary experiments will treat mice with tumors derived from patient tumors. Such experiments will be used to determine if ALK3-Fc has efficacy against a heterogeneous tumor cell population that has not been altered by culture conditions.

In initial experiments in this phase, nude mice will be injected with A375+vector cells or A375+SMAD1-DVD cells then treated with ALK3-Fc (2mg/kg intraperitoneal injection every other day). As described above for the *in vitro* phase, it is anticipated that tumors derived from A375+vector cells will be sensitive to ALK3-Fc whereas tumors derived from A375+SMAD1-DVD will be resistant. These experiments will be conducted firstly with ALK3-Fc treatment beginning simultaneously with tumor cell inoculation. Secondly, regression experiments will be performed in which ALK3-Fc treatment begins after inoculation, when small, palpable tumors have already been established. Subsequent experiments will be performed using patient-derived xenografts established in NSG immunodeficient mice. Similar treatment regimens as above will be performed in which treatment in one group will begin simultaneously with inoculation, and treatment in another group will commence only when palpable tumors have been established. Together these studies will obtain useful pre-clinical measures of ALK3-Fc efficacy.

### Example 17: Comparative genomic analysis of genes that are recurrently amplified in human melanomas

We hypothesized that a cross-species comparative approach with zebrafish would aid in identification of cancer genes in regions of broad CNVs. Humans and zebrafish are diverged by 420 million years, and the genomic reorganization that has occurred over time is predicted to frequently place orthologous driver genes next to different neighboring genes in each species. Consequently, orthologous driver genes would be altered in both species, but changes to neighboring passenger genes would be limited to a single species. To test this hypothesis, we sought to compare genes that are recurrently amplified in human melanomas, roughly 10% of the genome, to genes recurrently amplified in zebrafish melanomas. Using melanomas that arose autochthonously in a *Tg(mitfa:BRAFV600E); p53(lf)* zebrafish strain (E. E. Patton et al., BRAF mutations are sufficient to promote nevi formation and cooperate with p53 in the genesis of melanoma. Current biology : CB 15, 249-254 (2005)), we performed array comparative genomic hybridization (aCGH) to generate CNV profiles (Fig. 19A). aCGH values were analyzed with the JISTIC algorithm (F. Sanchez-Garcia, U. D. Akavia, E. Mozes, D. Pe'er, JISTIC: identification of significant targets in cancer. BMC bioinformatics 11, 189 (2010)) to define recurrently varied intervals, which largely overlapped with intervals obtained in independent studies of zebrafish melanomas. Recurrently amplified genes from JISTIC intervals were compared to their human orthologs. The degree of overlap between orthologs amplified in both species is greater than would be expected by chance (Fig. 19B) suggesting that amplification of similar driver genes mechanistically underlies tumor formation in both species. As further indication of mechanistic conservation, known melanoma drivers were recurrently amplified in both species, including *TERT, MYC* and *SETDB1.*

### Example 18: Comparative expression analysis of genes that are recurrently amplified in human melanomas

Expression analyses were used to further winnow the list of candidates. Since copy-number-amplified driver genes predominantly act by upregulation of wild-type transcripts, we obtained transcriptional profiles of zebrafish melanomas and normal melanocytes. Genes recurrently amplified in both species and transcriptionally upregulated in zebrafish included *gdf6b* (Figs. 19C. 19H, and 19I), an ortholog of the BMP factor *GDF6* (a.k.a. *BMP13*). Human genes often have two zebrafish orthologs because of a partial genome duplication in the teleost lineage. The second zebrafish ortholog of human *GDF6, gdf6a,* was not recurrently amplified but was among the most transcriptionally upregulated genes in melanomas as compared to melanocytes (Figs. 19C and 19I).

### Example 19: GDF6 is amplified and, along with BMP signaling, upregulated in melanomas

Encoding BMP ligands, *GDF6* genes are predicted to act through *SMAD1*/*5*/*8* transcription factors. Using an antibody that specifically recognizes phosphorylated SMAD1/5/8 proteins to monitor BMP pathway activity, we found robust phospho-SMAD 1/5/8 nuclear staining in zebrafish melanomas (Fig. 19D). Furthermore, transcriptome analyses indicated robust upregulation in melanomas of genes that support BMP signaling, including the *BMPR1A* and *BMPR2* receptor subunits, through which GDF6 is known to act (Fig. 19E). Transcriptome analyses also indicated that *GDF6* orthologs were the only BMP ligands upregulated in zebrafish melanomas (Fig. 19F). Furthermore, immunostaining with antibodies specific to zebrafish GDF6 orthologs showed expression of GDF6 proteins specifically in melanomas (Figs. 19D and 19G). Taken together, these data indicate that the BMP pathway is active in zebrafish melanomas and that GDF6 orthologs may be critical in this activation.

### Example 20: GDF6 modulation affects growth and survival of melanoma cells

We next assessed how a gain or loss of *GDF6* affected melanoma progression. In zebrafish, we elevated *gdf6b* expression in melanocytes in a *Tg(mitfa:BRAFV600E); p53(lf)* background using the miniCoopR system, as previously described (C. J. Ceol et al., The histone methyltransferase SETDB1 is recurrently amplified in melanoma and accelerates its onset. Nature 471, 513-517 (2011)). Fish with *gdf6b*-overexpressing melanocytes had accelerated melanoma onset (median onset = 13 weeks) as compared to *EGFP*-expressing controls (median onset = 17 weeks) (Figs. 3 and 20A). In A375 human melanoma cells, which are *BRAFV600E-*mutant, overexpression of *GDF6* resulted in improved anchorage-independent growth in soft agar assays (Figs. 12A, 20B,and 20C), although there was no marked difference in the proliferation rate (Fig. 21A) or anchorage-dependent growth (Fig. 21B) as compared to control cells. When transplanted into immunocompromised mice, *GDF6*-overexpressing cells displayed elevated tumorigenic potential (Figs.12B and 20D). To determine the effects of *GDF6* loss, we knocked down *GDF6* in A375 cells using multiple, independent shRNAs (Figs. 14A and 20E). *GDF6* knockdown led to a growth disadvantage *in vitro* (Figs. 20F and 21C) and caused a pronounced reduction in tumorigenic potential in mouse xenografts as compared to an *EGFP* knockdown control (Figs. 14B and 20G). Collectively these data indicate that *GDF6* acts as an oncogene via elevated expression and its knockdown leads to an abrogation of tumorigenic potential.

### Example 21: Effects of GDF6 modulation on A375 melanoma cell growth and survival

We further investigated the consequences of *GDF6* modulation. To begin with, we performed massively parallel RNA sequencing (RNA-seq) on *GDF6*-overexpressing A375 cells as compared to empty vector controls. Gene set enrichment analysis (GSEA) revealed that *GDF6* expression negatively correlated with expression of apoptotic pathway genes (Fig. 20H). Because elevated *GDF6* expression was linked to downregulation of apoptotic genes, we hypothesized that knockdown of *GDF6* would cause upregulation of apoptotic genes, leading to death of melanoma cells. Indeed, RNA-seq of *GDF6* knockdown cells showed upregulation of the same suite of apoptotic genes (Fig. 21D). In directly measuring apoptosis in A375 melanoma cells with *GDF6* knockdown, we found an increase in AnnexinV levels (Fig. 21E) and Caspase 3/7 activity (Fig. 20I). SK-MEL-28 (*BRAFV600E*-mutant) and MeWo (*BRAF* wild-type) melanoma cell lines also had increased cell death and showed growth defects *in vitro* upon *GDF6* knockdown (Figs.22A-22E). *In vivo,* staining of A375 mouse xenografts showed increased TUNEL (Fig. 20J) and cleaved caspase3-positivity (Fig. 21F) in *GDF6* knockdown tumors as compared to controls. By contrast, mouse xenografts with GDF6 overexpression showed reduced TUNEL (Figs. 13A and 20K) and cleaved caspase3 staining (Figs. 13B and 21G) as compared to basal levels of cell death in control xenografts. GDF6 overexpression xenografts had a slightly increased Ki67 proliferative index, suggesting that the reduction in cell death was not caused by an obligate coupling of cell death to cell proliferation (Fig. 21H). The involvement of *GDF6* in cell death is consistent with findings in which loss of *GDF6* orthologs in fish and *Xenopus* cause a significant increase in apoptosis during eye and neural development. In sum, *GDF6* is an essential survival factor for melanoma cells, and its loss leads to apoptotic cell death.

### Example 22: GDF6 and SMAD1 modulation alter expression of BMP pathway targets ID1 and ID3

Robust phospho-SMAD 1/5/8 staining in zebrafish melanomas suggested that GDF6, like other BMP ligands, acts via the SMAD 1/5/8 signaling cascade. To more directly test this possibility, we modulated pathway activity in A375 melanoma cells. *GDF6* knockdown in A375 cells led to a decrease in phospho-SMAD 1/5/8 levels (Fig. 23A) as well as reduced transcripts of *ID1* and *ID3* (Fig. 24), genes that are BMP signaling targets and directly regulated by SMAD proteins. Knockdown of *SMAD1* led to defects in cell growth and tumorigenic potential, as well as increased cell death (Figs. 14D, 23B, and Fig. 25); similar to what was observed in *GDF6* knockdown cells. Inhibition of BMP signaling with the small molecule DMH1 similarly led to decreased tumorigenicity (Figs. 17 and 23C). To test the relationship of *GDF6* to *SMAD1* in epistasis analyses, we expressed a phosphomimetic variant of *SMAD1, SMAD1DVD,* in A375 cells (Fig. 26A). Whereas A375 cell growth and tumorigenic potential were abrogated by *GDF6* knockdown, A375 cells expressing *SMAD1DVD* were rescued, exhibiting robust growth in colony formation (Fig. 26B) and xenotranplantation assays (Figs. 16C and 23D). In addition, *SMAD1DVD* also reversed the apoptotic induction and reduction in *ID1* and *ID3* gene expression caused by *GDF6* knockdown (Figs. 15B, 23E, 23F, 24, 26C, and 26D). Growth defects caused by treatment with DMH1 were also reversed by *SMAD1DVD* (Fig. 27). These data indicate that *GDF6* acts, at least in part, through *SMAD1,* and by extension the *SMAD1*/*5*/*8* axis, in promoting melanoma cell survival.

### Example 23: GDF6 acts via SMAD1 to promote melanoma cell survival and modulate neural crest gene expression

To further investigate the effect of GDF6 and BMP signaling on melanomas, we compared transcriptomes of A375 cells in which *GDF6* or *SMAD1* activity was modulated. We defined the set of genes that was differentially regulated upon *GDF6* knockdown and showed reciprocal differential regulation in *SMADIDVD*-expressing cells that were subjected to *GDF6* knockdown (Fig. 23G). Pathway analysis showed that this gene set most significantly overlapped with genes defining ossification and neural crest pathways. Interestingly, included amongst the reciprocally regulated genes found in the neural crest pathway were *FOXD3, SNAI2, SOX10* and others that are known to promote survival of neural crest cells and their derivatives (Figs. 23H and Fig. 28). These pro-survival genes were downregulated upon *GDF6* knockdown but were reciprocally upregulated in *SMAD1DVD-*expressing cells with *GDF6* knockdown. Melanocytes initially develop in the neural crest, and a suite of genes that promotes proliferation and migration of neural crest cells is turned off in melanocytes upon terminal differentiation. However, melanomas express some of these neural crest genes, and adopting a neural crest-like identity can contribute to the aggressive nature of melanoma cells. During development *GDF6* and its orthologs are regulators of neural crest cells and their derivatives, and loss of *GDF6* is linked to death of neural crest-derived tissues. We speculate that GDF6 promotes a neural crest identity of melanoma cells, invoking pro-survival mechanisms that normally function in the embryonic neural crest.

### Example 24: GDF6 expression and prognostic significance in patient-derived melanomas

We next assessed expression of *GDF6* in human melanomas and examined potential clinical implications. From an analysis of Cancer Genome Atlas and other publicly available datasets, *GDF6* transcripts were widely present in human melanoma patient samples, but were nearly absent in normal melanocytes (Fig. 29A). In support of our finding that *GDF6* is a pro-survival gene, GSEA using human melanoma samples showed that the previously described apoptotic gene signature once again negatively correlated with *GDF6* expression (Fig. 29B). Immunohistochemistry on melanoma patient samples detected high levels of GDF6 protein in melanomas; however, normal melanocytes of adjacent skin (Fig. 29C) or tumor-infiltrating cells (Fig. 30) rarely expressed GDF6. In the same samples, we found high BMP pathway activity, as measured by phospho-SMAD1/5/8 staining, in human melanomas but little to no BMP activity in melanocytes of normal adjacent skin (Fig. 29D). To determine whether expression of GDF6 was correlated with melanoma patient clinical characteristics, we performed immunohistochemistry on a microarray with 104 patient melanoma tissue cores (78 primary melanomas and 26 metastatic melanomas). Consistent with the initial cohort, robust GDF6 expression was observed in a majority of melanomas (80% of total; n=104 cases) (Fig. 29E). Importantly, Kaplan-Meier curves show that patients with high GDF6 melanomas had significantly lower survival probabilities than patients with no or low GDF6 melanomas at diagnosis (Fig. 29E). Experiments described above show that *GDF6* promotes melanoma progression, and these immunohistochemistry data indicate that tumors expressing GDF6 are associated with a poorer prognosis and are thus likely to be more aggressive.

### Example 25: GDF6 knockdown and SMAD1DVD alter expression of genes important for survival of neural crest cells and their derivatives

In this study, comparative and integrative genomics identified *GDF6* as a gene that is recurrently amplified and transcriptionally upregulated in melanomas. Functional analyses point to its role, as well as that of BMP pathway signaling, in promoting melanoma cell survival. During embryonic development, *GDF6* and its orthologs are expressed in the neural crest, adjacent neural tube, and neural crest-derived cartilage and retinal cell precursors. Loss of *GDF6* orthologs in mice, Xenopus and zebrafish leads to skeletal and ocular abnormalities, the latter of which is caused by inappropriate death of retinal precursor cells. These studies are consistent with the notion that *GDF6* normally functions to protect tissue progenitors from cell death, and its upregulation, by chromosomal amplification or other means, in melanomas and other tumors prevents cell death, thereby facilitating tumor growth (Fig. 29F). Knockdown studies indicate that *GDF6* inhibition slows tumor growth. As a secreted molecule, GDF6 inhibition could be accomplished *in vivo* by a variety of means, including cell-impermeable therapies. While great strides have been made in melanoma treatment through the use of targeted therapies and immune checkpoint inhibitors, a majority of patients with advanced disease still succumb within five years of diagnosis. Targets such as GDF6 represent excellent therapeutic opportunities to further treat this lethal disease.

### Example 26: Materials and methods

### Cell lines and cell culture

A375, MeWo and HEK293T cells (ATCC) were maintained in Dulbecco's Modified Eagle's Medium (DMEM) and SK-MEL-28 cells (ATCC) were maintained in Roswell Park Memorial Institute (RPMI) 1640 media supplemented with 10% fetal bovine serum (FBS) and 2 ug/ml Pen Strep (Gibco) at 37°C and 5% CO₂. Cells cultured at the same time were pooled, counted and then seeded in a 10cm dish. Wells/dishes were then subjected to treatment with lentiviral vectors.

### Lentiviral infection

Lentiviral infections were performed as described previously (C. J. Ceol et al., The histone methyltransferase SETDB1 is recurrently amplified in melanoma and accelerates its onset. (Nature 471, 513-517 (2011)). For stable gene knockdowns, we used pLKO-1 lentiviral vectors to deliver short hairpin sequences (shRNAs) (obtained from the RNAi Consortium (TRC)/Broad Institute through the UMMS RNAi core facility) specific for *GDF6* (GDF6.1: TRCN0000141818, target sequence: GCCAAGTGTTACATTGAGCTT (SEQ ID NO:10); GDF6.2: TRCN0000140097, target sequence: GTGTCCATGCTCTCAGACAAA (SEQ ID NO:11)) or *SMAD1* (SMAD1.1: TRCN0000021781, target sequence: CGGTTGCTTATGAGGAACCAA (SEQ ID NO:12); SMAD1.2: TRCN0000021782, target sequence: GCCGATGGACACAAACATGAT (SEQ ID NO:13)) or *EGFP* (TRCN0000072181, target sequence: ACAACAGCCACAACGTCTATA (SEQ ID NO:14)). Virus was made using a second generation lentiviral packaging system in HEK293T cells and titrated using a p24 ELISA kit (Clontech). Cells were infected with virus at a multiplicity of infection of 2.5 with 8 µg/ml polybrene followed by puromycin selection (2 µg/ml) for 2 days in appropriate media. For overexpression, we used Gateway cloning (Life Technologies) to insert the *GDF6* ORF (GE life sciences) or *SMAD1DVD* ORF (provided by Takenobu Katagiri) into the pLenti CMV Hygro DEST (w117-1) vector (provided by Paul Kaufman). Infection and monitoring was performed as described (C. J. Ceol et al., The histone methyltransferase SETDB1 is recurrently amplified in melanoma and accelerates its onset. (Nature 471, 513-517 (2011)), except that selection was done with 300 ug/ml hygromycin for 10 days.

### Growth curve, clonogenic and soft agar assays

For growth curves 50,000 live cells were seeded per well in a 6-well tissue culture plate on day 0. The numbers of live cells were calculated every day using an automated cell counter (Nexcelom Bioscience Cellometer Auto T4) following standard procedures. All assays were performed with technical replicates. For clonogenic assays, 3,000 live cells were seeded in a 10 cm tissue culture plate. After 3 weeks, colonies were fixed and stained using bromophenol blue in acetone. ImageJ was used to quantify the number of colonies. In assays with DMH1 treatment, control or DMH1-containing media was replaced every other day. For soft agar assays a 0.5% bottom layer (1:1 with 1% agar and 2X DMEM with 20% FBS) and a 0.3% top layer (1:1 with 0.6% agar and 2X DMEM with 20% FBS) were used. 3,000 live cells per well of a 6-well tissue culture plate were added in the top layer. Media was added initially then replaced every 3 days. After 3 weeks, colonies were stained with nitroblue tetrazolium chloride overnight at 37°C. Once stained, individual wells were photographed, and ImageJ was used to count the number of colonies. All these assays were done in triplicate, and experiments were repeated at least twice. A representative image of each has been shown.

### Cell death assays

A375 melanoma cells after knockdown of *GDF6* or *SMAD1* or *EGFP* were stained for Annexin V and 7-AAD (BD Pharmingen PR Annexin V Apoptosis Detection kit) as per manufacturer's instructions, followed by flow cytometry using a FACSCalibur instrument (BD Biosciences). Caspase3/7 activity was measured using the Caspase-Glo 3/7 assay (Promega) as per manufacturer's instructions.

### Animal experiments

All animal protocols were approved by the UMMS Institution Animal Care and Use Committee (A-2016, A-2171). Mice were randomly allocated to individual experimental groups. No blinding was done as animal groups were identified by tagging and cage labeling. Animals were excluded, according to pre-established criteria, if the tumor volume reached >1,000 mm³; if tumor size or location affected the mobility or general health of animal, the animal was euthanized and excluded from the experiment or the complete experiment was terminated. A375 cells stably expressing an *EGFP* or *GDF6* or *SMAD1* shRNA and/or empty vector or *GDF6-*expressing vector or *SMAD1DVD*-overexpressing vector were subcutaneously injected into the flanks of 6-8-week-old BALB/c nu/nu female mice (Taconic Farms) to produce orthotopic primary tumor. Primary tumor growth was monitored every 3 days with calipers, and the tumor volume was calculated as described previously (C. Gazin, N. Wajapeyee, S. Gobeil, C. M. Virbasius, M. R. Green, An elaborate pathway required for Ras-mediated epigenetic silencing. Nature 449, 1073-1077 (2007)). For *GDF6* knockdown, *SMAD1* knockdown and epistasis experiments, 1 x 10⁷ live cells were injected. For *GDF6* overexpression experiments, 1 x 10⁶ live cells were injected. For *GDF6* overexpression and *GDF6* knockdowns, a representative of two independent experiments (*n*=3 animals per experiment) is shown. For DMH1 drug experiments, 1 x 10⁶ live A375 cells were subcutaneously injected in the flanks of BALB/c nu/nu female mice. Beginning on the day cells were injected, mice were injected intraperitoneally with vehicle 12.5% 2-hydroxypropyl-β-cyclodextrin or 25 mg/kg DMH1 in vehicle every other day. The growth of the tumor was monitored as described above. This experiment was repeated twice and the weighted average of both experiments (*n*=8 total) is represented.

### miniCoopR assay

The miniCoopR assay measuring the effect of *gdf6b* on melanoma onset in zebrafish was performed as previously described (C. J. Ceol et al., The histone methyltransferase SETDB1 is recurrently amplified in melanoma and accelerates its onset. Nature 471, 513-517 (2011)). For miniCoopR-*EGFP* experiments a weighted average of two independent experiments is represented, and for miniCoopR-*gdf6b* experiments a weighted average of four independent experiments is represented.

### cDNA synthesis and qRT-PCR

For zebrafish, total RNA was extracted from melanoma cells and from normal scale-associated melanocytes of *Tg(mitfa:BRAF(V600E)); p53*^{*-*/*-*}; *alb*^{*-*/}*⁻; Tg(mitfa:EGFP)* zebrafish. For isolation of melanoma cells, melanomas were dissected, dissociated using Liberase TH treatment and subjected to Fluorescence Activated Cell Sorting (FACS) to isolate EGFP-positive cells. The same protocol was used for normal melanocytes, except dorsal scales from zebrafish were plucked to isolate melanocytes. Total RNA from zebrafish melanomas and melanocytes was isolated using Trizol-chloroform extraction, followed by RNA clean up (Qiagen RNeasy). Total RNA was reverse transcribed using the Superscript 2 Reverse Transcriptase kit (Invitrogen). Quantitative real-time PCR with SYBR green master mix (Biorad) was performed using the following primers: *gdf6a* F: CTGAGAAACTGGGGCTCAAT, (SEQ ID NO:15) *gdf6a* R: CGACCAGCTCCTCTTTGTCT (SEQ ID NO:16), *gdf6b* F: CGTCTAAAGCAGCAAACACC (SEQ ID NO:17), *gdf6b* R: CCAAAGTGGAGAGTTCAAATGG (SEQ ID NO:18), *actb1* F: CGAGCAGGAGATGGGAACC (SEQ ID NO:19), *actb1* R: CAACGGAAACGCTCATTGC (SEQ ID NO:20).

For A375 human melanoma cells with *GDF6* and/or *SMAD1DVD* modulation, total RNA was prepared in the same manner, and quantitative real-time PCR was performed using the following primers: *ID1* F: CCAACGCGCCTCGCCGGATC (SEQ ID NO:21), *ID1* R: CTCCTCGCCAGTGCCTCAG (SEQ ID NO:22), *ID3* F: CTGGACGACATGAACCACTG (SEQ ID NO:23), *ID3* R: GTAGTCGATGACGCGCTGTA (SEQ ID NO:24), *GAPDH* F: TGCACCACCAACTGCTTAGC (SEQ ID NO:25), *GAPDH* R: GGCATGGACTGTGGTCATGAG (SEQ ID NO:26).

### Immunofluorescence

For adults, dorsal scales bearing normal melanocytes or melanomas were plucked from anesthetized zebrafish. After fixation, scales were bleached of melanin pigment to visualize fluorescence after staining. Scales were incubated with primary antibody (Gdf6b (1:250), Mitfa (1:250)) overnight. Subsequently the scales were washed, incubated in appropriate secondary antibodies (Jackson Labs), incubated with DAPI and mounted on slides with Vectashield (Vectorlabs). Fluorescence was visualized using confocal fluorescence microscopy.

### Immunoblotting

Protein extracts were prepared as previously described (S. D. Epstein LB, Hunte-McDonough B, harris CA, Cytokines: A pratical approach (Balkwill FR., ed). 81-93 (1991)). Extracts were separated on 12% SDS-PAGE gels. Blots were probed with primary antibodies (GDF6 (Sigma PRS4691; 1:1000), phospho-SMAD1/5/8 (Cell Signaling 13820;1:1000), SMAD1 (Cell Signaling 9743; 1:500), Total SMAD1/5/8 (Santa Cruz sc-6031-R; 1:1000), FLAG (Sigma F3165, 1:2000), GAPDH (Abcam 8245; 1:2000)) overnight at 4°C, washed five times in TBS plus 0.1% Tween (TBST) and then incubated with the appropriate HRP-conjugated secondary antibody (Jackson Labs) for 1 hour at room temperature. Membranes were washed five times in TBST and visualized on autoradiography film after incubating with ECL reagent (Supersignal West Pico or Supersignal West Femto; Thermo Scientific).

### Immunohistochemistry (IHC) and TUNEL staining

From mouse xenografts, formalin-fixed, paraffin-embedded tissues were processed to obtain 5µm sections. Sections were stained with H&E, GDF6 (Sigma PRS4691; 1:1000), pSMAD1/5/8 (Sigma 9511; 1:150), cleaved caspase3 (Cell signaling 9664; 1:100), Ki-67 (Dako M7240; 1:100) and evaluated. TUNEL staining was performed on sections using the In Situ Cell Death Detection kit (Roche) as per manufacturer's protocol. For stained mouse xenograft sections, 100 independent fields from sections of three different tumors per genotype were imaged. The numbers of TUNEL-positive or cleaved caspase 3-positive or Ki67-positive cells were counted manually and the total number of cells in each field was calculated using ImageJ software. Individual patient melanoma and tissue microarray cores consisted of 5 µm sections of formalin-fixed, paraffin-embedded tissues. Slides were first deparaffinized with two changes of xylene, and rehydrated with changes of decreasing concentrations of alcohols, then rinsed in distilled water. Antigen retrieval was carried out with 0.01M citrate buffer at pH 6.0, or 0.001M EDTA at pH 8.0. Slides were heated in a 770W microwave oven for 14 minutes, cooled to room temperature, and rinsed in distilled water. The sections were first blocked for endogenous nonspecific protein and peroxidase activity with an application of Dual Endogenous Block (Dako) for 10 minutes, followed by a buffer wash followed by staining with antibodies recognizing GDF6 (Sigma PRS4691; 1:1000) and p-SMAD1/5/8 (Cell signaling 9664; 1:100) for 30 minutes. Staining with a second antibody recognizing GDF6 (Sigma HPA045206; 1:100) yielded concordant results. For negative controls, non-immune immunoglobulin G (a cocktail of Mouse Whole IgG and Rabbit Whole IgG (Pierce antibodies 31204 and 31207, respectively; both 1ug/ml)) staining was used. Following a buffer wash, sections were incubated with the EnVision+ Dual Link (Dako) detection reagent for 30 minutes. The sections were washed, and treated with a solution of diaminobenzidine and hydrogen peroxide (Dako) for 10 minutes, to produce the visible brown pigment. After rinsing, a toning solution (DAB Enhancer, Dako) was used for 2 minutes to enrich the final color. The sections were counterstained with hematoxylin, dehydrated, and coverslipped with permanent mounting media. Positive staining was defined as dark brown staining pattern, confined to the expected cellular region. Scant, or fine granular background staining, or no staining was considered negative. Zebrafish formalin-fixed, 5mM EDTA treated, paraffin-embedded tissues were processed to obtain 5µm transverse sections. Sections were stained as mentioned above with GDF6 (Sigma PRS4691; 1:1000) and p-SMAD 1/5/8 (Sigma 9511; 1:150) and also counterstained with hematoxylin, dehydrated, and coverslipped with permanent mounting media.

### IHC scoring

For both the UMass patient cohort and the tissue microarray, a modified visual semiquantitative method was used. Sections were scored for immunointensity (0-4) and immunopositivity (0-3), which were then multiplied. For the UMass patient cohort, scoring was done by C.J.C. and A.M.V., and the scores were averaged. Scores were verified by A.D. For the tissue microarray cohort, scoring was conducted independently by C.L. and C.B.F.G. and the scores were averaged. Sections with scores less than or equal to four were considered in the low or no staining group and sections with scores greater than four were considered in high staining group.

### aCGH probe design

We custom designed the G3 array format of 2x400K probes for the Zebrafish ZV9 genome assembly using Agilent's eArray (eArray id# 036041). The array has 398426 unique probes covering 97% of the zebrafish genome (based on Zv9 assembly). The probes are 60 bases long and are spaced across the genome with an average separation of 3550 bases.

### aCGH, JISTIC analysis and comparative analysis

aCGH was performed as per Agilent's array-based genomic DNA hybridization protocol. Briefly, genomic DNA was extracted from zebrafish melanomas or a normal region of the same fish using the Qiagen DNeasy Blood and Tissue kit. 5 µg of tumor or matched normal gDNA was fragmented to 200-500bp by sonication (Covaris S220R High Performance Sample Preparation Ultrasonicator System 220x S), labeled in a random-primed reaction using Cy5-dCTP or Cy3-dCTP, respectively, and hybridized in Agilent's hybridization buffer with Cot1 DNA (1mg/ml) at 65°C overnight. Arrays were then washed, and Cy5 and Cy3 signals were measured using an Agilent G2565 Microarray Scanner. Raw data was generated from scanned images with the Agilent Feature Extraction software (v10.7). Raw values were normalized using the Agilent Genomic workbench and copy number alterations were detected. The JISTIC algorithm was used in limited peel-off mode to calculate significantly altered regions, and peak calling was done using a q-value cut-off of 0.25. Gene-based JISTIC G-scores and -log10 transformed Q-values are represented using the Circos package (M. Krzywinski et al., Circos: an information aesthetic for comparative genomics. Genome research 19, 1639-1645 (2009)). For representation of data, the G-score scale for amplifications was 0 (minimum) and 1550 (maximum), and for deletions it was 0 (minimum) and 2150 (maximum). The -log10-transformed Q-value scale for both amplifications and deletions was 0 (minimum) and 11 (maximum). For human melanomas, copy number data was downloaded from Tumorscape (R. Beroukhim et al., The landscape of somatic copy-number alteration across human cancers. Nature 463, 899-905 (2010); W. M. Lin et al., Modeling genomic diversity and tumor dependency in malignant melanoma. Cancer research 68, 664-673 (2008)), and JISTIC analysis was conducted as described above. Genes from within peaks were pooled to define species-specific sets of recurrently amplified genes. Human orthologs of zebrafish genes were determined using Ensembl (J. E. Collins, S. White, S. M. Searle, D. L. Stemple, Incorporating RNA-seq data into the zebrafish Ensembl genebuild. Genome research 22, 2067-2078 (2012); K. Howe et al., The zebrafish reference genome sequence and its relationship to the human genome. Nature 496, 498-503 (2013)), and supplemented by performing BLAST (S. F. Altschul, W. Gish, W. Miller, E. W. Myers, D. J. Lipman, Basic local alignment search tool. Journal of molecular biology 215, 403-410 (1990)). Recurrently amplified zebrafish and human genes, as determined by JISTIC, were compared to find the overlapping set of commonly amplified genes.

### cDNA amplification and gene expression analysis

Total RNA was extracted and prepared from melanoma cells and from normal scale-associated melanocytes of *Tg(mitfa:BRAF(V600E)); p53*^{*-*/}*⁻; alb*^{*-*/}*⁻; Tg(mitfa:EGFP)* zebrafish as described above. Total RNA was amplified using the Nugen Ovation RNA Amplification system V2 as per manufacturer's protocol. For microarrays, amplified cDNA was hybridized to a 385K microarray (NimbleGen 0711105Zv7EXPR) as per manufacturer's protocol. Briefly, amplified cDNA from melanomas and melanocytes were labeled with Cy3 and Cy5 independently, hybridized to the microarray, washed and scanned with a GenePix 4000B Scanner. Images were analyzed and normalized using NimbleScan software, and differentially expressed genes were identified.

### Massively parallel RNA sequencing

For zebrafish melanomas and melanocytes, total RNA was isolated as described above and libraries were prepared using the TrueSeq Stranded mRNA Library Prep Kit as per manufacturer's protocol (Illumina). FASTQ files were analyzed using FASTQC v0.10.1 (A. S, FastQC: a quality control tool for high throughput sequence data (2010)) to ensure uniform read quality (phred>30). Paired-end reads were aligned to the zebrafish genome using STAR v2.3 (A. Dobin et al., STAR: ultrafast universal RNA-seq aligner. (Bioinformatics (Oxford, England) 29, 15-21 (2013)) (Zv9). The mapped reads were counted using htseq-count (v0.6.0, parameters -t exon) (S. Anders, P. T. Pyl, W. Huber, HTSeq--a Python framework to work with high-throughput sequencing data. Bioinformatics (Oxford, England) 31, 166-169 (2015)) and gene models from the Ensembl transcriptome (K. Howe et al., The zebrafish reference genome sequence and its relationship to the human genome. (Nature 496, 498-503 (2013)). Analyses of differential gene expression were performed using DESeq2 (M. E. Ritchie et al., limma powers differential expression analyses for RNA-sequencing and microarray studies. Nucleic acids research 43, e47 (2015)). Orthology to human genes was determined using Ensembl (J. E. Collins, S. White, S. M. Searle, D. L. Stemple, Incorporating RNA-seq data into the zebrafish Ensembl genebuild. Genome research 22, 2067-2078 (2012); K. Howe et al., The zebrafish reference genome sequence and its relationship to the human genome. Nature 496, 498-503 (2013)), and supplemented by performing BLAST (S. F. Altschul, W. Gish, W. Miller, E. W. Myers, D. J. Lipman, Basic local alignment search tool. (Journal of molecular biology 215, 403-410 (1990)). The heatmap of BMP pathway genes (REACTOME_SIGNALING_BY_BMP; MSigDB (Broad Institute)) was developed with expression of human orthologs of fish BMP pathway genes. The fish orthologs of human genes represented are *SMAD5*=*smad5, SMAD4*=*si:dkey-239n17.4, ACVR2A*=*acvr2a, ACVR2B*=*acvr2b, BMPR1A*=*bmpr1aa, FSTL1*=*fstl1b, SMAD7*=*smad7, BMPR2*=*bmpr2a, SMURF2*=*smurf2, SMAD6*=*smad6b, ZFYVE16*=*zfyve16, SKI*=*skib, GREM2*=*grem2, SMURF1*=*smurf1, UBE2D1*=*ube2d1, CER1*=*dand5, NOG*=*nog, BMP2*=*bmp2b, BMPR1B*=*bmpr1bb.*For A375 human melanoma cells with *GDF6* and/or *SMAD1DVD* modulation, total RNA was isolated and libraries prepared as described above. Prepared libraries were sequenced using Illumina Hiseq technology (NY Genome Center). FASTQC v0.10.1 (A. S, FastQC: a quality control tool for high throughput sequence data. (2010)) was used on the FASTQ sequences for the A375 samples to generate sequence quality reports. Data were analyzed using two different bioinformatics pipelines. In the first pipeline, reads were aligned to the human reference genome (Ensembl GRCh37) using Bowtie2 (v 2-2.1.0) (B. Langmead, S. L. Salzberg, Fast gapped-read alignment with Bowtie 2. Nature methods 9, 357-359 (2012)) and Tophat2 (v 2.0.9) (D. Kim et al., TopHat2: accurate alignment of transcriptomes in the presence of insertions, deletions and gene fusions. (Genome biology 14, R36 (2013)). Samtools (v 0.0.19) (H. Li et al., The Sequence Alignment/Map format and SAMtools. Bioinformatics (Oxford, England) 25, 2078-2079 (2009)) and IGV (v 2.3.60) (H. Thorvaldsdottir, J. T. Robinson, J. P. Mesirov, Integrative Genomics Viewer (IGV): high-performance genomics data visualization and exploration. Briefings in bioinformatics 14, 178-192 (2013)) were used for indexing the alignment files and viewing the aligned reads, respectively. Gene expression was quantitated as fragments per kilobase of exon model per million mapped fragments (FPKM) using Cufflinks (v 2.2.0) (C. Trapnell et al., Differential gene and transcript expression analysis of RNA-seq experiments with TopHat and Cufflinks. Nature protocols 7, 562-578 (2012)). Differentially-expressed genes were identified using the Cufflinks tools (Cuffmerge and Cuffdiff). erbund (v 2.4.1) (C. Trapnell et al., Differential gene and transcript expression analysis of RNA-seq experiments with TopHat and Cufflinks. Nature protocols 7, 562-578 (2012)) was used to assess replicate concordance. In the second pipeline, reads were mapped against the human reference genome (Ensembl GRCh37) using the aligner STAR (v 2.4.2a), and gene level counts of uniquely mapped reads were obtained using htseq-count (v 0.6.1) (S. Anders, P. T. Pyl, W. Huber, HTSeq--a Python framework to work with high-throughput sequencing data. Bioinformatics (Oxford, England) 31, 166-169 (2015)). Differential expression analysis was performed using DESeq2 (M. I. Love, W. Huber, S. Anders, Moderated estimation of fold change and dispersion for RNA-seq data with DESeq2. Genome biology 15, 550 (2014)) for each pairwise condition using a p-adj threshold of 0.05. The FPKM-based method and the counts-based method generated concordant results. Analyses using the FPKM-based method have been represented in results.

### Gene set enrichment analysis and pathway analysis

For GSEA, the enrichment score (ES), normalized enrichment score (NES) and familywise error rate (FWER) were calculated based on a running metric, which increased when a gene (vertical line in the graphical representation) in the gene set was encountered and decreased when one was not. For GSEA of the apoptotic pathway gene signature, a rank-ordered gene list was made with FPKM values from *GDF6*-overexpressing A375 melanoma cells as compared to empty vector control cells or A375 cells expressing an shRNA targeting *EGFP* as compared to *GDF6.1* shRNA-expressing cells. Default parameters of GSEA were used and the Student's *t*-test was used to calculate significance. Pathway analysis was performed using the WEB-based Gene SeT AnaLysis Toolkit (WebGestalt) (J. Wang, D. Duncan, Z. Shi, B. Zhang, WEB-based GEne SeT AnaLysis Toolkit (WebGestalt): update 2013. Nucleic acids research 41, W77-83 (2013); B. Zhang, S. Kirov, J. Snoddy, WebGestalt: an integrated system for exploring gene sets in various biological contexts. Nucleic acids research 33, W741-748 (2005)). Default parameters were used, except the minimum number of genes for a category was set to 10. For GSEA based on TCGA samples, a rank-ordered gene list was derived from the expression profiles of 385 melanoma samples, using *GDF6* expression level as a continuous variable. Default parameters of GSEA were used, and Pearson correlation was used to calculate significance.

### Human melanocyte and melanoma transcriptome analysis

Three hundred and eighty-five human RNA-seq samples were downloaded from the Cancer Genomics Hub (CGHub) (cghub.ucsc.edu) using GeneTorrent (v 3.8.5a). The RNA-seq TCGA dataset is comprised of three sample types: 302 metastatic melanoma samples, 82 primary melanomas, and 1 solid tissue normal (Genomic Classification of Cutaneous Melanoma. Cell 161, 1681-1696 (2015)). For the normal melanocyte datasets, two RNA-seq samples were downloaded from the Short Read Archive (SRA) (www.ncbi.nlm.nih.gov/sra/; accession codes: SRR522118, SRR522119) (T. Barrett et al., NCBI GEO: archive for functional genomics data sets--update. Nucleic acids research 41, D991-995 (2013)) and four from the ENCODE project (www.encodeproject.org/; experiment: ENCSROOOCUQ) (An integrated encyclopedia of DNA elements in the human genome. Nature 489, 57-74 (2012)). The datasets downloaded from TCGA, SRA and ENCODE were aligned to the human reference genome (Ensembl GRCh37) and analyzed using the FPKM-based method described above.

## Claims

1. A GDF6 inhibitor for use in treating a melanoma in a subject, comprising administering to a subject in need thereof an effective amount of said inhibitor.

2. The GDF6 inhibitor for use of claim 1, wherein the subject is a non-human mammal or a human.

3. The GDF6 inhibitor for use of claim 1, wherein inhibiting GDF6 activity causes apoptosis of at least one melanoma cell.

4. The GDF6 inhibitor for use of claim 1, wherein inhibiting GDF6 activity reduces downstream signaling from a BMP receptor with which the GDF6 interacts when compared to a suitable control.

5. The GDF6 inhibitor for use of claim 4, wherein reducing the GDF6 activity reduces SMAD1/5/8 phosphorylation.

6. The GDF6 inhibitor for use of claim 1, wherein the inhibitor is selected from the group consisting of an antibody, an antibody fragment, an anti-sense nucleic acid, a soluble receptor polypeptide, and a small molecule.

7. The GDF6 inhibitor for use of claim 6, wherein the anti-sense nucleic acid is selected from the group consisting of a RNAi, a ribozyme, an α-anomeric nucleic acid molecule, and a peptide nucleic acid.

8. The GDF6 inhibitor for use of claim 7, wherein the anti-sense molecule is an RNAi molecule and is a siRNA or a shRNA.

9. The GDF6 inhibitor for use of claim 6, wherein the soluble receptor polypeptide comprises an ALK3 polypeptide.

10. The GDF6 inhibitor for use of claim 9, wherein the soluble receptor polypeptide comprises an ALK3(24-152)-Fc fusion polypeptide comprising an amino acid sequence of SEQ ID NO:9.

11. A GDF6 inhibitor for use in treating a melanoma in a subject, comprising
(a) obtaining a sample from a suspected area of the subject's skin;
(b) determining the presence of GDF6;
(c) obtaining a suitable control;
(d) comparing the presence of GDF6 in the sample of step (b) with the suitable control of step (c);
(e) determining if the subject displays a melanoma, wherein the presence of GDF6 in the sample but not in the suitable control indicates melanoma;
(f) treating the subject with said GDF6 inhibitor to inhibit GDF6 if the subject has melanoma.

12. The GDF6 inhibitor for use of claim 11, wherein the subject is a non-human mammal, or the subject is a human.

13. The GDF6 inhibitor for use of claim 11, wherein inhibiting GDF6 activity causes apoptosis of at least one melanoma cell, or inhibiting the GDF6 activity reduces downstream signaling from a BMP receptor with which the GDF6 interacts when compared to a suitable control, optionally wherein reducing GDF6 activity reduces SMAD1/5/8 phosphorylation.

14. The GDF6 inhibitor for use of claim 11, wherein the inhibitor is selected from the group consisting of an antibody, an antibody fragment, anti-sense nucleic acid, a soluble receptor polypeptide, and a small molecule, optionally wherein the anti-sense nucleic acid is selected from the group consisting of a RNAi, a ribozyme, an α-anomeric nucleic acid molecule, and a peptide nucleic acid, optionally wherein the anti-sense molecule is an RNAi molecule and is a siRNA or a shRNA.

15. The GDF6 inhibitor for use of claim 14, wherein the soluble receptor polypeptide comprises an ALK3 polypeptide, optionally wherein the soluble receptor polypeptide comprises an ALK3(24-152)-Fc fusion polypeptide comprising an amino acid sequence of SEQ ID NO:9.

## Patentansprüche

1. Ein GDF6-Inhibitor zur Verwendung bei der Behandlung eines Melanoms bei einem Patienten, umfassend die Verabreichung einer wirksamen Menge des Inhibitors an einen Patienten, der ihn benötigt.

2. Der GDF6-Inhibitor zur Anwendung gemäß Anspruch 1, wobei der Patient ein nichtmenschliches Säugetier oder ein Mensch ist.

3. Der GDF6-Inhibitor zur Verwendung gemäß Anspruch 1, wobei die Hemmung der GDF6-Aktivität die Apoptose mindestens einer Melanomzelle verursacht.

4. Der GDF6-Inhibitor zur Verwendung gemäß Anspruch 1, wobei die Hemmung der GDF6-Aktivität den stromabwärts gerichteten Signalweg eines BMP-Rezeptor, mit dem GDF6 interagiert, im Vergleich zu einer geeigneten Kontrolle reduziert.

5. Der GDF6-Inhibitor zur Verwendung gemäß Anspruch 4, wobei die Reduzierung der GDF6-Aktivität die SMAD1/5/8-Phosphorylierung reduziert.

6. Der GDF6-Inhibitor zur Verwendung gemäß Anspruch 1, wobei der Inhibitor aus der Gruppe ausgewählt ist, die aus einem Antikörper, einem Antikörperfragment, einer Antisense-Nukleinsäure, einem löslichen Rezeptorpolypeptid und einem 'Small Molecule' besteht.

7. Der GDF6-Inhibitor zur Verwendung gemäß Anspruch 6, wobei die Antisense-Nukleinsäure aus der Gruppe ausgewählt ist, die aus einer RNAi, einem Ribozym, einem α-anomeren Nukleinsäuremolekül und einer Peptid-Nukleinsäure besteht.

8. Der GDF6-Inhibitor zur Verwendung gemäß Anspruch 7, wobei das Antisense-Molekül ein RNAi-Molekül ist und eine siRNA oder eine shRNA ist.

9. Der GDF6-Inhibitor zur Verwendung gemäß Anspruch 6, wobei das lösliche Rezeptorpolypeptid ein ALK3-Polypeptid umfasst.

10. Der GDF6-Inhibitor zur Verwendung gemäß Anspruch 9, wobei das lösliche Rezeptorpolypeptid ein ALK3(24-152)-Fc-Fusionspolypeptid umfasst, das eine Aminosäuresequenz gemäß SEQ ID NO:9 umfasst.

11. Ein GDF6-Inhibitor zur Verwendung bei der Behandlung eines Melanoms bei einem Patienten, umfassend
(a) Entnahme einer Probe aus einem verdächtigen Hautbereich des Patienten;
(b) Bestimmung des Vorhandenseins von GDF6;
(c) Gewinnung einer geeigneten Kontrolle;
(d) Vergleichen des Vorhandenseins von GDF6 in der Probe von Schritt (b) mit der geeigneten Kontrolle von Schritt (c);
(e) Bestimmen, ob der Patient ein Melanom aufweist, wobei das Vorhandensein von GDF6 in der Probe, aber nicht in der geeigneten Kontrolle auf ein Melanom hinweist;
(f) Behandlung des Patienten mit dem GDF6-Inhibitor, um GDF6 zu hemmen, wenn der Patient ein Melanom hat.

12. Der GDF6-Inhibitor zur Anwendung gemäß Anspruch 11, wobei der Patient ein nichtmenschliches Säugetier oder der Patient ein Mensch ist.

13. Der GDF6-Inhibitor zur Verwendung gemäß Anspruch 11, wobei die Hemmung der GDF6-Aktivität die Apoptose mindestens einer Melanomzelle bewirkt oder die Hemmung der GDF6-Aktivität den stromabwärts gerichteten Signalweg eines BMP-Rezeptor, mit dem GDF6 interagiert, im Vergleich zu einer geeigneten Kontrolle reduziert, optional wobei die Reduzierung der GDF6-Aktivität die SMAD1/5/8-Phosphorylierung reduziert.

14. Der GDF6-Inhibitor zur Verwendung gemäß Anspruch 11, wobei der Inhibitor aus der Gruppe ausgewählt ist, die aus einem Antikörper, einem Antikörperfragment, einer Anti-Sense-Nukleinsäure, einem löslichen Rezeptorpolypeptid und einem 'Small molecule' besteht, optional wobei die Anti-Sense-Nukleinsäure aus der Gruppe ausgewählt ist, die aus einer RNAi, einem Ribozym, einem α-anomeren Nukleinsäuremolekül und einer Peptidnukleinsäure besteht, optional wobei das Anti-Sense-Molekül ein RNAi-Molekül und eine siRNA oder eine shRNA ist.

15. Der GDF6-Inhibitor zur Verwendung gemäß Anspruch 14, wobei das lösliche Rezeptorpolypeptid ein ALK3-Polypeptid umfasst, optional wobei das lösliche Rezeptorpolypeptid ein ALK3(24-152)-Fc-Fusionspolypeptid umfasst, das eine Aminosäuresequenz gemäß SEQ ID NO:9 umfasst.

## Revendications

1. Inhibiteur de GDF6 pour utilisation dans le traitement d'un mélanome chez un sujet, comprenant le fait d'administrer ledit inhibiteur, en une quantité efficace, à un sujet qui en a besoin.

2. Inhibiteur de GDF6 pour utilisation conforme à la revendication 1, où le sujet est un mammifère non humain ou un humain.

3. Inhibiteur de GDF6 pour utilisation conforme à la revendication 1, où inhiber l'activité de GDF6 provoque l'apoptose d'au moins une cellule de mélanome.

4. Inhibiteur de GDF6 pour utilisation conforme à la revendication 1, où inhiber l'activité de GDF6 réduit, par comparaison avec un témoin approprié, la signalisation aval à partir d'un récepteur de BMP avec lequel le GDF6 interagit.

5. Inhibiteur de GDF6 pour utilisation conforme à la revendication 4, où réduire l'activité de GDF6 réduit la phosphorylation de SMAD1/5/8.

6. Inhibiteur de GDF6 pour utilisation conforme à la revendication 1, lequel inhibiteur est choisi dans l'ensemble constitué par un anticorps, un fragment d'anticorps, un acide nucléique antisens, un polypeptide récepteur soluble et une petite molécule.

7. Inhibiteur de GDF6 pour utilisation conforme à la revendication 6, où l'acide nucléique antisens est choisi dans l'ensemble formé par un ARNi, un ribozyme, une molécule d'acide nucléique α-anomère et un acide nucléique peptidique.

8. Inhibiteur de GDF6 pour utilisation conforme à la revendication 7, où la molécule antisens est une molécule d'ARNi et est un siARN (petit ARN interférent) ou un shARN (petit ARN en épingle à cheveux).

9. Inhibiteur de GDF6 pour utilisation conforme à la revendication 6, où le polypeptide récepteur soluble comprend un polypeptide ALK3.

10. Inhibiteur de GDF6 pour utilisation conforme à la revendication 9, où le polypeptide récepteur soluble comprend un polypeptide de fusion ALK3(24-152)-Fc qui comporte une séquence d'acides aminés de séquence SEQ ID NO : 9.

11. Inhibiteur de GDF6 pour utilisation dans le traitement d'un mélanome chez un sujet, comprenant :
a) prélever un échantillon d'une zone suspecte de la peau du sujet,
b) déterminer la présence de GDF6,
c) se procurer un échantillon témoin approprié,
d) comparer, pour ce qui est de la présence de GDF6, l'échantillon de l'étape (b) avec le témoin approprié de l'étape (c),
e) déterminer si le sujet présente un mélanome, étant entendu que la présence de GDF6 dans l'échantillon, mais pas dans le témoin approprié, est l'indice d'un mélanome,
f) et traiter le sujet avec ledit inhibiteur de GDF6 pour inhiber le GDF6, si le sujet présente un mélanome.

12. Inhibiteur de GDF6 pour utilisation conforme à la revendication 11, où le sujet est un mammifère non humain ou un humain.

13. Inhibiteur de GDF6 pour utilisation conforme à la revendication 11, où inhiber l'activité de GDF6 provoque l'apoptose d'au moins une cellule de mélanome, ou bien où inhiber l'activité de GDF6 réduit, par comparaison avec un témoin approprié, la signalisation aval à partir d'un récepteur de BMP avec lequel le GDF6 interagit, et en option, où réduire l'activité de GDF6 réduit la phosphorylation de SMAD1/5/8.

14. Inhibiteur de GDF6 pour utilisation conforme à la revendication 11, lequel inhibiteur est choisi dans l'ensemble constitué par un anticorps, un fragment d'anticorps, un acide nucléique antisens, un polypeptide récepteur soluble et une petite molécule, étant entendu que, en option, l'acide nucléique antisens est choisi dans l'ensemble formé par un ARNi, un ribozyme, une molécule d'acide nucléique α-anomère et un acide nucléique peptidique, et que, en option, la molécule antisens est une molécule d'ARNi et est un siARN ou un shARN.

15. Inhibiteur de GDF6 pour utilisation conforme à la revendication 14, où le polypeptide récepteur soluble comprend un polypeptide ALK3, étant entendu que, en option, le polypeptide récepteur soluble comprend un polypeptide de fusion ALK3(24-152)-Fc qui comporte une séquence d'acides aminés de séquence SEQ ID NO : 9.


### SEQUENCE LISTING

<110> Ceol, Craig Venkatesan, Arvind
<120> TARGETING GDF6 AND BMP SIGNALING FOR ANTI-MELANOMA THERAPY
<130> UM9-202PC
<150> 62/130,749
   <151> 2015-03-10
<150> 62/209,789
   <151> 2015-08-25
<150> 62/245,614
   <151> 2015-10-23
<150> 62/164,304
   <151> 2015-05-20
<160> 26
<170> PatentIn version 3.5
<210> 1
   <211> 21
   <212> DNA
   <213> Homo sapiens
<400> 1
   gccaagtgtt acattgagct t 21
<210> 2
   <211> 21
   <212> DNA
   <213> Homo sapiens
<400> 2
   gtgtccatgc tctcagacaa a 21
<210> 3
   <211> 21
   <212> DNA
   <213> Homo sapiens
<400> 3
   acaacagcca caacgtctat a 21
<210> 4
   <211> 21
   <212> DNA
   <213> Homo sapiens
<400> 4
   cggttgctta tgaggaacca a 21
<210> 5
   <211> 21
   <212> DNA
   <213> Homo sapiens
<400> 5
   gccgatggac acaaacatga t 21
<210> 6
   <211> 19
   <212> DNA
   <213> Homo sapiens
<400> 6
   tctctgaacc aaacaagca 19
<210> 7
   <211> 19
   <212> DNA
   <213> Homo sapiens
<400> 7
   tgtggtttct cattatcct 19
<210> 8
   <211> 532
   <212> PRT
   <213> Homo sapiens
<400> 8
<210> 9
   <211> 358
   <212> PRT
   <213> Homo sapiens
<400> 9
   Gln Asn Leu Asp Ser Met Leu His Gly Thr Gly Met Lys Ser Asp Ser
   1               5                   10                  15
   Asp Gln Lys Lys Ser Glu Asn Gly Val Thr Leu Ala Pro Glu Asp Thr
               20                  25                  30
   Leu Pro Phe Leu Lys Cys Tyr Cys Ser Gly His Cys Pro Asp Asp Ala
           35                  40                  45
   Ile Asn Asn Thr Cys Ile Thr Asn Gly His Cys Phe Ala Ile Ile Glu
       50                  55                  60
   Glu Asp Asp Gln Gly Glu Thr Thr Leu Ala Ser Gly Cys Met Lys Tyr
   65                  70                  75                  80
   Glu Gly Ser Asp Phe Gln Cys Lys Asp Ser Pro Lys Ala Gln Leu Arg
                   85                  90                  95
   Arg Thr Ile Glu Cys Cys Arg Thr Asn Leu Cys Asn Gln Tyr Leu Gln
               100                 105                 110
   Pro Thr Leu Pro Pro Val Val Ile Gly Pro Phe Phe Asp Gly Ser Ile
           115                 120                 125
   Arg Thr Gly Gly Gly Thr His Thr Cys Pro Pro Cys Pro Ala Pro Glu
       130                 135                 140
   Leu Leu Gly Gly Pro Ser Val Phe Leu Phe Pro Pro Lys Pro Lys Asp
   145                 150                 155                 160
   Thr Leu Met Ile Ser Arg Thr Pro Glu Val Thr Cys Val Val Val Asp
                   165                 170                 175
   Val Ser His Glu Asp Pro Glu Val Lys Phe Asn Trp Tyr Val Asp Gly
               180                 185                 190
   Val Glu Val His Asn Ala Lys Thr Lys Pro Arg Glu Glu Gln Tyr Asn
           195                 200                 205
   Ser Thr Tyr Arg Val Val Ser Val Leu Thr Val Leu His Gln Asp Trp
       210                 215                 220
   Leu Asn Gly Lys Glu Tyr Lys Cys Lys Val Ser Asn Lys Ala Leu Pro
   225                 230                 235                 240
   Ala Pro Ile Glu Lys Thr Ile Ser Lys Ala Lys Gly Gln Pro Arg Glu
                   245                 250                 255
   Pro Gln Val Tyr Thr Leu Pro Pro Ser Arg Glu Glu Met Thr Lys Asn
               260                 265                 270
   Gln Val Ser Leu Thr Cys Leu Val Lys Gly Phe Tyr Pro Ser Asp Ile
           275                 280                 285
   Ala Val Glu Trp Glu Ser Asn Gly Gln Pro Glu Asn Asn Tyr Lys Thr
       290                 295                 300
   Thr Pro Pro Val Leu Asp Ser Asp Gly Ser Phe Phe Leu Tyr Ser Lys
   305                 310                 315                 320
   Leu Thr Val Asp Lys Ser Arg Trp Gln Gln Gly Asn Val Phe Ser Cys
                   325                 330                 335
   Ser Val Met His Glu Ala Leu His Asn His Tyr Thr Gln Lys Ser Leu
               340                 345                 350
   Ser Leu Ser Pro Gly Lys
           355
<210> 10
   <211> 21
   <212> DNA
   <213> Artificial Sequence
<220>
   <223> synthetic oligonucleotide
<400> 10
   gccaagtgtt acattgagct t 21
<210> 11
   <211> 21
   <212> DNA
   <213> Artificial Sequence
<220>
   <223> synthetic oligonucleotide
<400> 11
   gtgtccatgc tctcagacaa a 21
<210> 12
   <211> 21
   <212> DNA
   <213> Artificial Sequence
<220>
   <223> synthetic oligonucleotide
<400> 12
   cggttgctta tgaggaacca a 21
<210> 13
   <211> 21
   <212> DNA
   <213> Artificial Sequence
<220>
   <223> synthetic oligonucleotide
<400> 13
   gccgatggac acaaacatga t 21
<210> 14
   <211> 21
   <212> DNA
   <213> Artificial Sequence
<220>
   <223> synthetic oligonucleotide
<400> 14
   acaacagcca caacgtctat a 21
<210> 15
   <211> 20
   <212> DNA
   <213> Artificial Sequence
<220>
   <223> synthetic oligonucleotide
<400> 15
   ctgagaaact ggggctcaat 20
<210> 16
   <211> 20
   <212> DNA
   <213> Artificial Sequence
<220>
   <223> synthetic oligonucleotide
<400> 16
   cgaccagctc ctctttgtct 20
<210> 17
   <211> 20
   <212> DNA
   <213> Artificial Sequence
<220>
   <223> synthetic oligonucleotide
<400> 17
   cgtctaaagc agcaaacacc 20
<210> 18
   <211> 22
   <212> DNA
   <213> Artificial Sequence
<220>
   <223> synthetic oligonucleotide
<400> 18
   ccaaagtgga gagttcaaat gg 22
<210> 19
   <211> 19
   <212> DNA
   <213> Artificial Sequence
<220>
   <223> synthetic oligonucleotide
<400> 19
   cgagcaggag atgggaacc 19
<210> 20
   <211> 19
   <212> DNA
   <213> Artificial Sequence
<220>
   <223> synthetic oligonucleotide
<400> 20
   caacggaaac gctcattgc 19
<210> 21
   <211> 20
   <212> DNA
   <213> Artificial Sequence
<220>
   <223> synthetic oligonucleotide
<400> 21
   ccaacgcgcc tcgccggatc 20
<210> 22
   <211> 19
   <212> DNA
   <213> Artificial Sequence
<220>
   <223> synthetic oligonucleotide
<400> 22
   ctcctcgcca gtgcctcag 19
<210> 23
   <211> 20
   <212> DNA
   <213> Artificial Sequence
<220>
   <223> synthetic oligonucleotide
<400> 23
   ctggacgaca tgaaccactg 20
<210> 24
   <211> 20
   <212> DNA
   <213> Artificial Sequence
<220>
   <223> synthetic oligonucleotide
<400> 24
   gtagtcgatg acgcgctgta 20
<210> 25
   <211> 20
   <212> DNA
   <213> Artificial Sequence
<220>
   <223> synthetic oligonucleotide
<400> 25
   tgcaccacca actgcttagc 20
<210> 26
   <211> 21
   <212> DNA
   <213> Artificial Sequence
<220>
   <223> synthetic oligonucleotide
<400> 26
   ggcatggact gtggtcatga g 21